# EUROPEAN PATENT SPECIFICATION

(11) **EP 2 121 004 B1**
(45) Date of publication and mention of the grant of the patent: **03.07.2013**
(21) Application number: 08751043.4
(22) Date of filing: 31.01.2008
(51) Int. Cl.: A61K 38/46

(54) **OBLIGATE HETERODIMER MEGANUCLEASES AND USES THEREOF**
OBLIGATE HETERODIMER-MEGANUKLEASEN UND IHRE VERWENDUNG
MÉGANUCLÉASES HÉTÉRODIMÈRES OBLIGATOIRES ET LEURS UTILISATIONS

(30) Priority: 01.02.2007 WO PCT/IB2007/000849
(43) Date of publication of application: 25.11.2009
(60) Divisional application: 11193586.2
(73) Proprietor: CELLECTIS, 93230 Romainville (FR)
(72) Inventor: FAJARDO SANCHEZ, Emmanuel, E-03011 Alicante (ES); GRIZOT, Sylvestre, F-92250 La Garenne Colombes (FR); ISALAN, Mark, London SW14 8EA (GB); SERRANO PUBUL, Luis, Barcelona (ES); STRICHER, François, E-08173 Sant Cugat Del Valles (ES)
(74) Representative: Thomas, Dean
(86) International application number: PCT/IB2008/001333
(87) International publication number: WO 2008/093249

(56) References cited:
- WO-A-03/078619
- WO-A-2004/031346
- WO-A-2006/097854
- WO-A-2007/047859
- CHEVALIER B S ET AL: "Design, activity, and structure of a highly specific artificial endonuclease" MOLECULAR CELL, CELL PRESS, CAMBRIDGE, MA, US, vol. 10, no. 4, 20 October 2002 (2002-10-20), pages 895-905, XP002248750 ISSN: 1097-2765
- EPINAT J-C ET AL: "A novel engineered meganuclease induces homologous recombination in yeast and mammalian cells" NUCLEIC ACIDS RESEARCH, OXFORD UNIVERSITY PRESS, SURREY, GB, vol. 31, no. 11, 1 June 2003 (2003-06-01), pages 2952-2962, XP002248751 ISSN: 0305-1048
- ARNOULD ET AL: "Engineering of Large Numbers of Highly Specific Homing Endonucleases that Induce Recombination on Novel DNA Targets" JOURNAL OF MOLECULAR BIOLOGY, LONDON, GB, vol. 355, no. 3, 20 January 2006 (2006-01-20), pages 443-458, XP005206991 ISSN: 0022-2836
- FAJARDO-SANCHEZ E ET AL: "Computer design of obligate heterodimer meganucleases allows efficient cutting of custom DNA sequences" NUCLEIC ACIDS RESEARCH 200804 GB, vol. 36, no. 7, April 2008 (2008-04), pages 2163-2173, XP002491715 ISSN: 0305-1048 1362-4962

## Description

The invention relates to an obligate heterodimer meganuclease derived from homodimeric LAGLIDADG endonuclease monomers, to a vector encoding said meganuclease, to a cell, an animal or a plant modified by said vector and to the use of said meganuclease and derived products for molecular biology, genome engineering and genome therapy.

Meganucleases are by definition sequence-specific endonucleases with large (12-45 bp) cleavage sites that can deliver DNA double-strand breaks (DSBs) at specific loci in living cells (Thierry and Dujon, Nucleic Acids Res., 1992, 20, 5625-5631). Meganucleases have been used to stimulate homologous recombination in the vicinity of their target sequences in cultured cells and plants (Rouet et al., Mol. Cell. Biol., 1994, 14, 8096-106; Choulika et al., Mol. Cell. Biol., 1995, 15, 1968-73; Donoho et al., Mol. Cell. Biol, 1998, 18, 4070-8; Elliott et al., Mol. Cell. Biol., 1998, 18, 93-101; Sargent et al., Mol. Cell. Biol., 1997, 17, 267-77; Puchta et al., Proc. Natl. Acad. Sci. USA, 1996, 93, 5055-60; Chiurazzi et al., Plant Cell, 1996, 8, 2057-2066), making meganuclease-induced recombination an efficient and robust method for genome engineering.

The use of meganuclease-induced recombination has long been limited by the repertoire of natural meganucleases, and the major limitation of the current technology is the requirement for the prior introduction of a meganuclease cleavage site in the locus of interest. Thus, the engineering of redesigned meganucleases cleaving chosen targets is under intense investigation.

Such proteins could be used to cleave genuine chromosomal sequences and open new perspectives for genome engineering in wide range of applications. For example, meganucleases could be used to knock-out endogenous genes or knock-in exogenous sequences in the chromosome. It can as well be used for gene correction, and in principle, for the correction of mutations linked with monogenic diseases.

Recently, Zinc-Finger DNA binding domains of Cys2-His2 type Zinc-Finger Proteins (ZFP) were fused with the catalytic domain of the *Fok*I endonuclease, to induce recombination in various cell types: mammalian cultured cells including human lymphoid cells, plants and insects (Smith et al., Nucleic Acids Res, 1999, 27, 674-81; Pabo et al., Annu. Rev. Biochem, 2001, 70, 313-40; Porteus and Baltimore, Science, 2003, 300, 763; Urnov et al., Nature, 2005, 435, 646-651; Bibikova et al., Science, 2003, 300, 764; Durai et al., Nucleic Acids Res., 2005, 33, 5978-5990; Porteus M.H., Mol. Ther., 2006, 13, 438-446). The binding specificity of ZFPs is relatively easy to manipulate, and a repertoire of novel artificial ZFPs, able to bind many (g/a)nn(g/a)nn(g/a)nn sequences is now available (Pabo et al., precited; Segal and Barbas, Curr. Opin. Biotechnol., 2001, 12, 632-7; Isalan et al., Nat. Biotechnol., 2001, 19, 656-60). However, preserving a very narrow specificity is one of the major issues for genome engineering applications, and presently it is unclear whether ZFPs would fulfill the very strict requirements for therapeutic applications. Furthermore, these fusion proteins have demonstrated high toxicity in Drosophila (Bibikova et al., Science, 2003, 300, 764; Bibikova et al., Genetics, 2002, 161, 1169-1175) and mammalian NIHT3 cells (Alwin et al., Mol. Ther., 2005, 12, 610-617; Porteus M.H. and Baltimore D., Science, 2003, 300,763; Porteus M.H. and Carroll D., Nat. Biotechnol., 2005, 967-973), a genotoxic effect that is probably due to frequent off-site cleavage (Porteus, M.H., Mol. Ther., 2006, 13,438-446).

In nature, meganucleases are essentially represented by homing endonucleases (HEs), a family of endonucleases encoded by mobile genetic elements, whose function is to initiate DNA double-strand break (DSB)-induced recombination events in a process referred to as homing (Chevalier and Stoddard, Nucleic Acids Res., 2001, 29, 3757-74; Kostriken et al., Cell; 1983, 35, 167-74; Jacquier and Dujon, Cell, 1985, 41, 383-94). Several hundreds of HEs have been identified in bacteria, eukaryotes, and archea (Chevalier and Stoddard, Nucleic Acids Res., 2001, 29, 3757-74); however the probability of finding a HE cleavage site in a chosen gene is very low.

Given their biological function and their exceptional cleavage properties in terms of efficacy and specificity, HEs provide ideal scaffolds to derive novel endonucleases for genome engineering.

Data have accumulated over the last decade, allowing a relatively good characterization of the LAGLIDADG family, the largest of the four HE families (Chevalier and Stoddard, Nucleic Acids Res., 2001, 29, 3757-74). LAGLIDADG refers to the only sequence actually conserved throughout the family, and is found in one or (more often) two copies in the protein. Proteins with a single motif, such as I-*Cre*I form homodimers and cleave palindromic or pseudo-palindromic DNA sequences, whereas the larger, double motif proteins, such as PI-SceI are monomers and cleave non palindromic targets. Nine different LAGLIDADG proteins have been crystallized, showing a very striking core structure conservation that contrasts with the lack of similarity at the primary sequence level (Chevalier et al., Nat. Struct. Biol., 2001, 8, 312-316; Jurica et al., Mol. Cell., 1998, 2, 469-476; Chevalier et al., J. Mol. Biol., 2003, 329, 253-269; Moure et al., J. Mol. Biol., 2003, 334, 685-695; Moure et al., Nat. Struct. Biol., 2002, 9, 764-770; Ichiyanagi et al., J. Mol. Biol., 2000, 300, 889-901; Gimble et al., J. Biol. Chem., 1998, 273, 30524-30529; Bolduc et al., Genes Dev. 2003, 17, 2875-2888; Silva et al., J. Mol. Biol., 1999, 286, 1123-1136; Nakayama et al., J. Mol. Biol., Epub 29 septembre 2006, Spiegel et al., Structure, 2006, 14, 869-880).

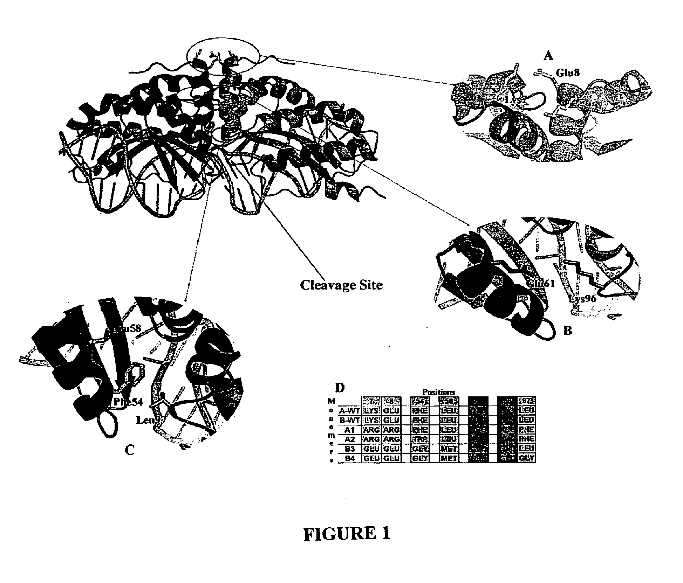

In this core structure (Figure 1), two characteristic αββαββα folds, contributed by two monomers, or two domains in double LAGLIDADG proteins, are facing each other with a two-fold symmetry. DNA binding depends on the four β strands from each domain, folded into an antiparallel β-sheet, and forming a saddle on the DNA helix major groove. Analysis of I-*Cre*I structure bound to its natural target shows that in each monomer, eight residues (Y33, Q38, N30, K28, Q26, Q44, R68 and R70) establish direct interaction with seven bases at positions ± 3, 4, 5, 6, 7, 9 and 10 of the target DNA (Jurica et al., Mol. Cell., 1998, 2, 469-76). In addition, some residues establish water-mediated contact with several bases; for example S40, K28 and N30 with the base pair at positions +8 and -8 (Chevalier et al., J. Mol. Biol., 2003, 329, 253-269).

The catalytic site is central, formed with contributions from helices of both monomers. Just above the catalytic site, the two LAGLIDADG peptides play also an essential role in the dimerization interface. In addition to this core structure, other domains can be found, for instance, PI-*Sce*I, an intein, has a protein splicing domain, and an additional DNA-binding domain (Moure et al., Nat. Struct. Biol., 2002, 9, 764-770; Pingoud et al., Biochemistry, 1998, 37, 8233-8243).

The extensive structural conservation within the meganuclease family has encouraged, both the mutagenesis of HEs and the construction of chimeric and single chain HEs, which withstood extensive modifications.

The making of functional chimeric and single chain artificial HEs, by fusing the N-terminal I-*Dmo*I domain with an I*-Cre*I monomer (Epinat et al., Nucleic Acids Res., 2003, 31, 2952-62; Chevalier et al., Mol. Cell., 2002, 10, 895-905; International PCT Applications WO 03/078619 and WO 2004/031346) has demonstrated the plasticity of LAGLIDADG proteins: different monomers or core domains could be fused in a single protein, to obtain novel meganucleases cleaving novel (non-palindromic) target sequences.

Besides, different groups have used a rational approach to locally alter the specificity of the I*-Cre*I (Seligman et al., Nucleic Acids Res., 2002, 30, 3870-3879; Sussman et al., J. Mol. Biol., 2004, 342, 31-41; Rosen et al., Nucleic Acids Res., 2006, 34, 4791-4800; Arnould et al., J. Mol. Biol., 2006, 355, 443-458 and International PCT Applications WO 2006/097853 and WO 2006/097784; Smith et al., Nucleic Acids Res., 2006, 34, e149), I*-Sce*I (Doyon et al., J. Am. Chem. Soc., 2006, 128, 2477-2484), PI-*Sce*I (Gimble et al., J. Mol. Biol., 2003, 334, 993-1008) and I-*Mso*I (Ashworth et al., Nature, 2006, 441, 656-659).

Recent work has shown that it is possible to obtain large number of locally altered variants of the I*-Cre*I meganuclease that recognize a variety of targets and to assemble them by a combinatorial process, to obtain entirely redesigned mutants with chosen specificity (Arnould et al., J. Mol. Biol., 2006, 355, 443-458; International PCT Applications WO 2006/097853 and WO 2006/097784; Smith et al., Nucleic Acids Res., 2006, 34, e149).

Thus, hundreds of I-*Cre*I derivatives with altered specificity were engineered by combining the semi-rational approach and High Throughput Screening:
- Residues Q44, R68 and R70 or Q44, R68, D75 and I77 of I*-Cre*I were mutagenized and a collection of variants with altered specificity at positions ± 3 to 5 of the DNA target (5NNN DNA target) were identified by screening (International PCT Applications WO 2006/097784 and WO 2006/097853; Arnould et al., J. Mol. Biol., 2006, 355, 443-458; Smith et al., Nucleic Acids Res., 2006, 34, e149).
- Residues K28, N30 and Q38, N30, Y33 and Q38 or K28, Y33, Q38 and S40 of I*-Cre*I were mutagenized and a collection of variants with altered specificity at positions ± 8 to 10 of the DNA target (10NNN DNA target) were identified by screening (Smith et al., Nucleic Acids Res., 2006, 34, e149).

Residues 28 to 40 and 44 to 77 of I*-Cre*I were shown to form two separable functional subdomains, able to bind distinct parts of a homing endonuclease half-site (Smith et al. Nucleic Acids Res., 2006, 34, e149).

The combination of mutations from the two subdomains of I*-Cre*I within the same monomer allowed the design of novel chimeric molecules (homodimers) able to cleave a palindromic combined DNA target sequence comprising the nucleotides at positions ± 3 to 5 and ± 8 to 10 which are bound by each subdomain (Smith et al., Nucleic Acids Res., 2006, 34, e149).

Two different variants were combined and assembled in a functional heterodimeric endonuclease able to cleave a chimeric target resulting from the fusion of a different half of each variant DNA target sequence (Arnould *et al.,* precited; International PCT Application WO 2006/097854). Interestingly, the novel proteins had kept proper folding and stability, high activity, and a narrow specificity.

The combination of the two former steps allows a larger combinatorial approach, involving four different subdomains. The different subdomains can be modified separately and combined in one meganuclease variant (heterodimer or single-chain molecule) which is able to cleave a target from a gene of interest. In a first step, couples of novel meganucleases are combined in new molecules ("half-meganucleases") cleaving palindromic targets derived from the target one wants to cleave. Then, the combination of such "half-meganuclease" can result in an heterodimeric species cleaving the target of interest. The assembly of four set of mutations into heterodimeric endonucleases cleaving a model target sequence or a sequence from the human RAG 1 gene has been described in Smith et al. (Nucleic Acids Res., 2006, 34, e 149).

These variants can be used to cleave genuine chromosomal sequences and have paved the way for novel perspectives in several fields, including gene therapy. For example, meganuclease-induced recombination, could be used for the correction of mutations linked with monogenic inherited diseases such as SCID, SCA or CFTR. This strategy would have the advantage to bypass the odds associated with current strategies of random insertion of a complementing transgene.

Although the I*-Sce*I homing endonuclease has been shown to be less toxic than ZFPs (Alwin et al., Mol. Ther., 2005, 12, 610-617; Porteus M.H. and Baltimore D., Science, 2003, 300, 763; Porteus M.H. and Carroll D., Nat. Biotechnol., 2005, 23, 967-973), probably because of better specificity, I*-Sce*I can still be harmful at very high doses (Gouble et al., J. Gene Med., 2006, 8, 616-622). Off-site cleavage is severely enhanced by the formation of protein engineering by-products. Most engineered endonucleases (ZFNs and HEs) so far are heterodimers, and include two separately engineered monomers, each binding one half of the target. Heterodimer formation is obtained by co-expression of the two monomers in the same cells (Porteus H.M., Mol. Ther., 2006, 13, 438-446; Smith et al., Nucleic acids Res., 2006, 34, e 149).

However, it is actually associated with the formation of two homodimers (Arnould et al., J. Mol. Biol., 2006, 355, 443-458; Bibikova et al., Genetics, 2002, 161, 1169-1175), recognizing different targets, and individual homodimers can sometimes result in an extremely high level of toxicity (Bibikova et al., Genetics, 2002, 161, 1169-1175; Beumer et al., Genetics, 2006, 172, 2391-403).

Thus, a limiting factor that still remains for a more widespread use of the single-LAGLIDADG homing endonucleases such as I*-Cre*I*,* is the fact that the proteins are homodimers. Although co-expression of two different I*-Cre*I variants that target two different DNA sequences result in the formation of a functional heterodimer that recognizes a hybrid DNA sequence, this still results in a mixture of three different enzymes, including both homodimers (Arnould et al., J. Mol. Biol., 2006, 355, 443-458; International PCT Applications WO 2006/097853 and WO 2006/097784; Smith et al., Nucleic Acids Res., 2006, 34, e149).

This loss of specificity can be solved only by the suppression of functional homodimer formation. This outcome could, in theory, be achieved by the fusion of the two monomers in a single chain molecule (Epinat et al., Nucleic Acids Res., 2005, 33, 5978-5990; International Application WO 03/078619). However, the single chain molecule would not necessarily alleviate the interaction of αββαββα folds from distinct molecules, especially if the linker is long and flexible, but it should at least favor interactions between αββαββα folds from a same molecule.

The making of obligatory heterodimeric meganucleases provides functional, well folded proteins that solve a major specificity and toxicity issue in genome engineering applications.

Furthermore, the making of single chain molecule and the redesign of the dimerization interface are not exclusive strategies and could be used jointly.

Therefore, the inventors have re-designed the interaction surface (interface) between two I-*Cre*I meganuclease monomers, to obtain an obligatory heterodimer.

A large part of the dimerisation interface of the homodimer is composed of two α-helices (Lys7 to Gly 19 in both monomers), arranged in a coiled-coil, making them difficult to re-design. The amino acids below these helices (Asp20 and onward) are contacting the DNA and thus are responsible of both the activity (active site) and specificity (DNA recognition) of the endonucleases. These functions alone prevent any of these residues to be modified easily in the design process. Thus, this left few possibilities to impair the formation of functional homodimers. Nevertheless, the inventors have identified four sites of interactions involved in the interface that could be disturbed and changed in the dimers, without preventing their binding capacity or their enzymatic activity.

In each site, two residues (Z and Z') in one monomer (A) establish favorable interactions with the corresponding residues (Z' and Z, respectively) in the other monomer (B). In order to keep this interaction in the heterodimer, and at the same time impair formation of functional homodimers, the residues Z and Z' were replaced with two residues Z in one monomer and two residues Z' in the other. Therefore, one residue of one monomer, for example Z in monomer (A) was replaced with a residue functionally equivalent to Z', and in the other monomer (B), Z' was replaced with a residue which is functionally equivalent to Z. Thus, AA and BB homodimers undergo repulsion whereas AB heterodimer formation is favorable.

The new monomers, which were themselves engineered to recognise different DNA sequences, allow functional heterodimer formation and prevent homodimer site cleavage. This design dramatically improves the ability to engineer very specific reagents for genome engineering and removes one of the last hurdles on the way of using redesigned meganucleases for gene therapy and other applications. For therapeutic applications, which require a minimal genotoxicity, this gain in specificity might simply make all the difference.

The invention relates to an obligate heterodimer meganuclease consisting of a first and a second monomer (A and B) deriving from two different homodimeric LAGLIDADG endonuclease (parent monomers), and having at least one pair of mutations interesting corresponding residues of said parent monomers which make an intermolecular interaction between the two monomers of each parent homodimeric LAGLIDADG endonuclease, wherein the first mutation of said pair(s) is in the first monomer and the second mutation of said pair(s) is in the second monomer and said pair(s) of mutations impairs the formation of functional homodimers from each monomer without preventing the formation of a functional heterodimer, able to cleave a non-palindromic hybrid DNA target comprising one different half of the DNA target cleaved by each parent homodimeric endonuclease.

Each parent monomer has at least two residues Z and Z' of the dimerisation interface which interact with residues Z' and Z, respectively of the same or another parent monomer (two pairs ZZ' of interacting residues) to form two homodimers and one heterodimer. According to the present invention, one of the two pairs of interacting residues of the dimerisation interface is swapped to obtain a monomer A having two residues Z or Z' and a monomer B having two residues Z' or Z, respectively. As a result, A and B monomers each having two residues Z or two residues Z' can less easily homodimerize than their parent counterpart, whereas the presence of two pairs ZZ' of interacting residues at the heterodimer AB interface makes AB heterodimer formation favourable.

### Definitions

- Amino acid refers to a natural or synthetic amino acid including enantiomers and stereoisomers of the preceding amino acids.
   Amino acid residues in a polypeptide sequence are designated herein according to the one-letter code, in which, for example, Q means Gln or Glutamine residue, R means Arg or Arginine residue and D means Asp or Aspartic acid residue.
- Acidic amino acid refers to aspartic acid (D) and Glutamic acid (E).
- Basic amino acid refers to lysine (K), arginine (R) and histidine (H).
- Small amino acid refers to glycine (G) and alanine (A).
- Aromatic amino acid refers to phenylalanine (F), tryptophane (W) and tyrosine (Y).
- Nucleotides are designated as follows: one-letter code is used for designating the base of a nucleoside: a is adenine, t is thymine, c is cytosine, and g is guanine. For the degenerated nucleotides, r represents g or a (purine nucleotides), k represents g or t, s represents g or c, w represents a or t, m represents a or c, y represents t or c (pyrimidine nucleotides), d represents g, a or t, v represents g, a or c, b represents g, t or c, h represents a, t or c, and n represents g, a, t or c.
- by "meganuclease" is intended an endonuclease having a double-stranded DNA target sequence of 12 to 45 bp.
- by "homodimeric LAGLIDADG homing endonuclease" is intended a wild-type homodimeric LAGLIDADG homing endonuclease having a single LAGLIDADG motif and cleaving palindromic DNA target sequences, such as I*-Cre*I or I*-Mso*I or a functional variant thereof.
- by "LAGLIDADG homing endonuclease variant" or "variant" is intended a protein obtained by replacing at least one amino acid of a LAGLIDADG homing endonuclease sequence, with a different amino acid.
- by "functional variant" is intended a LAGLIDADG homing endonuclease variant which is able to cleave a DNA target, preferably a new DNA target which is not cleaved by a wild-type LAGLIDADG Homing Endonuclease. For example, such variants have amino acid variation at positions contacting the DNA target sequence or interacting directly or indirectly with said DNA target.
- by "variant with novel specificity" is intended a variant having a pattern of cleaved targets different from that of the parent homing endonuclease. The terms "novel specificity", "modified specificity", "novel cleavage specificity", "novel substrate specificity" which are equivalent and used indifferently, refer to the specificity of the variant towards the nucleotides of the DNA target sequence.
- by *"*I-*Cre*I*"* is intended the wild-type I*-Cre*I having the sequence SWISSPROT P05725 (SEQ ID NO: 1), or pdb accession code 1g9y (SEQ ID NO: 43).
- by "domain" or "core domain" is intended the "LAGLIDADG homing endonuclease core domain" which is the characteristic α₁β₁β₂α₂β₃β₄α fold of the homing endonucleases of the LAGLIDADG family, corresponding to a sequence of about one hundred amino acid residues. Said domain comprises four beta-strands (β₁, β₂, β₃, β₄) folded in an antiparallel beta-sheet which interacts with one half of the DNA target. This domain is able to associate with another LAGLIDADG Homing Endonuclease Core Domain which interacts with the other half of the DNA target to form a functional endonuclease able to cleave said DNA target. For example, in the case of the dimeric homing endonuclease I*-Cre*I (163 amino acids), the LAGLIDADG Homing Endonuclease Core Domain corresponds to the residues 6 to 94.
- by "subdomain" is intended the region of a LAGLIDADG homing endonuclease core domain which interacts with a distinct part of a homing endonuclease DNA target half-site.
- by "beta-hairpin" is intended two consecutive beta-strands of the antiparallel beta-sheet of a LAGLIDADG homing endonuclease core domain (β₁β₂ or, β₃β₄) which are connected by a loop or a turn,
- by "DNA target", "DNA target sequence", "target sequence" , "target-site", "target" , "site"; "recognition site", "recognition sequence", "homing recognition site", "homing site", "cleavage site" is intended a 22 to 24 bp double-stranded palindromic, partially palindromic (pseudo-palindromic) or non-palindromic polynucleotide sequence that is recognized and cleaved by a LAGLIDADG homing endonuclease. These terms refer to a distinct DNA location, preferably a genomic location, at which a double stranded break (cleavage) is to be induced by the endonuclease. The DNA target is defined by the 5' to 3' sequence of one strand of the double-stranded polynucleotide. For example, the palindromic DNA target sequence cleaved by wild-type I*-Cre*I is defined by the sequence 5'- t₋₁₂c₋₁₁a₋₁₀a₋₉a₋₈a₋₇c₋₆g₋₅t₋₄c-₃g₋₂t₋₁a₊₁c₊₂g₊₃a₊₄c₊₅g₊₆t₊₇t₊₈t₊₉t₊₁₀g₊₁₁a₊₁₂ (SEQ ID NO :2).
- by " DNA target half-site", "half cleavage site" or half-site" is intended the portion of the DNA target which is bound by each LAGLIDADG homing endonuclease core domain/monomer.
- by "chimeric DNA target" or "hybrid DNA target" is intended the fusion of a different half of two parent meganuclease target sequences. In addition at least one half of said target may comprise the combination of nucleotides which are bound by at least two separate subdomains (combined DNA target).
- by "vector" is intended a nucleic acid molecule capable of transporting another nucleic acid to which it has been linked.
- by "homologous" is intended a sequence with enough identity to another one to lead to a homologous recombination between sequences, more particularly having at least 95 % identity, preferably 97 % identity and more preferably 99 %.
- "Identity" refers to sequence identity between two nucleic acid molecules or polypeptides. Identity can be determined by comparing a position in each sequence which may be aligned for purposes of comparison. When a position in the compared sequence is occupied by the same base, then the molecules are identical at that position. A degree of similarity or identity between nucleic acid or amino acid sequences is a function of the number of identical or matching nucleotides at positions shared by the nucleic acid sequences. Various alignment algorithms and/or programs may be used to calculate the identity between two sequences, including FASTA, or BLAST which are available as a part of the GCG sequence analysis package (University of Wisconsin, Madison, Wis.), and can be used with, e.g., default settings.
- "individual" includes mammals, as well as other vertebrates (e.g., birds, fish and reptiles). The terms "mammal" and "mammalian", as used herein, refer to any vertebrate animal, including monotremes, marsupials and placental, that suckle their young and either give birth to living young (eutharian or placental mammals) or are egg-laying (metatharian or nonplacental mammals). Examples of mammalian species include humans and other primates (e.g., monkeys, chimpanzees), rodents (e.g., rats, mice, guinea pigs) and ruminants (e.g., cows, pigs, horses).
- "genetic disease" refers to any disease, partially or completely, directly or indirectly, due to an abnormality in one or several genes. Said abnormality can be a mutation, an insertion or a deletion. Said mutation can be a punctual mutation. Said abnormality can affect the coding sequence of the gene or its regulatory sequence. Said abnormality can affect the structure of the genomic sequence or the structure or stability of the encoded mRNA. Said genetic disease can be recessive or dominant. Such genetic disease could be, but are not limited to, cystic fibrosis, Huntington's chorea, familial hyperchoiesterolemia (LDL receptor defect), hepatoblastoma, Wilson's disease, congenital hepatic porphyrias, inherited disorders of hepatic metabolism, Lesch Nyhan syndrome, sickle cell anemia, thalassaemias, xeroderma pigmentosum, Fanconi's anemia, retinitis pigmentosa, ataxia telangiectasia, Bloom's syndrome, retinoblastoma, Duchenne's muscular dystrophy, and Tay-Sachs disease.

According to the present invention the positions of the mutations are indicated by reference to the I*-Cre*I amino acid sequence SEQ ID NO: 1. Knowing the positions of the mutations in I*-Cre*I structure (pdb accession code 1g9y), one skilled in the art can easily deduce the corresponding positions in another homodimeric LAGLIDADG homing endonuclease, using well-known protein structure analyses softwares such as Pymol. For example, the positions K96 and E61 in I*-Cre*I correspond to R102 and Q64 in I-*Mso*I. In addition, for I*-Mso*I*,* the two functional subdomains situated from positions 26 to 40 and 44 to 77 in I*-Cre*I are situated from positions 28 to 43 and 47 to 83, respectively.

According to a preferred embodiment of said obligate heterodimer meganuclease, the monomers have at least one of the following pairs of mutations, respectively for the first and the second monomer:
a) the substitution of the glutamic acid at position 8 with a basic amino acid (monomer A) and the substitution of the lysine at position 7 with an acidic amino acid (monomer B),
b) the substitution of the glutamic acid at position 61 with a basic amino acid (monomer A) and the substitution of the lysine at position 96 with an acidic amino acid (monomer B),
c) the substitution of the leucine at position 97 with an aromatic amino acid (monomer A) and the substitution of the phenylalanine at position 54 with a small amino acid (monomer B), and
d) the substitution of the aspartic acid at position 137 with a basic amino acid (monomer A) and the substitution of the arginine at position 51 with an acidic amino acid (monomer B), said positions being indicated by reference to the I-*CreI* amino acid sequence SEQ ID NO: 1.

According to a more preferred embodiment of said obligate heterodimer meganuclease, the monomer having the substitution of the glutamic acid at position 8 or 61 by a basic amino acid, as defined in a) or b), further comprises the substitution of at least one of the lysine residues at positions 7 and 96, by an arginine.

According to another more preferred embodiment of said obligate heterodimer meganuclease, the monomer having the substitution of the leucine 97 by an aromatic amino acid as defined in c), further comprises the substitution of the phenylalanine at position 54 by a tryptophane.

According to another more preferred embodiment of said obligate heterodimer meganuclease, the monomer having the substitution of the phenylalanine at position 54 by a small amino acid as defined in c), further comprises the substitution of the leucine at position 58 or lysine at position 57, by a methionine.

According to another more preferred embodiment of said obligate heterodimer meganuclease, the acidic amino acid is a glutamic acid.

According to another more preferred embodiment of said obligate heterodimer meganuclease, the basic amino acid is an arginine.

According to another more preferred embodiment of said obligate heterodimer meganuclease, the aromatic amino acid is a phenylalanine.

According to another more preferred embodiment of said obligate heterodimer meganuclease, the small amino acid is a glycine.

According to another more preferred embodiment of said obligate heterodimer meganuclease, it consists of a first monomer (A) having the mutation D137R and a second monomer (B) having the mutation R51D.

According to another more preferred embodiment of said obligate heterodimer meganuclease, it comprises at least two pairs of mutations as defined in a), b) c) or d), above; one of the pairs of mutation is advantageously as defined in c) or d)Preferably, one monomer comprises the substitution of the lysine residues at positions 7 and 96 by an acidic amino acid and the other monomer comprises the substitution of the glutamic acid residues at positions 8 and 61 by a basic amino acid.

More preferably, the obligate heterodimer meganuclease, comprises three pairs of mutations as defined in a), b) and c), above. The obligate heterodimer meganuclease consists advantageously of a first monomer (A) having at least the mutations selected from: (i) E8R, E8K or E8H, E61R, E61K or E61H and L97F, L97W or L97Y; (ii) K7R, E8R, E61R, K96R and L97F, or (iii) K7R, E8R, F54W, E61R, K96R and L97F and a second monomer (B) having at least the mutations (iv) K7E or K7D, F54G or F54A and K96D or K96E; (v) K7E, F54G, L58M and K96E, or (vi) K7E, F54G, K57M and K96E.

The obligate heterodimer meganuclease according to the present invention is derived from a wild-type homodimeric LAGLIDADG homing endonuclease or a functional variant thereof. Examples of wild-type homodimeric LAGLIDAG homing endonucleases are presented in Table 1 of Lucas et al., Nucleic Acids Res., 2001, 29, 960-969. The wild-type homodimeric LAGLIDADG homing endonuclease may be advantageously selected from the group consisting of: I*-Cre*I*,* I-*Ceu*I*,* I*-Mso*I and I*-Cpa*I*,* preferably I*-Cre*I*.*

The monomers A and B differ from the wild-type monomer by one or more mutations outside of the dimerisation interface. The additional mutations are advantageously at positions of amino acid residues which interact with a DNA target half-site. The LAGLIDADG homing endonucleases DNA interacting residues are well-known in the art (Jurica et al., Molecular Cell., 1998, 2, 469-476; Chevalier et al., J. Mol. Biol., 2003, 329, 253-269). The residues which are mutated may interact with the DNA backbone or with the nucleotide bases, directly or via a water molecule. Preferably said mutations modify the cleavage specificity of the meganuclease and result in a meganuclease with novel specificity, which is able to cleave a DNA target from a gene of interest. More preferably, said mutations are substitutions of one or more amino acids in a first functional subdomain corresponding to that situated from positions 26 to 40 of I*-Cre*I amino acid sequence, that alter the specificity towards the nucleotide at positions ± 8 to 10 of the DNA target, and/or substitutions in a second functional subdomain corresponding to that situated from positions 44 to 77 of I-*Cre*I amino acid sequence, that alter the specificity towards the nucleotide at positions ± 3 to 5 of the DNA target, as described previously (International PCT Applications WO 2006/097784, WO 2006/097853, WO 2007/060495, WO 2007/049156, WO 2007/049095 and WO 2007/057781; Arnould et al., J. Mol. Biol., 2006, 355, 443-458; Smith et al., Nucleic Acids Res., 2006, 34, e149). The substitutions correspond advantageously to positions 26, 28, 30, 32, 33, 38, and/or 40, 44, 68, 70, 75 and/or 77 of I*-Cre*I amino acid sequence. For cleaving a DNA target, wherein n₋₄ is t or n₊₄ is a, said variant has advantageously a glutamine (Q) at position 44; for cleaving a DNA target, wherein n₋₄ is a or n₊₄ is t, said variant has an alanine (A) or an asparagine at position 44, and for cleaving a DNA target, wherein n₋₉ is g or n₊₉ is c, said variant has advantageously an arginine (R) or a lysine (K) at position 38.

According to another advantageous embodiment of said obligate heterodimer meganuclease, the monomers A and B are different I*-Cre*I monomer variants, preferably variants having mutations at positions 26 to 40 and/or 44 to 77 of I*-Cre*I,; the obligate heterodimer meganuclease AB consisting of these two monomers is able to cleave a non-palindromic DNA target, wherein at least the nucleotides at positions +3 to +5, +8 to +10, -10 to -8 and -5 to -3 of said DNA target correspond to the nucleotides at positions +3 to +5, +8 to +10 , -10 to -8 and -5 to -3 of a DNA target from a gene of interest. Preferably, both monomers of the heterodimer are mutated at positions 26 to 40 and/or 44 to 77. More preferably, both monomers have different mutations at positions 26 to 40 and 44 to 77 of I*-Cre*I*.*

The monomers A and B may comprise one or more mutations at other positions that interact with the DNA target sequence. In particular, additional substitutions may be introduced at positions contacting the phosphate backbone, for example in the final C-terminal loop (positions 137 to 143; Prieto et al., Nucleic Acids Res., Epub 22 April 2007). Preferably said residues are involved in binding and cleavage of said DNA cleavage site. More preferably, said residues are at positions 138, 139, 142 or 143 of I-*Cre*I. Two residues may be mutated in one domain provided that each mutation is in a different pair of residues chosen from the pair of residues at positions 138 and 139 and the pair of residues at positions 142 and 143. The mutations which are introduced modify the interaction(s) of said amino acid(s) of the final C-terminal loop with the phosphate backbone of the I*-Cre*I site. Preferably, the residue at position 138 or 139 is substituted by an hydrophobic amino acid to avoid the formation of hydrogen bonds with the phosphate backbone of the DNA cleavage site. For example, the residue at position 138 is substituted by an alanine or the residue at position 139 is substituted by a methionine. The residue at position 142 or 143 is advantageously substituted by a small amino acid, for example a glycine, to decrease the size of the side chains of these amino acid residues. More, preferably, said substitution in the final C-terminal loop modifies the specificity of the obligate heterodimer meganuclease towards the nucleotide at positions ± 1 to 2, ± 6 to 7 and/or ± 11 to 12 of the I*-Cre*I site.

Furthermore, the monomers A and B may comprise one or more additional mutations that improve the binding and/or the cleavage properties of the obligate heterodimer meganuclease towards the DNA target sequence from a gene of interest. The additional residues which are mutated may be on the entire sequence of the monomer(s). Example of mutations include the following mutations, by reference to I*-Cre*I amino acid sequence: I24V, R70S, the mutation of the aspartic acid at position 75, in an uncharged amino acid, preferably an asparagine (D75N) or a valine (D75V) and substitutions in the C-terminal half of the monomer sequence, preferably at positions: 80, 82, 85, 86, 87, 94, 96, 100, 103, 114, 115, 117, 125, 129, 131, 132, 147, 151, 153, 154, 55, 157, 159 and 160.

In another advantageous embodiment of said obligate heterodimer meganuclease, said mutations are replacement of the initial amino acids with amino acids selected from the group consisting of: A, D, E, G, H, K, N, P, Q, R, S, T, Y, C, V, L and W.

In addition, one or more residues may be inserted at the NH₂ terminus and/or COOH terminus of the monomer(s). For example, a methionine residue is introduced at the NH₂ terminus, a tag (epitope or polyhistidine sequence) is introduced at the NH₂ terminus and/or COOH terminus; said tag is useful for the detection and/or the purification of the heterodimer.

The invention relates also to the monomer A or B of an obligate heterodimer meganuclease as defined above.

The invention relates also to a single-chain meganuclease (fusion protein) comprising the monomers A and B as defined above, connected by a peptidic linker.

The subject-matter of the present invention is also a polynucleotide fragment encoding at least one of the monomers of an obligate heterodimer meganuclease or single-chain derivative, as defined above.

The subject-matter of the present invention is also a recombinant vector comprising at least one polynucleotide fragment as defined above. Said vector may advantageously comprise two different polynucleotide fragments, each encoding one of the monomers of the obligate heterodimer meganuclease of the invention.

A vector which can be used in the present invention includes, but is not limited to, a viral vector, a plasmid, a RNA vector or a linear or circular DNA or RNA molecule which may consists of a chromosomal, non chromosomal, semisynthetic or synthetic nucleic acids. Preferred vectors are those capable of autonomous replication (episomal vector) and/or expression of nucleic acids to which they are linked (expression vectors). Large numbers of suitable vectors are known to those of skill in the art and commercially available.

Viral vectors include retrovirus, adenovirus, parvovirus (e. g. adeno-associated viruses), coronavirus, negative strand RNA viruses such as orthomyxovirus (e. g., influenza virus), rhabdovirus (e. g., rabies and vesicular stomatitis virus), para-myxovirus (e. g. measles and Sendai), positive strand RNA viruses such as picor-navirus and alphavirus, and double-stranded DNA viruses including adenovirus, herpesvirus (e. g., Herpes Simplex virus types 1 and 2, Epstein-Barr virus, cytomegalovirus), and poxvirus (e. g., vaccinia, fowlpox and canarypox). Other viruses include Norwalk virus, togavirus, flavivirus, reoviruses, papovavirus, hepadnavirus, and hepatitis virus, for example. Examples of retroviruses include: avian leukosis-sarcoma, mammalian C-type, B-type viruses, D type viruses, HTLV-BLV group, lentivirus, spumavirus (Coffin, J. M., Retroviridae: The viruses and their replication, In Fundamental Virology, Third Edition, B. N. Fields, et *al.,* Eds., Lippincott-Raven Publishers, Philadelphia, 1996).

Preferred vectors include lentiviral vectors, and particularly self inactivacting lentiviral vectors.

Vectors can comprise selectable markers, for example: neomycin phosphotransferase, histidinol dehydrogenase, dihydrofolate reductase, hygromycin phosphotransferase, herpes simplex virus thymidine kinase, adenosine deaminase, glutamine synthetase, and hypoxanthine-guanine phosphoribosyl transferase for eukaryotic cell culture; TRP1 for S. *cerevisiae*; tetracycline, rifampicin or ampicillin resistance in E. *coli.*

Preferably said vectors are expression vectors, wherein the sequence(s) encoding the obligate heterodimermeganuclease/single-chain derivative of the invention is placed under control of appropriate transcription and translation regulatory sequences to permit production or synthesis of said meganuclease. Therefore, said polynucleotide is comprised in expression cassette. More particularly, the vector comprises a replication origin, a promoter operatively linked to said encoding polynucleotide, a ribosome-binding site, an RNA-splicing site (when genomic DNA is used), a polyadenylation site and a transcription termination site. It also can comprise an enhancer. Selection of the promoter will depend upon the cell in which the polypeptide is expressed. Preferably, the two polynucleotides encoding each of the monomers of the obligate heterodimer meganuclease are included in one vector which is able to drive the expression of both polynucleotides, simultaneously. Suitable promoters include tissue specific and/or inducible promoters. Examples of inducible promoters are: eukaryotic metallothionine promoter which is induced by increased levels of heavy metals, prokaryotic lacZ promoter which is induced in response to isopropyl-β-D-thiogalacto-pyranoside (IPTG) and eukaryotic heat shock promoter which is induced by increased temperature. Examples of tissue specific promoters are skeletal muscle creatine kinase, prostate-specific antigen (PSA), α-antitrypsin protease, human surfactant (SP) A and B proteins, β-casein and acidic whey protein genes.

According to another advantageous embodiment of said vector, it includes a targeting DNA construct comprising sequences sharing homologies with the region surrounding the genomic site of interest comprising the hybrid DNA target sequence as defined above.

More preferably, said targeting DNA construct comprises:
a) sequences sharing homologies with the region surrounding the genomic site of interest comprising the hybrid DNA target sequence as defined above, and
b) sequences to be introduced flanked by sequence as in a).

Preferably, homologous sequences of at least 50 bp, preferably more than 100 bp and more preferably more than 200 bp are used. Indeed, shared DNA homologies are located in regions flanking upstream and downstream the site of the break and the DNA sequence to be introduced should be located between the two arms. The sequence to be introduced comprises an exogenous gene of interest or a sequence to inactivate or delete a gene or part thereof.

The invention also concerns a prokaryotic or eukaryotic host cell which is modified by a polynucleotide or a vector as defined above, preferably an expression vector.

The invention also concerns a non-human transgenic animal or a transgenic plant, characterized in that all or part of their cells are modified by a polynucleotide or a vector as defined above.

As used herein, a cell refers to a prokaryotic cell, such as a bacterial cell, or eukaryotic cell, such as an animal, plant or yeast cell.

The subject-matter of the present disclosure is further the use of an obligate heterodimer meganuclease/single-chain meganuclease derivative, one or two polynucleotide(s), preferably included in expression vector(s), a cell, a transgenic plant, a non-human transgenic mammal, as defined above, for molecular biology, for *in vivo* or *in vitro* genetic engineering, and for *in vivo* or *in vitro* genome engineering, for non-therapeutic purposes.

Non therapeutic purposes include for example (i) gene targeting of specific loci in cell packaging lines for protein production, (ii) gene targeting of specific loci in crop plants, for strain improvements and metabolic engineering, (iii) targeted recombination for the removal of markers in genetically modified crop plants, (iv) targeted recombination for the removal of markers in genetically modified microorganism strains (for antibiotic production for example).

According to an advantageous embodiment of said use, it is for inducing a double-strand break in a site of interest comprising an hybrid DNA target sequence, thereby inducing a DNA recombination event, a DNA loss or cell death.

According to the disclosure, said double-strand break is for: repairing a specific sequence, modifying a specific sequence, restoring a functional gene in place of a mutated one, attenuating or activating an endogenous gene of interest, introducing a mutation into a site of interest, introducing an exogenous gene or a part thereof, inactivating or detecting an endogenous gene or a part thereof, translocating a chromosomal arm, or leaving the DNA unrepaired and degraded.

The subject-matter of the present disclosure is also a method of genetic engineering, characterized in that it comprises a step of double-strand nucleic acid breaking in a site of interest located on a vector comprising an hybrid DNA target as defined hereabove, by contacting said vector with an obligate heterodimer-meganuclease/single-chain meganuclease derivative as defined above, thereby inducing a homologous recombination with another vector presenting homology with the sequence surrounding the cleavage site of said obligate heterodimer meganuclease.

The subjet-matter of the present disclosure is also a method of genome engineering, characterized in that it comprises the following steps: 1) double-strand breaking a genomic locus comprising at least one hybrid DNA target of an obligate heterodimer meganuclease/single-chain meganuclease derivative as defined above, by contacting said target with said meganuclease ; 2) maintaining said broken genomic locus under conditions appropriate for homologous recombination with a targeting DNA construct comprising the sequence to be introduced in said locus, flanked by sequences sharing homologies with the targeted locus.

The subject-matter of the present disclosure is also a method of genome engineering, characterized in that it comprises the following steps: 1) double-strand breaking a genomic locus comprising at least one hybrid DNA target of an obligate heterodimer meganuclease/single-chain meganuclease derivative as defined above, by contacting said cleavage site with said meganuclease; 2) maintaining said broken genomic locus under conditions appropriate for homologous recombination with chromosomal DNA sharing homologies to regions surrounding the cleavage site.

The subject-matter of the present invention is also the use of at least one obligate heterodimer meganuclease/single-chain meganuclease derivative, one or two polynucleotide(s), preferably included in expression vector(s), as defined above, for the preparation of a medicament for preventing, improving or curing a genetic disease in an individual in need thereof, said medicament being administrated by any means to said individual.

The subject-matter of the present invention is also a method for preventing, improving or curing a genetic disease in an individual in need thereof, said method comprising at least the step of administering to said individual a composition as defined above, by any means.

In this case, the use of the obligate heterodimer meganuclease/single-chain meganuclease derivative as defined above, comprises at least the step of (a) inducing in somatic tissue(s) of the individual a double stranded cleavage at a site of interest of a gene comprising at least one recognition and cleavage site of said meganuclease, and (b) introducing into the individual a targeting DNA, wherein said targeting DNA comprises (1) DNA sharing homologies to the region surrounding the cleavage site and (2) DNA which repairs the site of interest upon recombination between the targeting DNA and the chromosomal DNA. The targeting DNA is introduced into the individual under conditions appropriate for introduction of the targeting DNA into the site of interest.

According to the present invention, said double-stranded cleavage is induced, either in *toto* by administration of said meganuclease to an individual, or ex vivo by introduction of said meganuclease into somatic cells removed from an individual and returned into the individual after modification.

In a preferred embodiment of said use, the meganuclease is combined with a targeting DNA construct comprising a sequence which repairs a mutation in the gene flanked by sequences sharing homologies with the regions of the gene surrounding the genomic DNA cleavage site of said meganuclease, as defined above. The sequence which repairs the mutation is either a fragment of the gene with the correct sequence or an exon knock-in construct.

For correcting a gene, cleavage of the gene occurs in the vicinity of the mutation, preferably, within 500 bp of the mutation. The targeting construct comprises a gene fragment which has at least 200 bp of homologous sequence flanking the genomic DNA cleavage site (minimal repair matrix) for repairing the cleavage, and includes the correct sequence of the gene for repairing the mutation. Consequently, the targeting construct for gene correction comprises or consists of the minimal repair matrix; it is preferably from 200 pb to 6000 pb, more preferably from 1000 pb to 2000 pb.

For restoring a functional gene, cleavage of the gene occurs upstream of a mutation. Preferably said mutation is the first known mutation in the sequence of the gene, so that all the downstream mutations of the gene can be corrected simultaneously. The targeting construct comprises the exons downstream of the genomic DNA cleavage site fused in frame (as in the cDNA) and with a polyadenylation site to stop transcription in 3'. The sequence to be introduced (exon knock-in construct) is flanked by introns or exons sequences surrounding the cleavage site, so as to allow the transcription of the engineered gene (exon knock-in gene) into a mRNA able to code for a functional protein. For example, the exon knock-in construct is flanked by sequences upstream and downstream.

The subject-matter of the present invention is also the use of at least one obligate heterodimer meganuclease/single-chain meganuclease derivative, one or or two polynucleotide(s), preferably included in expression vector(s), as defined above for the preparation of a medicament for preventing, improving or curing a disease caused by an infectious agent that presents a DNA intermediate, in an individual in need thereof, said medicament being administrated by any means to said individual.

The subject-matter of the present invention is also a method for preventing, improving or curing a disease caused by an infectious agent that presents a DNA intermediate, in an individual in need thereof, said method comprising at least the step of administering to said individual a composition as defined above, by any means.

The subject-matter of the present invention is also the use of at least one obligate heterodimer meganuclease/single-chain meganuclease derivative, one or two polynucleotide(s), preferably included in expression vector(s), as defined above, *in vitro,* for inhibiting the propagation, inactivating or deleting an infectious agent that presents a DNA intermediate, in biological derived products or products intended for biological uses or for disinfecting an object.

The subject-matter of the present invention is also a method for decontaminating a product or a material from an infectious agent that presents a DNA intermediate, said method comprising at least the step of contacting a biological derived product, a product intended for biological use or an object, with a composition as defined above, for a time sufficient to inhibit the propagation, inactivate or delete said infectious agent.

In a particular embodiment, said infectious agent is a virus. For example said virus is an adenovirus (Ad11, Ad21), herpesvirus (HSV, VZV, EBV, CMV, herpesvirus 6, 7 or 8), hepadnavirus (HBV), papovavirus (HPV), poxvirus or retrovirus (HTLV, HIV).

The subject-matter of the present invention is also a composition characterized in that it comprises at least one obligate heterodimer meganuclease, one or two polynucleotide(s), preferably included in expression vector(s), as defined above.

In a preferred embodiment of said composition, it comprises a targeting DNA construct comprising the sequence which repairs the site of interest flanked by sequences sharing homologies with the targeted locus as defined above. Preferably, said targeting DNA construct is either included in a recombinant vector or it is included in an expression vector comprising the polynucleotide(s) encoding the meganuclease, as defined in the present invention.

The subject-matter of the present invention is also products containing at least a meganuclease or one or two expression vector(s) encoding said meganuclease, and a vector including a targeting construct, as defined above, as a combined preparation for simultaneous, separate or sequential use in the prevention or the treatment of a genetic disease.

For purposes of therapy, the meganuclease and a pharmaceutically acceptable excipient are administered in a therapeutically effective amount. Such a combination is said to be administered in a "therapeutically effective amount" if the amount administered is physiologically significant. An agent is physiologically significant if its presence results in a detectable change in the physiology of the recipient. In the present context, an agent is physiologically significant if its presence results in a decrease in the severity of one or more symptoms of the targeted disease and in a genome correction of the lesion or abnormality.

In one embodiment of the uses according to the present invention, the meganuclease is substantially non-immunogenic, i.e., engenders little or no adverse immunological response. A variety of methods for ameliorating or eliminating deleterious immunological reactions of this sort can be used in accordance with the invention. In a preferred embodiment, the meganuclease is substantially free of N-formyl methionine. Another way to avoid unwanted immunological reactions is to conjugate meganucleases to polyethylene glycol ("PEG") or polypropylene glycol ("PPG") (preferably of 500 to 20,000 daltons average molecular weight (MW)). Conjugation with PEG or PPG, as described by Davis et al. (US 4,179,337) for example, can provide non-immunogenic, physiologically active, water soluble endonuclease conjugates with anti-viral activity. Similar methods also using a polyethylene-polypropylene glycol copolymer are described in Saifer et al. (US 5,006,333).

The meganuclease can be used either as a polypeptide or as a polynucleotide construct/vector encoding said polypeptide. It is introduced into cells, *in vitro, ex vivo* or *in vivo,* by any convenient means well-known to those in the art, which are appropriate for the particular cell type, alone or in association with either at least an appropriate vehicle or carrier and/or with the targeting DNA. Once in a cell, the meganuclease and if present, the vector comprising targeting DNA and/or nucleic acid encoding a meganuclease are imported or translocated by the cell from the cytoplasm to the site of action in the nucleus.

The obligate heterodimer meganuclease/single-chain meganuclease derivative (polypeptide) may be advantageously associated with: liposomes, polyethyleneimine (PEI), and/or membrane translocating peptides (Bonetta, The Scientist, 2002, 16, 38; Ford et al., Gene Ther., 2001, 8, 1-4 ; Wadia and Dowdy, Curr. Opin. Biotechnol., 2002, 13, 52-56); in the latter case, the sequence of the meganuclease fused with the sequence of a membrane translocating peptide (fusion protein).

Vectors comprising targeting DNA and/or nucleic acid encoding a meganuclease can be introduced into a cell by a variety of methods (e.g., injection, direct uptake, projectile bombardment, liposomes, electroporation). Meganucleases can be stably or transiently expressed into cells using expression vectors. Techniques of expression in eukaryotic cells are well known to those in the art. (See Current Protocols in Human Genetics: Chapter 12 "Vectors For Gene Therapy" & Chapter 13 "Delivery Systems for Gene Therapy"). Optionally, it may be preferable to incorporate a nuclear localization signal into the recombinant protein to be sure that it is expressed within the nucleus.

The uses of the obligate heterodimer meganuclease/single-chain meganuclease derivative and the methods of using said meganucleases according to the present invention include also the use of the polynucleotide(s), vector(s), cell, transgenic plant or non-human transgenic mammal encoding said obligate heterodimer meganuclease, as defined above.

According to another advantageous embodiment of the uses and methods according to the present invention, said obligate heterodimer meganuclease/single-chain meganuclease derivative, polynucleotide(s), vector(s), cell, transgenic plant or non-human transgenic mammal are associated with a targeting DNA construct as defined above. Preferably, said vector encoding the monomer(s) of the obligate heterodimer meganuclease/single-chain meganuclease derivative, comprises the targeting DNA construct, as defined above.

The obligate heterodimer meganuclease according to the invention is derived from the monomers of the parent homodimeric LAGLIDADG endonucleases, according to standard site-directed mutagenesis methods which are well-known in the art and commercially available. They may be advantageously produced by amplifying overlapping fragments comprising each of the mutated positions, as defined above, according to well-known overlapping PCR techniques.

This may be achieved by using complementary primer sets. For example the three pairs SEQ ID NO: 3 and SEQ ID NO: 4, SEQ ID NO: 5 and SEQ ID NO: 6, and SEQ ID NO: 7 or SEQ ID NO: 35 and SEQ ID NO: 8 may be used for amplifying the coding sequence (CDS) of monomer A and the three pairs SEQ ID NO: 9 and SEQ ID NO: 1 0; SEQ ID NO: 11 and SEQ ID NO: 12; SEQ ID NO: 13 or SEQ ID NO: 36 and SEQ ID NO: 14 may be used for amplifying the CDS of monomer B.

Alternatively the two pairs SEQ ID NO: 37 and SEQ ID NO: 41, SEQ ID NO: 40 and SEQ ID NO: 42 may be used for amplifying the CDS of monomer A and the two pairs SEQ ID NO: 37 and SEQ ID NO: 38, SEQ ID NO: 39 and SEQ ID NO: 40 may be used for amplifying the CDS of monomer B.

The monomers of the parent homodimeric LAGLIDADG endonucleases may be obtained by a method for engineering variants able to cleave a genomic DNA target sequence of interest, as described previously in *Smith et al.,* Nucleic Acids Res., 2006, 34, e 149, said method comprising at least the steps of:
(a) constructing a first series of I*-Cre*I variants having at least one substitution in a first functional subdomain of the LAGLIDADG core domain situated from positions 26 to 40 of I-*Cre*I,
(b) constructing a second series of I-*Cre*I variants having at least one substitution in a second functional subdomain of the LAGLIDADG core domain situated from positions 44 to 77 of I-*Cre*I,
(c) selecting and/or screening the variants from the first series of step (a) which are able to cleave a mutant I*-Cre*I site wherein (i) the nucleotide triplet at positions -10 to -8 of the I*-Cre*I site has been replaced with the nucleotide triplet which is present at positions -10 to -8 of said genomic target and (ii) the nucleotide triplet at positions +8 to +10 has been replaced with the reverse complementary sequence of the nucleotide triplet which is present at positions -10 to -8 of said genomic target,
(d) selecting and/or screening the variants from the second series of step (b) which are able to cleave a mutant I*-Cre*I site wherein (i) the nucleotide triplet at positions -5 to -3 of the I-*Cre*I site has been replaced with the nucleotide triplet which is present at positions -5 to -3 of said genomic target and (ii) the nucleotide triplet at positions +3 to +5 has been replaced with the reverse complementary sequence of the nucleotide triplet which is present at positions -5 to -3 of said genomic target,
(e) selecting and/or screening the variants from the first series of step (a) which are able to cleave a mutant I*-Cre*I site wherein (i) the nucleotide triplet at positions +8 to +10 of the I*-Cre*I site has been replaced with the nucleotide triplet which is present at positions +8 to +10 of said genomic target and (ii) the nucleotide triplet at positions -10 to -8 has been replaced with the reverse complementary sequence of the nucleotide triplet which is present at positions +8 to +10 of said genomic target,
(f) selecting and/or screening the variants from the second series of step (b) which are able to cleave a mutant I*-Cre*I site wherein (i) the nucleotide triplet at positions +3 to +5 of the I*-Cre*I site has been replaced with the nucleotide triplet which is present at positions +3 to +5 of said genomic target and (ii) the nucleotide triplet at positions -5 to -3 has been replaced with the reverse complementary sequence of the nucleotide triplet which is present at positions +3 to +5 of said genomic target,
(g) combining in a single variant, the mutation(s) at positions 26 to 40 and 44 to 77 of two variants from step (c) and step (d), to obtain a novel homodimeric I*-Cre*I variant which cleaves a sequence wherein (i) the nucleotide triplet at positions -10 to -8 is identical to the nucleotide triplet which is present at positions -10 to -8 of said genomic target, (ii) the nucleotide triplet at positions +8 to +10 is identical to the reverse complementary sequence of the nucleotide triplet which is present at positions -10 to -8 of said genomic target, (iii) the nucleotide triplet at positions -5 to -3 is identical to the nucleotide triplet which is present at positions -5 to -3 of said genomic target and (iv) the nucleotide triplet at positions +3 to +5 is identical to the reverse complementary sequence of the nucleotide triplet which is present at positions -5 to -3 of said genomic target,
(h) combining in a single variant, the mutation(s) at positions 26 to 40 and 44 to 77 of two variants from step (e) and step (f), to obtain a novel homodimeric I*-Cre*I variant which cleaves a sequence wherein (i) the nucleotide triplet at positions +3 to +5 is identical to the nucleotide triplet which is present at positions +3 to +5 of said genomic target, (ii) the nucleotide triplet at positions -5 to -3 is identical to the reverse complementary sequence of the nucleotide triplet which is present at positions +3 to +5 of said genomic target, (iii) the nucleotide triplet at positions +8 to +10 of the I*-Cre*I site has been replaced with the nucleotide triplet which is present at positions +8 to +10 of said genomic target and (iv) the nucleotide triplet at positions -10 to -8 is identical to the reverse complementary sequence of the nucleotide triplet at positions +8 to +10 of said genomic target,
(i) combining the variants obtained in steps (g) and (h) to form heterodimers, and
(j) selecting and/or screening the heterodimers from step (i) which are able to cleave said genomic DNA target situated in a mammalian gene.

Steps (a) and (b) may comprise the introduction of additional mutations in order to improve the binding and/or cleavage properties of the mutants, particularly at other positions contacting the DNA target sequence or interacting directly or indirectly with said DNA target. These steps may be performed by generating combinatorial libraries as described in the International PCT Application WO 2004/067736 and Arnould et al. (J. Mol. Biol., 2006, 355, 443-458).

The selection and/or screening in steps (c), (d), (e), (f) and/or (j) may be performed by using a cleavage assay *in vitro* or *in vivo,* as described in the International PCT Application WO 2004/067736, Epinat et al. (Nucleic Acids Res., 2003, 31, 2952-2962), Chames et al. (Nucleic Acids Res., 2005, 33, e178), and Arnould et al. (J. Mol. Biol., 2006, 355, 443-458). Preferably, steps (c), (d), (e), (f) and/or (j) are performed *in vivo,* under conditions where the double-strand break in the mutated DNA target sequence which is generated by said variant leads to the activation of a positive selection marker or a reporter gene, or the inactivation of a negative selection marker or a reporter gene, by recombination-mediated repair of said DNA double-strand break, as described in the International PCT Application WO 2004/067736, Epinat et al. (Nucleic Acids Res., 2003, 31, 2952-2962), Chames et al. (Nucleic Acids Res., 2005, 33, e178), and Arnould et al. (J. Mol. Biol., 2006, 355, 443-458).

The (intramolecular) combination of mutations in steps (g) and (h) may be performed by amplifying overlapping fragments comprising each of the two subdomains, according to well-known overlapping PCR techniques.

In addition, step (g) and/or (h) may further comprise the introduction of random mutations on the whole variant or in a part of the variant, in particular the C-terminal half of the variant (positions 80 to 163). This may be performed by generating random mutagenesis libraries on a pool of variants, according to standard mutagenesis methods which are well-known in the art and commercially available.

The (intermolecular) combination of the variants in step (i) is performed by co-expressing one variant from step (g) with one variant from step (h), so as to allow the formation of heterodimers. For example, host cells may be modified by one or two recombinant expression vector(s) encoding said variant(s). The cells are then cultured under conditions allowing the expression of the variant(s), so that heterodimers are formed in the host cells, as described previously in the International PCT Application WO 2006/097854 and Arnould et al. (J. Mol. Biol., 2006, 355, 443-458).

In this case the mutations of monomers A as defined in the present invention, are introduced in one monomer of the heterodimer obtained in step (j) and the mutations of monomer B as defined in the present invention, are introduced in the other monomer of said heterodimer.

Alternatively, the obligate heterodimer meganuclease of the invention may be obtained by a method derived from the hereabove method of engineering meganuclease variants, by introducing the following modifications:
- step (a) and step (b) are performed on two types of initial scaffold proteins: a first I*-Cre*I scaffold having the mutations of monomer A and a second I-CreI scaffold having the mutations of monomer B, and
- the selection/screening of steps (c) to (f) is performed by transforming the library of variants having the mutations of monomer A or B as defined above in a host cell that expresses a I*-Cre*I mutant having the corresponding mutations (from monomer B or A, respectively) to allow the formation of heterodimers and selecting the functional heteodimeric variants by using a non-palindromic DNA target wherein one half of the I*-Cre*I site is modified at positions ± 3 to 5 or ± 8 to 10 and the other half is not modified.

The steps (g) and (h) are performed by combining in a single variant, the mutations of two variants derived from the same monomer (A) or (B).

Step (i) is performed by combining the variants derived from one of the monomers (A or B), obtained in step (g) with the variants derived from the other monomer, obtained in step (h) to form heterodimers.

Single-chain meganucleases able to cleave a DNA target from the gene of interest are derived from the obligate heterodimer meganuclease according to the invention by methods well-known in the art (Epinat et al., Nucleic Acids Res., 2003, 31, 2952-62; Chevalier et al., Mol. Cell., 2002, 10, 895-905; Steuer et al., Chembiochem., 2004, 5, 206-13; International PCT Applications WO 03/078619 and WO 2004/031346). Any of such methods, may be applied for constructing the single-chain meganuclease of the invention.

The polynucleotide sequence(s) encoding the monomers A and B as defined in the present invention may be prepared by any method known by the man skilled in the art. For example, they are amplified from a cDNA template, by polymerase chain reaction with specific primers. Preferably the codons of said cDNA are chosen to favour the expression of said protein in the desired expression system.

The recombinant vector comprising said polynucleotides may be obtained and introduced in a host cell by the well-known recombinant DNA and genetic engineering techniques.

The obligate heterodimer meganuclease of the invention is produced by co-expressing the monomers A and B as defined above, in a host cell or a transgenic animal/plant modified modified by one or two expression vector(s), under conditions suitable for the co-expression of the monomers, and the obligate heterodimer meganuclease is recovered from the host cell culture or from the transgenic animal/plant, by any appropriate means.

The single-chain meganuclease of the invention is produced by expressing a fusion protein comprising the monomers A and B as defined above, in a host cell or a transgenic animal/plant modified modified by one expression vector, under conditions suitable for the expression of said fusion protein, and the single-chain meganuclease is recovered from the host cell culture or from the transgenic animal/plant, by any appropriate means.

The subject-matter of the present invention is also the use of at least one obligate heterodimer meganuclease/single-chain meganuclease derivative, as defined above, as a scaffold for making other meganucleases. For example a third round of mutagenesis and selection/screening can be performed on the monomers, for the purpose of making novel, third generation homing endonucleases.

The practice of the present invention will employ, unless otherwise indicated, conventional techniques of cell biology, cell culture, molecular biology, transgenic biology, microbiology, recombinant DNA, and immunology, which are within the skill of the art. Such techniques are explained fully in the literature. See, for example, Current Protocols in Molecular Biology (Frederick M. AUSUBEL, 2000, Wiley and son Inc, Library of Congress, USA); Molecular Cloning: A Laboratory Manual, Third Edition, (Sambrook et al, 2001, Cold Spring Harbor, New York: Cold Spring Harbor Laboratory Press); Oligonucleotide Synthesis (M. J. Gait ed., 1984); Mullis et al. U.S. Pat. No. 4,683,195; Nucleic Acid Hybridization (B. D. Harries & S. J. Higgins eds. 1984); Transcription And Translation (B. D. Hames & S. J. Higgins eds. 1984); Culture Of Animal Cells (R. I. Freshney, Alan R. Liss, Inc., 1987); Immobilized Cells And Enzymes (IRL Press, 1986); B. Perbal, A Practical Guide To Molecular Cloning (1984); the series, Methods In ENZYMOLOGY (J. Abelson and M. Simon, eds.-in-chief, Academic Press, Inc., New York), specifically, Vols. 154 and 155 (Wu et al. eds.) and Vol. 185, "Gene Expression Technology" (D. Goeddel, ed.); Gene Transfer Vectors For Mammalian Cells (J. H. Miller and M. P. Calos eds., 1987, Cold Spring Harbor Laboratory); Immunochemical Methods In Cell And Molecular Biology (Mayer and Walker, eds., Academic Press, London, 1987); Handbook Of Experimental Immunology, Volumes I-IV (D. M. Weir and C. C. Blackwell, eds., 1986); and Manipulating the Mouse Embryo, (Cold Spring Harbor Laboratory Press, Cold Spring Harbor, N.Y., 1986).

In addition to the preceding features, the invention further comprises other features which will emerge from the description which follows, which refers to examples illustrating the obligate heterodimer meganuclease according to the invention, as well as to the appended drawings in which:
- figure 1 represents a side view of the structure of the complex of meganuclease I-Cre-I (PDB:1G9Y) with its target DNA template. (A), (B), (C) Details of three modifiable interaction patches between the two monomers on the homodimer. (D) Designed heterodimeric interfaces, showing amino acid changes at each relevant position in the protein.

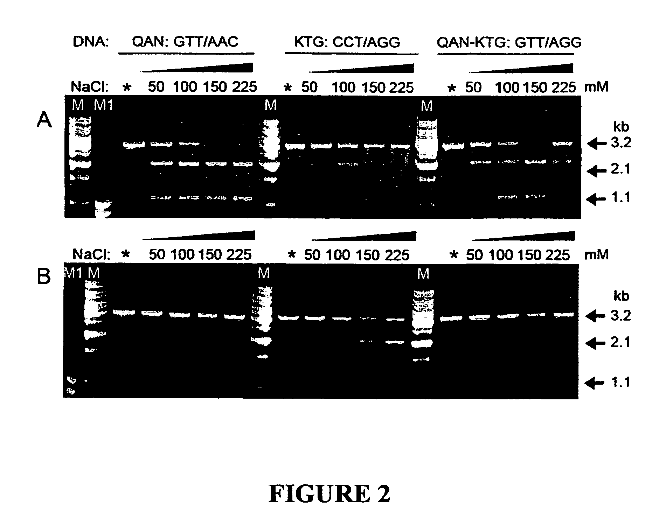

- figure 2 illustrates the effect of high salt concentration on the cleavage specificity. (A) QAN or (B) KTG protein was incubated with either the QAN homodimer site (GTT/AAC), the KTG homodimer DNA site (CCT/AGG), or a hybrid site QAN/KTG site (GTT/AGG), varying the concentration of NaCl between 50 and 225 mM. Arrows indicate the the uncut target DNA (3.2 kb) or the two bands resulting from digestion (1.1 and 2.1 kb). An asterisk (*) marks control lanes with DNA alone. 1 kb and 100 bp ladders (FERMENTAS) are marked by M and M1, respectively.

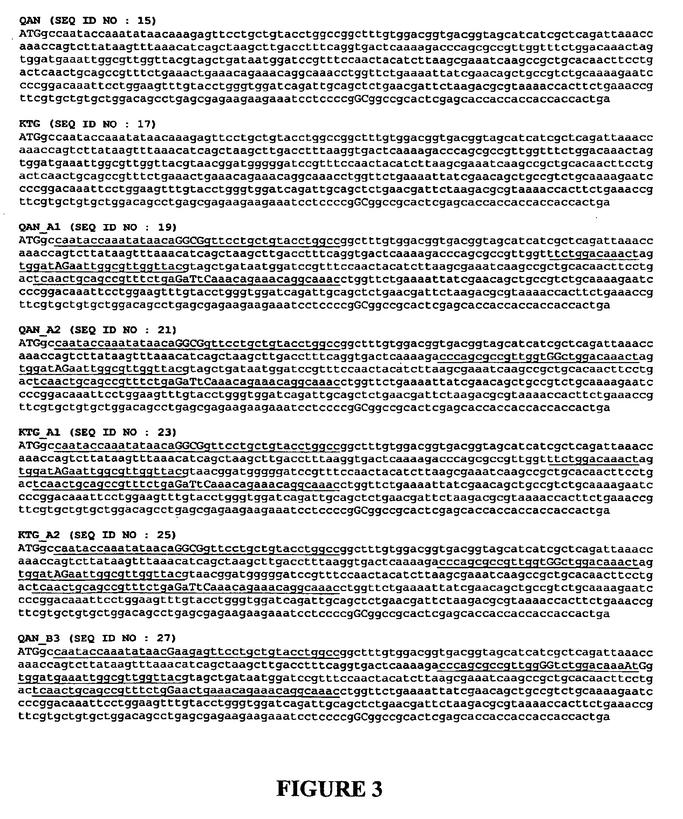

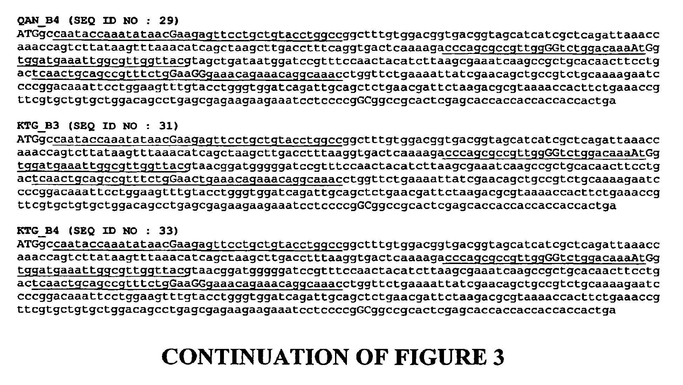

- figure 3 illustrates the CDS of the mutants derived from the I*-Cre*I variants QAN and KTG designed for the making of the obligate heterodimer meganucleases. The sequences of the three mutagenizing oligos are underlined. QAN-A1 and KTG-A1 mutants : oligos SEQ ID NO: 3, 35 and 5 respectively; QAN-A2 and KTG-A2 mutants : oligos SEQ ID NO: 3, 7 and 5 respectively; QAN-B3 and KTG-B3 mutants : oligos SEQ ID NO: 9, 11 and 13 respectively; QAN-B4 and KTG-B4 mutants : oligos SEQ ID NO: 9, 11 and 36 respectively.

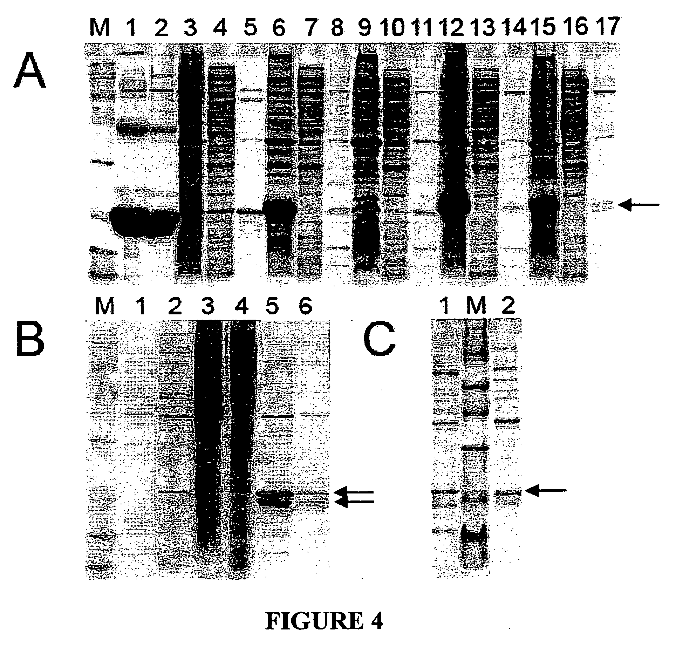

- figure 4 illustrates the expression and purification of the designed meganucleases. The target protein bands are marked by arrows. (A) Wild-type homodimers and mutant monomers. Lanes: M = Standard Broad range markers (BIORAD); 1. Purified QANwt; 2. Purified KTGwt; 3. Pellet QAN-A1; 4. Supernatant QAN-A1; 5. Purified QAN-A1; 6. Pellet KTG-B3; 7. Supernatant KTG-B3; 8. Purified KTG-B3; 9. Pellet KTG-A2; 10. Supernatant KTG-A2; 11. Purified KTG-A2; 12. Pellet QAN-B3; 13. Supernatant QAN-B3; 14. Purified QAN-B3; 15. Pellet QAN-B4; 16. Supernatant QAN-B4; 17. Purified QAN-B4. (B) Co-expression and purification of KTG-A2/QAN-B3. Lanes: 1. Uninduced; 2. Induced; 3. Pellet; 4. Supernatant; 5. Purification before dialysis; 6. Purified after dialysis. (C) Co-expression of the other two designs: Lane 1. Two bands are visible, corresponding to the heterodimer QAN-A1/KTG-B3; Lane 2. Only one band is visible, indicating that QAN-B4/KTG-A2 does not make a heterodimer.

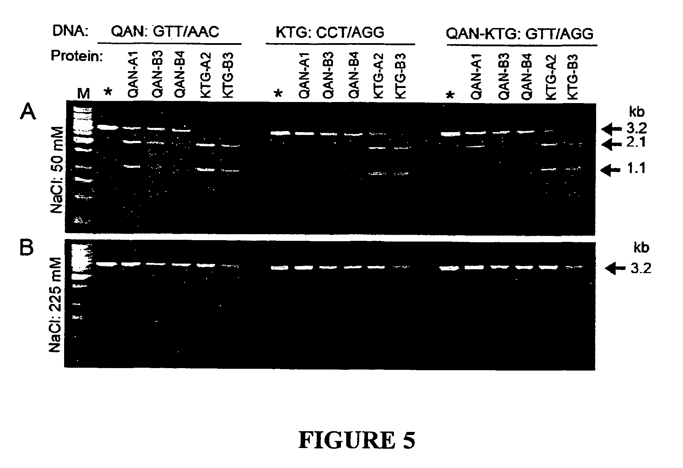

- figure 5 illustrates non-specific DNA cleavage and non-cleavage by singly-expressed designed meganuclease monomer variants under different salt conditions. Approximately 3.75 µM of each purified protein was incubated with 34 nM of purified plasmid (pre-linearized with *Xmn*I*),* containing either the QAN homodimer site (GTT/AAC), the KTG homodimer DNA site (CCT/AGG), or a hybrid site QAN/KTG site (Q-K: GTT/AGG). The concentration of NaCl was either (A) 50 mM or (B) 225 mM. Arrows indicate the uncut target DNA (3.2 kb) or the two bands resulting from digestion (1.1 and 2.1 kb). An asterisk (*) marks control lanes with DNA alone. 1 kb ladders (FERMENTAS) are marked by M.

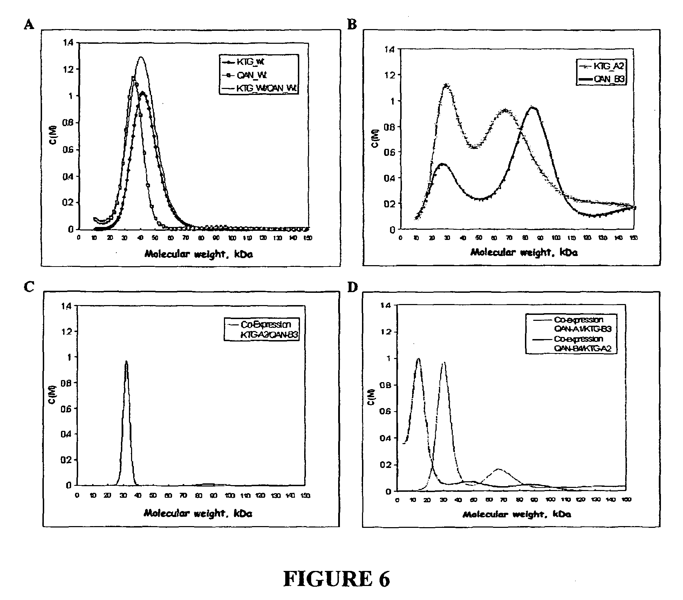

- figure 6 illustrates analytical ultracentrifugation of the different meganucleases. (A).The wild-type monomers form homodimers of about 400 kDa (KTG-wt; QAN-wt). (B) The designed non-homodimerising KTG-A2 and QAN-B3 form aggregates when expressed individually. (C) The co-expressed KTG-A2 and QAN-B3 form a perfect heterodimer. (D) The co-expressed QAN-B4 and KTG-A2 also form a heterodimer, to an extent, while QAN-A1 and KTG-B3 do not.

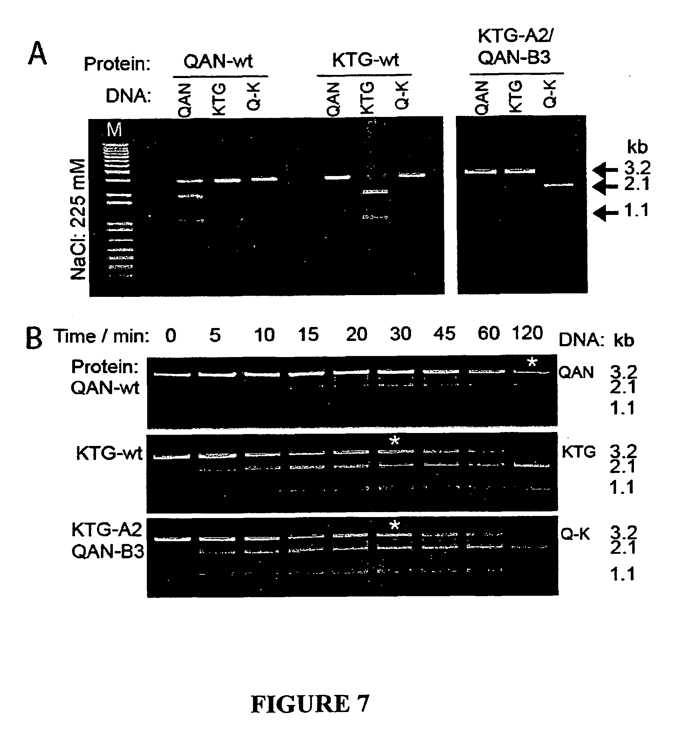

- figure 7 illustrates specific DNA cleavage by co-expressed wild-type (wt) and designed obligate heterodimer KTG-A2--QAN-B3 meganucleases. (A) Purified proteins were incubated with 3 nM of purified plasmid (pre-linearized with *Xmn*I*),* containing either the QAN homodimer site (GTT/AAC), the KTG homodimer DNA site (CCT/AGG), or a hybrid site QAN/KTG site (Q-K: GTT/AGG). Because different constructs have different reaction optima, homodimers were used at 0.25 µM concentration (4 h, 37°C), while heterodimer was used at0.50 µM concentration (30 min, 37°C). NaCl concentration was at 225 mM. Arrows indicate the uncut target DNA (3.2 kb) or the two bands resulting from digestion (1.1 and 2.1 kb). 1 kb ladders (Fermentas) are marked by M. (B) The relative activities of each enzyme sample were compared in a time-course experiment against their optimal DNA sites, using 1 µM protein and 6 nM purified plasmid target. White asterisks mark the positions of the samples estimated to be closest to having 50 % cleavage.

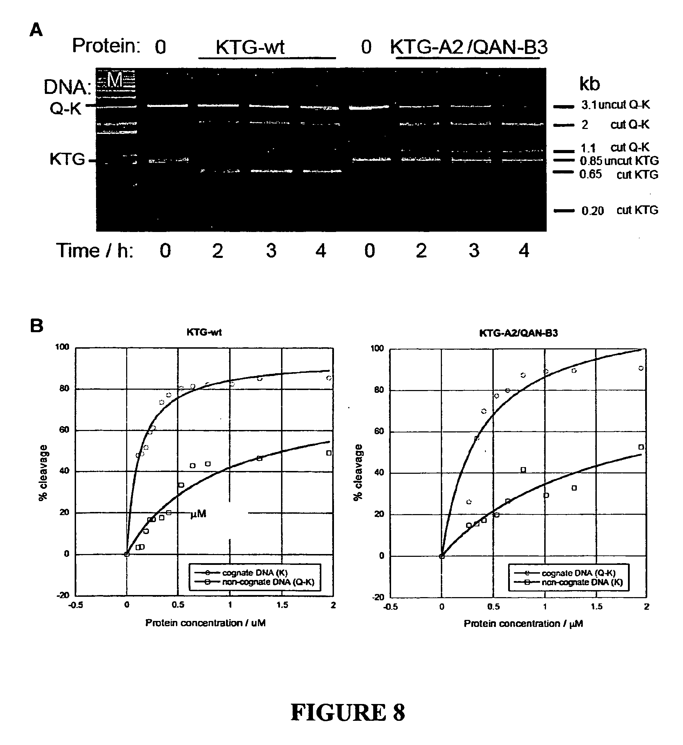

- figure 8 illustrates a competition assays to determine the relative specificity of KTG-wt homodimer and KTG-A2/QAN-B3 heterodimer proteins when exposed to equimolar mixtures of Q-K heterodimer DNA site (3.1 kb PCR product) and KTG homodimer DNA site (0.85 kb PCR product). Each target DNA was used at 5 nM final concentration. The different DNA targets are characteristic sizes and give specifically-sized cleavage products. (A) Time course experiment showing the relative specific and non-specific cutting by KTG and KTG-A2/QAN-B3 enzymes. Final protein concentration = 1 µM. (B) Enzyme titration assay to determine the difference in protein concentration for 50 % cleavage of cognate and non-cogate DNA target sites by each enzyme. Equimolar amounts of DNA target site PCR products (Q-K and KTG) were mixed and incubated against a dilution series of each enzyme, as indicated, for a 1 hour incubation. Gels were scanned and analysed with ImageJ and Kaleidagraph.

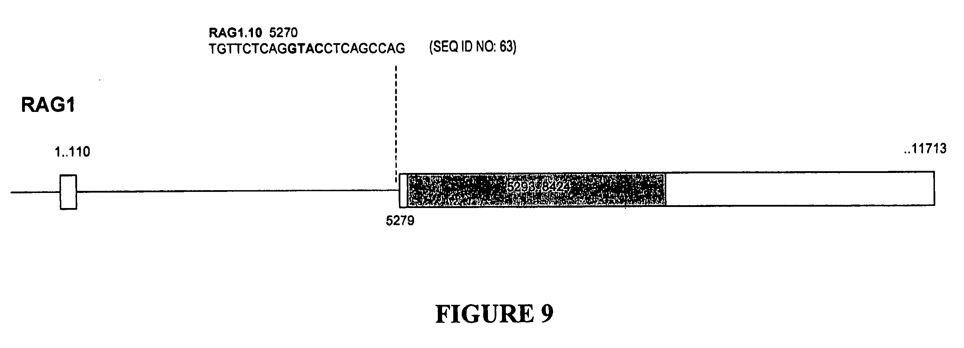

- figure 9 is a schematic representation of the human RAG1 gene (GenBank accession number NC_000011). Exonic sequences are boxed, and the Exon-Intron junctions are indicated. ORF is indicated as a grey box. The RAG1.10 sequence is indicated with its sequence and position.

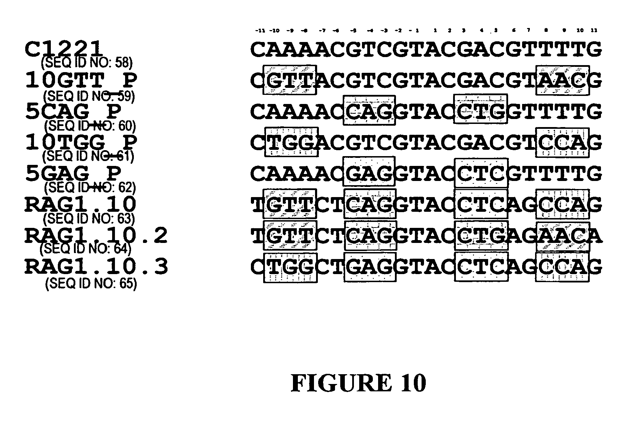

- figure 10 represents 22 bp DNA targets cleaved by I-*Cre*I or some of its derived variants (SEQ ID NO: 58 to 65, respectively). C1221 is the I*-Cre*I target. 10GTT_P, 5CAG_P, 10TGG_P and 5GAG_P are palindromic targets, which differ from C1221 by the boxed motifs. RAG1.10 is the RAG1 target, RAG1.10.2 and RAG1.10.3 are palindromic targets, which are derived from the left and the right part of RAG1.10, respectively. As shown in the Figure, the boxed motifs from 10GTT_P, 5CAG_P, 10TGG_P and 5GAG_P are found in the RAG 1.10 target.

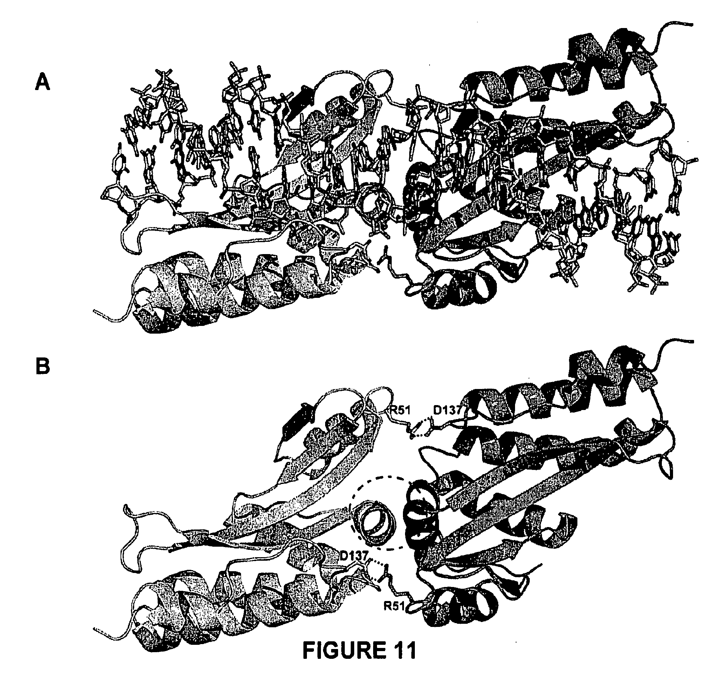

- figure 11 represents a bottom view of the structure of the complex of meganuclease I-Cre-I bound to DNA (PDB:1G9Y). A. View of the complex showing the target DNA template. B. The same view is represented but the DNA has been omitted. For each monomer the two residues R51 and D137 are represented in sticks and hydrogen bonds are represented by dashed lines. The circle in dashed lines delimits the active site, where the two DNA strands are cleaved.

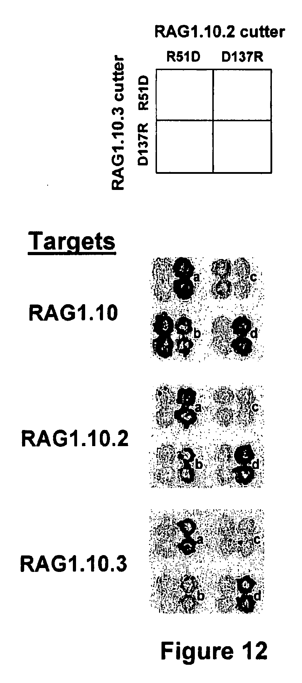

- figure 12 illustrates the cleavage of RAG1.10 by heterodimeric combinatorial mutants. The figure displays secondary screening of combinations of mutants of RAG1.10.2 and RAG1.10.3 cutters with the RAG 1.10, RAG1.10.2 and RAG1.10.3 targets. The experiment format is an 2 by 2 dots format. The two dots forming the left column are the mutants and the right column is a control used to assess the quality of the experiment. I*-Sce*I against an I*-Sce*I target is in a and d, a low activity form of I-*Sce*I is in b and the empty vector is in c.

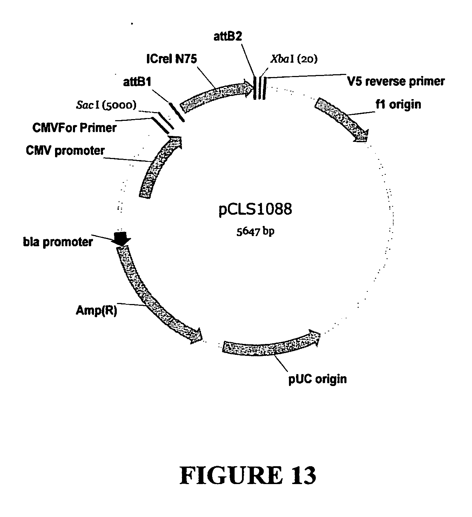

- figure 13 represents the map of pCLS1088, a plasmid for expression of meganucleases in mammalian cells.

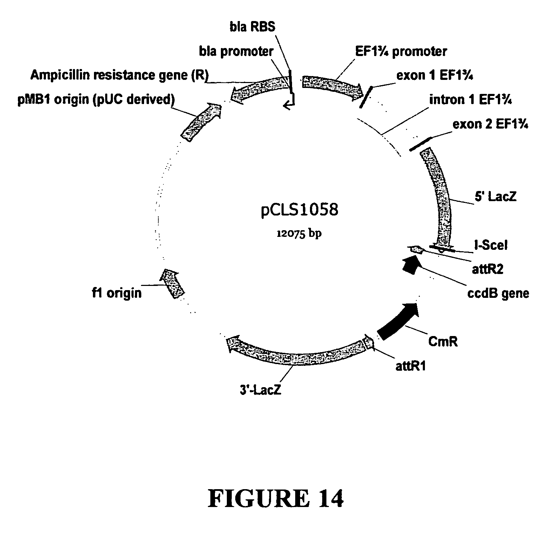

- figure 14: represents the pCLS1058 reporter vector map. The reporter vector is marked with blasticidine and ampicilline resistance genes. The LacZ tandem repeats share 800 bp of homology, and are separated by 1.3 kb of DNA. They are surrounded by EF1-alpha promoter and terminator sequences. Target sites are cloned using the Gateway protocol (INVITROGEN), resulting in the replacement of the CmR and ccdB genes with the chosen target site.

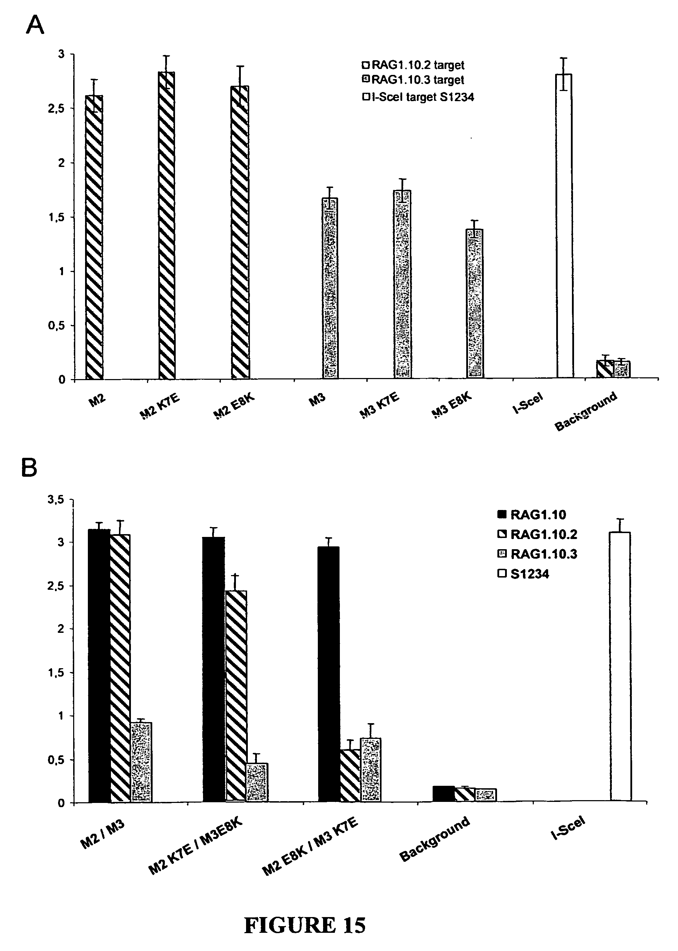

- figure 15 illustrates the activity of the homodimers and heterodimers against the three RAG1.10 targets as monitored in an extrachromosomal assay in CHO cells. A. Cleavage of the palindromic RAG1.10.2 and RAG1.10.3 targets by respectively the M2, M2 K7E, M2 E8K and M3, M3 K7E, M3 E8K homodimer. Background corresponds to the transfection of the cells with an empty expression vector. Cleavage of the S1234 I*-Sce*I target by I*-Sce*I in the same experiment is shown as a positive control. B. Activity of three RAG1.10 heterodimers against the three RAG 1.10 targets. Controls are the same than those described in A.

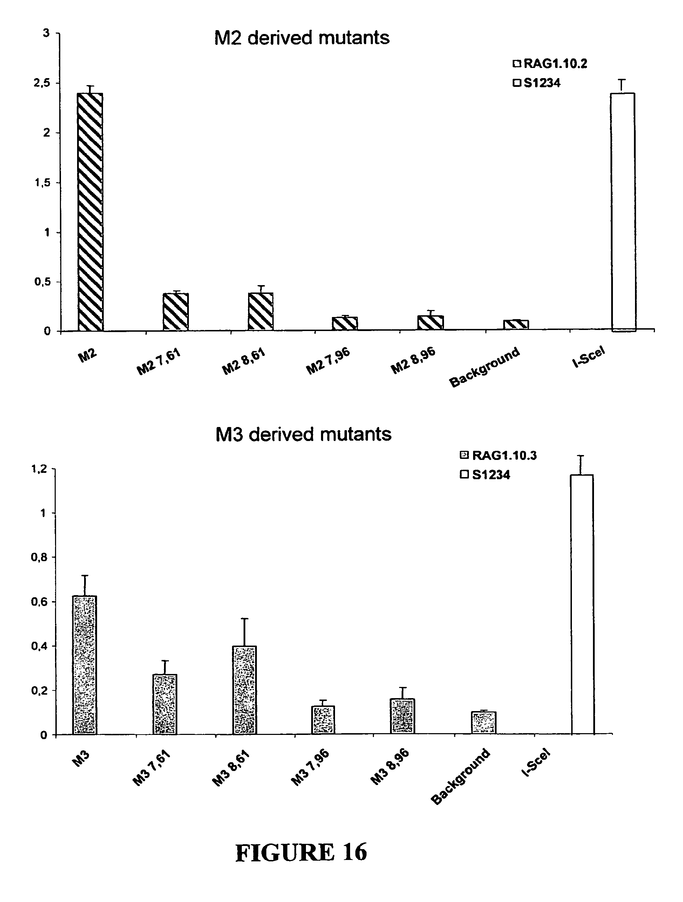

- figure 16 illustrates the homodimeric activity of the M2 and M3 derived mutants against respectively the RAG 1.10.2 and RAG 1.10.3 target. A double mutant is designed by the positions where it has been mutated: for example, M2 7,61 stands for M2 K7E E61R. As in Figure 15A, the background corresponds to the transfection of the cells with an empty expression vector and the cleavage of the S 1234 I*-Sce*I target by I*-Sce*I in the same experiment is shown as a positive control.

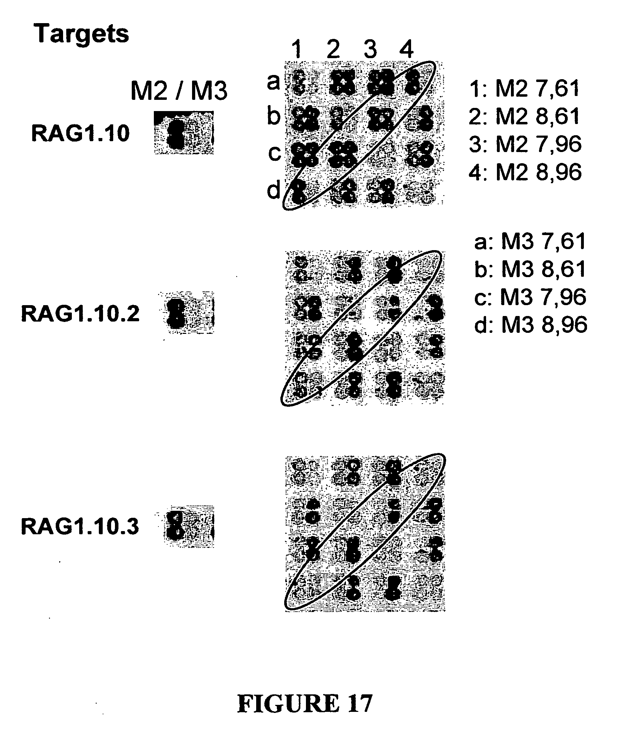

- figure 17 illustrates the screening in yeast of the 16 heterodimers obtained by co-expression of the four double mutated M2 mutants with the four double mutated M3 mutants against the three RAG1.10 targets. Activity of the initial M2 / M3 heterodimer against the three targets is shown as control. For each four dots yeast cluster, the two left dots are the result of the experiment, while the two right dots are various internal controls to assess the experiment quality and validity. The red ellipsoid indicates the four obligatory heterodimers.

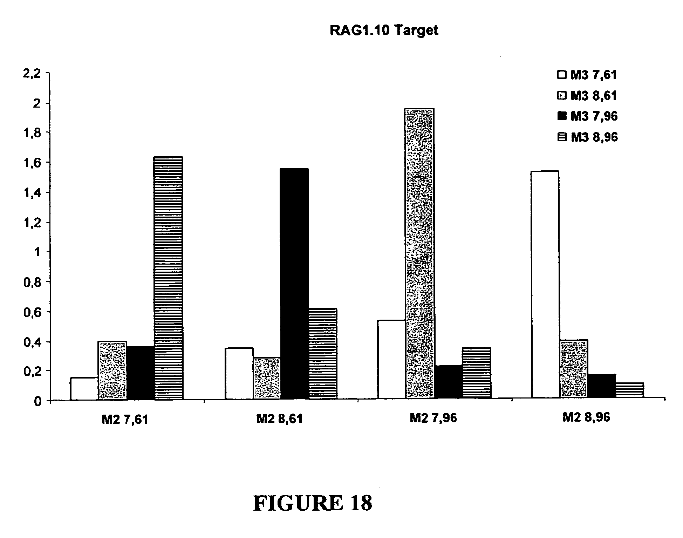

- figure 18 illustrates the activity of the 16 heterodimers obtained by co-expression of the four double mutated M2 mutants with the four double mutated M3 mutants against the RAG 1.10 target in an extrachromosomal assay in CHO cells.

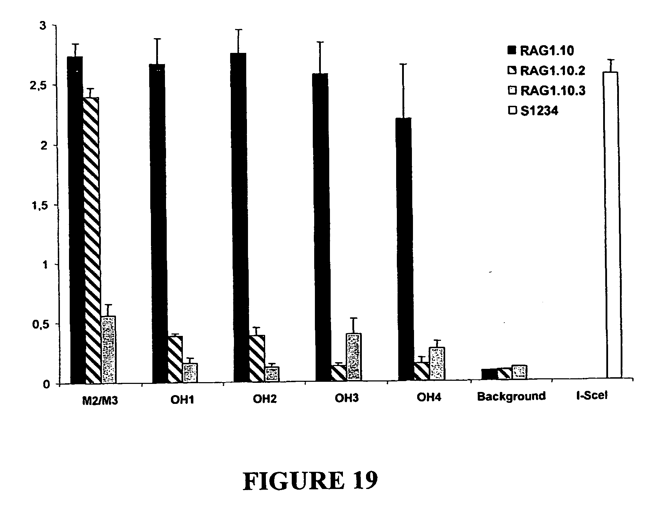

- figure 19 illustrates the activity of the M2 / M3 initial RAG1.10 heterodimer and the four obligatory heterodimers OH1 to OH4 against the three RAG1.10 targets in an extrachromosomal assay in CHO cells. Positive and negative controls are the same than those described in figure 15.

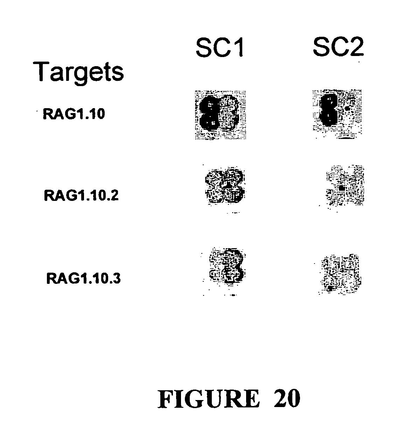

- figure 20 illustrates the screening in yeast of two single chain molecules SC1 and SC2 against the three RAG1.10 targets. SC1 is the M3-RM2-M2 molecule and SC2 stands for the M3(K7E K96E)-RM2-M2(E8K E61R) molecule. For each four dots yeast cluster, the two left dots are the result of the experiment, while the two right dots are various internal controls to assess the experiment quality and validity.

### Example 1: Protein Design

### 1) Material and methods

The different heterodimers were designed using FoldX (version 2.6.4), an automatic protein design algorithm (Guerois et al., J. Mol. Biol., 2002, 320, 369-387; Schymkowitz et al., Nucleic Acids Res, 2005, 33, W382-388; Schymkowitz et al., Proc. Natl. Acad. Sci. U.S.A., 2005, 102, 10147-10152). The crystal structure of I*-Cre*I meganuclease, in complex with DNA (PDB code: 1g9y.pdb; Chevalier et al., Nat. Struct. Biol., 2001, 8, 312-316), was used as template for the design. The structure was first optimised using the <RepairPDB> command of FoldX, in order to release any van der Waals clashes. Each position of interest was then mutated to Alanine and, using the <BuildModel> command, all models (heterodimers and homodimers alike) were generated separately. Finally, each model of the complex was analysed through the <AnalyseComplex> command to compute the different interaction energies.

### 2) Results

The X-ray structure of the I*-Cre*I homodimer determined at 2.05 A resolution (PDB:1g9y), bound to its cognate DNA target sequence (Chevalier et al., Nat. Struct. Biol., 2001, 8, 312-316, was used to design the heterodimeric interface of I*-Cre*I*,* The aim was to facilitate heterodimerisation and at the same time to prevent the formation of homodimers, or at least make them thermodynamically unstable.

A large part of the dimerisation interface of the homodimer is composed of two α-helices (Lys7 to Gly19 in both monomers), arranged in a coiled-coil, making them unsuitable for re-design. The amino acids below these helices (Asp20 and onward) are contacting the DNA and thus are responsible of both the activity (active site) and specificity (DNA recognition) of the endonucleases. These functions alone prevent any of these residues to be modified in the design process. Thus, this left few possibilities to enforce the heterodimerisation. After careful examination of the structure, three patches of interactions involved in the interface that could be disturbed and changed in the dimers, without impairing their binding capacity or their enzymatic activity, were identified (Figure 1).

One of these is the region above the two helices (Figure 1A), where Lys7 and Glu8 in one monomer establish favorable electrostatic interactions with the corresponding residues in the other monomer. In order to keep this interaction in the heterodimer, and at the same time impair monomer formation, these residues were replaced with two arginines in one monomer (named monomer A hereafter) and two glutamates in the other (called monomer B). Thus, AA and BB homodimers would undergo small electrostatic repulsion whereas AB heterodimer formation would be more electrostatically favorable.

The second patch was chosen with the same idea of creating small electrostatic imbalances for homodimers, relative to heterodimers, but is positioned on each side of the coiled-coil; a double cluster of charged residues is made by the Lys96 and the Glu61 of each monomer (Figure 1B). To re-enforce the electrostatic effects of the first mutation site, the second site was mutated with two arginines in monomer A, and two glutamates in monomer B, thus making a charged triangle in each monomer (positive in A, negative in B).

The third region of interest is around the middle of the two helices involved in the interaction surface and is mainly composed of hydrophobic interactions and hydrogen bonds, making a kind of minicore. As the H-bond network is quite intricate and extends all the way to the active site, it was decided to perturb only the hydrophobic patch made by residues Tyr12, Phe16, Val45, Trp53, Phe54, Leu55 and Leu58 of one monomer, with residue Leu97 of the other monomer (the latter acting like a cap closing the hydrophobic pocket) (Figure 1C). These two pockets were redesigned in order to introduce strong Van der Waals Clashes in the homodimers without disturbing the hydrophobic interactions in the heterodimers (i.e. without creating cavities and steric clashes). For this, bulky residues were introduced in monomer A (respectively Phe or Trp for position 54 and Phe for position 97) and small residues in monomer B (Gly and Leu, respectively). A glycin could be introduced at position 97 to give more space to position 54 when it is mutated in Tryptophan. As a result, AA homodimers develop huge steric hindrance, preventing their formation, and BB homodimers contain big cavities, making them unstable. By contrast, the minicore of AB heterodimers should be filled efficiently by these compatible amino acids. Finally, Leu 58 was mutated to methionine in monomer B, to prevent any cavity formation in the heterodimer, due to the introduction of the small sidechains. Note that replacing Lys 57 with a methionine would have been another solution for the same problem.

Thus, two types of monomer A, A 1 and A2, depending on the nature of the amino acid at position 54, respectively Phe or Trp and four types of corresponding monomer B, B3 to B6, were designed. B4 differs from B3 by a mutation in Glycine at position 97 to accommodate with the Trp mutation of monomer A2 (Figure 1D). B5 and B6 differ from B3 and B4, respectively, by a substitution of Lys57 with a methionine, whereas Leu58 is not mutated. The different mutations were tested with FoldX to model all homo-(A1:A1, A2:A2, B3:B3, B4:B4, B5:B5 and B6:B6) and heterodimers (A1:B3, A2:B3, A2:B4, A1:B5, A2:B5 and A2:B6) and get the different interaction energies (Table I).

**Table I: FoldX calculated free energies of interaction for wild-type or designed homodimers and heterodimers.**

| **Dimers** | **Difference in Interaction energies between mutants and wild type (kcal/mol)** |
|---|---|
| **A1_B5** | **0,911** |
| **A2_B3** | **0,9113** |
| **A2_B5** | **1,0488** |
| **A1_B3** | **1,2375** |
| **A2_B4** | **3,7899** |
| **A2_B6** | **3,9856** |
| **B3_B3** | **7,9921** |
| **B5_B5** | **8,9138** |
| **A1_A1** | **9,8489** |
| **A2_A2** | **12,0421** |
| **B4_B4** | **12,6754** |
| **B6_B6** | **13,1897** |

The last constructions, A2:B4 and A2_B6, presented a small decrease in interaction energy compared to the wild-type homodimer but was nonetheless significantly higher than the homodimers. Conversely, A1:A1, A2:A2, B3:B3, B4:B4, B5:B5 and B6:B6 homodimers were all strongly destabilised and thus these species were expected to remain monomeric.

### Example 2: Engineering of meganucleases with different cleavage specificity and optimization of cleavage conditions

### 1) Material and methods

### a) Engineering of meganucleases

Meganuclease variants with altered substrate specificity were engineered as described previously (International PCT Applications WO 2004/067736, WO 2006/097784 and WO 2006/097853; Epinat et al., Nucleic Acids Res., 2003, 31, 2952-2962; Chames et al., Nucleic Acids Res., 2005, 33, e178; Arnould et al., J. Mol. Biol., 2006, 355, 443-458, and Smith et al., Nucleic Acids Res., 2006, 34, e 149).

### b) Production and purification of meganucleases

Fresh BL21(DE3) (STRATAGENE) transformants carrying the pET (NOVAGEN) I*-Cre*I mutants, were grown overnight in 5 ml of Luria Broth (LB plus 30 µg/ml kanamycin) at 37°C on a shaker. This pre-culture was expanded to a larger culture (1:200). At an OD₆₀₀ of 0.6-0.8, flasks were put on ice for 15 min to arrest growth. Expression was induced by adding IPTG (1 mM final) for 18 hours at 16°C, and cells were harvested by centrifugation (15 min, 16,000 g). Pellets were resuspended in 30 ml ice-cold lysis buffer (50mM Tris·HCl pH 8, 200mM NaCl, 5mM MgCl₂, 10% Glycerol, 10 mM imidazole) containing 1 unit/µl DNAse I and the procedure was carried out at 4°C thereafter. The suspension was immediately frozen in liquid nitrogen and thawed for 16 hours at 4°C on a rotating platform (60 rpm). The suspension was homogenized with an ULTRA TURRAX T25 (JANKEL & KUNKEL, IKA-Labortechnik; 3 cycles of 1 min on ice) and then broken with an EmulsiFlex-C5 homogenizer (AVESTIN), for 5 rounds of 500-1000 psi (pounds per square inch) each. The lysate was centrifuged at 150,000 g for 60 min. This supernatant was cleared through a 0.45µm filter (MILLIPORE). A 5ml Hi-Trap column (AMERSHAM-PHARMACIA) was loaded with 2 bead volumes (vol) of 250 mM NiS0₄, and rinsed with 3 volumes of binding buffer (50mM Tris·HCl pH 8, 300mM NaCl, 1mM DTT, 20% glycerol, 10mM imidazole). The supernatant was then applied to the column and washed with washing buffer (binding buffer with 50 mM imidazole) until the A₂₈₀ₙₘ returned to its basal level. Protein was eluted with elution buffer (0.3M imidazole). The protein peak was collected and immediately applied to a dialysis membrane (Molecular Weight Cut Off =3.5 kDa, SPECTRA), placed in 2 litres of dialysis buffer (50 mM Tris·HCl pH 8, 200 mM NaCl, 1 mM DTT, 1mM EDTA, 50% glycerol) at 4°C, for at least 12 hours. The purified protein was aliquoted and snap-frozen in liquid nitrogen and stored at -80°C.

### c) DNA digestion assays.

Cleavage of the target sequences was determined as previously described (Epinat et al., Nucleic Acids Res., 2003, 31, 2952-2962) with modifications: co-expressed, purified enzymes were diluted to 1 µg/µl in fresh dialysis buffer (in the case of the designed monomers which were purified separately, 1.5 µg of each monomer was added, they were brought to 0.5 µg/µl each). Enzymes were stored at -80°C. The reaction mixture was prepared using 3.75 µM enzyme, 34 nM of purified 3.2 kb plasmid containing the appropriate target sequences (pre-linearized with *Xmn*I) and NaCl concentrations varying between 50 and 300 mM, in a 20 µl final reaction volume. The digestion mixtures were incubated for 60 min at 37 °C in a water bath and then mixed with 2.5 µl volume of Stop buffer, modified from Wang et al., Nucleic Acids Res., 1997, 25, 3767-3776 (5 ml Glycerol, 2 ml EDTA 0.5 M, 0.5 ml SDS 20 %, 0.5 ml Proteinase K at 20 mg/ml, 2.5 ml bromophenol blue (1% w/v), pH 8). Samples were incubated for 30 min more at 37°C, and then half of each sample was visualised on a 1 % agarose gel.

### 2) Results

The correct design of a specific heterodimer was verified by employing two previously obtained meganuclease variants that recognize different DNA sequences (International PCT Application WO 2006/097853; Arnould et al., J. Mol. Biol., 2006, 355, 443-458). These two enzymes have been obtained by screening of a I*-Cre*I library having random variations at positions 44, 68 and 70, with 64 palindromic targets resulting from substitutions at positions ±3, ±4 and ±5 of a 22 bp palindromic target cleaved by I*-Cre*I*.*

These variants both harbour an Asp75 to Asn mutation that decreases energetic strains caused by the replacement of the basic residues Arg68 and Arg70; these arginines normally satisfy the hydrogen-acceptor potential of the buried Asp75 in the I-*Cre*I structure. One meganuclease having mutations Q44K, R68T and R70G (denoted as "KTG") recognizes the bases cct at positions -5, -4 and -3 of the DNA target. The other meganuclease having mutations Q44Q, R68A and R70N (called "QAN") recognizes the bases gtt at positions -5, -4 and -3 of the DNA target. Throughout the examples, the target DNA sequences are denoted by a 6-base code, with the first 3 bases corresponding to positions -5, -4 and -3 of the target sequence and the second 3 to positions +3, +4 and +5 on the same DNA strand; the two triplets are separated by a slash (/). Thus the target of the KTG enzyme is cct/agg, that for the QAN target is gtt/aac, and the mixed DNA target for the heterodimer QAN-KTG is denominated as gtt/agg.

For the WT meganuclease I-*Cre*I, it has been reported that the ideal conditions for digestion of its target DNA are: 20 mM Tris-HCl (pH 8.0-9.0) with 10 mM MgCl₂ (Wang et al., Nucleic Acids Res., 1997, 25, 3767-3776), and the enzyme is reportedly inhibited above 25 mM NaCl ionic strength. When using these conditions with the KTG and QAN enzymes, suboptimal specificity was observed (Figure 2). In fact, at low ionic strength (≤50mM NaCl) both enzymes digest not only their target DNA sequence but also the mixed DNA target. This suggests that strong binding of only one of the monomers to the DNA is enough to allow digestion. Increasing ionic strength both improves the activity of the enzymes towards their targets and reduces the digestion of the mixed template: around 225mM NaCl, almost perfect specificity and good activity, were observed. This behaviour could be explained by the ionic strength decreasing the affinity for DNA (thus preventing binding if only one monomer establishes specific interactions in the dimer), while also increasing enzymatic activity. As a result of these tests, the following optimal buffer was selected for digestion of the meganuclease designs: 25 mM HEPES (pH 8), 5 % Glycerol, 10 mM MgCl₂ and 225 mM NaCl.

### Example 3: Expression and characterization of the designed mutants cleaving an artificial target

### 1) Material and methods

### a) Cloning of meganuclease mutants.

The two homodimerising meganucleases KTG and QAN, based on the I-*Cre*I meganuclease scaffold, were each mutated at up to 6 amino acid positions to form two compatible heterodimerising interfaces, denoted KTG-A2 and QAN-B3. Mutations were introduced using round-the-world PCR with a Quickchange® kit (STRATAGENE, # 200518).

KTG-A2 mutations (K7R, E8R, F54W, E61R, K96R, L97F) were introduced using three complementary primer sets:
(i) **A1_RR_F** (SEQ ID NO: 3) and **A1_RR_R** (SEQ ID NO:4):
   5' caa tac caa ata taa cag gcg gtt cct gct gta cct ggc cg 3' (SEQ ID NO: 3)
   5'cgg cca ggt aca gca gga acc gcc tgt tat att tgg tat tg 3'(SEQ ID NO: 4);
(ii) **A1_RF_F** (SEQ ID NO: 5) and **A1_RF_R** (SEQ ID NO: 6):
   5' tca act gca gcc gtt tct gag att caa aca gaa aca ggc aaa cc 3' (SEQ ID NO: 5)
   5' ggt ttg cct gtt tct gtt tga atc tca gaa acg gct gca gtt ga 3' (SEQ ID NO: 6);
(iii) **A2_WLR_F** 3' (SEQ ID NO: 7) and **A2_WLR_R** (SEQ ID NO: 8):
   5' cca gcg ccg ttg gtg gct gga caa act agt gga tag aat tgg cgt tgg tta cg 3' (SEQ ID NO: 7)
   5'cgt aac caa cgc caa ttc tat cca cta gtt tgt cca gcc acc aac ggc gct gg 3'(SEQ ID NO: 8).

QAN-B3 mutations (K7E, F54G, L58M, K96E) were introduced using three complementary primer sets:
(i) **B3_EE_F** (SEQ ID NO: 9) and **B3_EE_R** (SEQ ID NO: 10):
   5' caa tac caa ata taa cga aga gtt cct gct gta cct ggc cg 3' (SEQ ID NO: 9), and
   5' cgg cca ggt aca gca gga act ctt cgt tat att tgg tat tg 3' (SEQ ID NO: 10);
(ii) **B3_GME_F** (SEQ ID NO: 11) and **B3_GME_R** (SEQ ID NO: 12):
   5' cca gcg ccg ttg ggg tct gga caa aat ggt gga tga aat tgg cgt tgg tta cg 3' (SEQ ID NO: 11)
   5' cgt aac caa cgc caa ttt cat cca cca ttt tgt cca gac ccc aac ggc gct gg 3' (SEQ ID NO: 12);
(iii) **B3_EL_F** (SEQ ID NO: 13) and **B3_EL_R** (SEQ ID NO: 14):
   5' tca act gca gcc gtt tct gga act gaa aca gaa aca ggc aaa cc 3' (SEQ ID NO: 13) and
   5' ggt ttg cct gtt tct gtt tca gtt cca gaa acg gct gca gtt ga 3' (SEQ ID NO: 14).

The first primer set was used for PCR and transformation, according to the manufacturer's instructions (STRATAGENE, Quikchange®). Approximately 300 transformant bacterial colonies were pooled in 2 ml medium, and plasmid DNA was recovered by miniprep. This DNA was used as template for a second and then a third round of PCR with mutagenic primers. Five third-round mutants were verified by DNA sequencing.

It is worth noting that the primers above are universal for any I-*Cre*I mutant with altered specificity since the dimer interface mutations are outside the DNA recognition region.

Similar methods were used to make the alternative designs for the heterodimer pairs (QAN-A1, KTG-B3, QAN-B4), introducing the appropriate mutations in the oligos for mutagenic PCR (Figure 3).

### b) Production and purification of meganucleases

The experimental procedures are as described in example 2.

### c) DNA digestion assays.

The experimental procedures are as described in example 2, with a DNA digestion buffer consisting of: 25 mM HEPES (pH 8), 5 % Glycerol, 10 mM MgCl₂, and 50 mM NaCl (low ionic strength) or 225 mM NaCl (high ionic strength).

### d) Analytical Centrifugation

The oligomeric state of meganucleases and mutants was investigated by monitoring sedimentation properties in centrifugation experiments; 1.04 mg of pure protein was used per sample (0.52 mg/ml of each monomer or 1.04 mg/ml of individual WT homodimers) in storage buffer (50 mM Tris-HCl pH 8.0, 225 mM NaCl, 1 mm EDTA, 1 mM DTT, 8% glycerol).

The sedimentation velocity profiles were collected by monitoring the absorbance signal at 280 nm as the samples were centrifuged in a BECKMAN Optima XL-A centrifuge fitted with a four-hole AN-60 rotor and double-sector aluminium centerpieces (48 000 rpm, 4 °C). Molecular weight distributions were determined by the C(s) method implemented in the Sedfit (Schuck, P., Biophys., 2000, 78, 1606-1619) and UltraScan 7.1 software packages (Demeler.B.,2005, http://www.ultrascan.uthscsa.edu)

Buffer density and viscosity corrections were made according to data published by Laue et al. (In Analytical Ultracentrifugation in Biochemistry and Polymer Science, 1992, Harding S.E., Rowe A,J., Horton J.C. Eds, pp. 90-125, Royal Society of Chemistry, Cambridge) as implemented in UltraScan 7.1.

The partial specific volume of meganucleases and mutants was estimated from the protein sequence according to the method by Cohn E. J. and Edsall J.T.(In *Proteins, Amino Acids and Peptides, 1943, p.157, Reinhold, New York).*

### 2) Results

The designed mutants A1, A2, B3 and B4 were obtained by site-directed mutagenesis (STRATAGENE, Quikchange® Kit) of the original KTG and QAN enzyme expression vectors, followed by expression and purification of the corresponding proteins (Figure 4). Among the different combinations of mutants, the QAN-A1, KTG-B3, KTG-A2, QAN-B3 and QAN-B4 mutants were selected These were designed to give coverage of all the designed heterodimer interactions A1:B3, A2:B3 and A2:B4, resulting in the heterodimers QAN-A1:KTG-B3, KTG-A2:QAN-B3 and KTG-A2:QAN-B4.

Whereas the wild-type KTG and QAN enzymes yield the majority of protein in the soluble fraction, the opposite was observed in the case of the designed enzymes: the majority of the expressed proteins remained in inclusion bodies in the pellet, only a small fraction could be purified, and even this was contaminated by other proteins (Figure 4). This was a first indication that the designed mutants cannot homodimerize and thus become unstable and aggregate when expressed individually.

The activity of the purified A1, A2, B3 and B4 enzymes was tested on the three DNA targets (Figure 5) at low and high ionic strength (50 mM or 225 mM NaCl). At low salt concentration some specific DNA digestion activity could be detected for QAN-A1 only; specific cleavage could not be observed for the other enzymes. Moreover, at high ionic strength, the expected two DNA bands could not be detected, although the amount of DNA decreased in some cases, upon incubation with the enzymes.

These results were marred by the low yield and quality of the protein obtained when the non-homodimerising monomer designs were expressed individually; even with a large 6-litre volume of bacteria yielding inadequate protein (between 0.5-1.5 mg/ml for designed monomers compared with 30 mg/ml for wild-type dimerising monomers).

To check the oligomeric status of the purified designed enzymes, their size profiles was measured by analytical ultracentrifugation (Figure 6). In the case of individually-expressed A1, A2, B3 and B4 proteins, the appearance of the expected monomeric enzyme was observed. However, higher molecular weight aggregates were also detected, including trimers and tetramers (Figure 6B; only KTG-A2 and QAN-B3 are shown, although similar results were obtained with the other designs). Therefore the designed enzymes were indeed unable to homodimerise, and this may have affected their stability and aggregation properties during purification.

To investigate the potential for heterodimerisation, equimolar quantities of the individually purified designed enzymes (QAN-A1, KTG-A2, QAN-B3, KTG-B3, QAN-B4) were mixed in all possible combinations. In the case of the KTG-A2/QAN-B3, the appearance of a major species corresponding to the molecular weight of the dimer was observed, but this was not the only species formed. For the pair QAN-A1/KTG-B3 and KTG-A2/QAN-B4, the appearance of new peaks of molecular mass between the monomer and dimer, and a decrease of high molecular weight aggregates, were observed. For those combinations that should not produce a heterodimer, no significant changes in the behaviour of the proteins could be observed. Overall, these results indicated that separate expression of heterodimerising monomers was not an effective strategy, so co-expression assay within the bacterial cell was performed.

### Example 4: Co-expression and characterization of the designed mutants cleaving an artificial target

### 1) Material and methods.

### a) Co-expression of the designed monomers

In order to remove the His tag from the QAN-B3 monomer, it was excised from the parent plasmid pCLS1214 (pET-series) with *Nco*I*lNot*I (NEW ENGLAND BIOLABS). This fragment was then cloned into similarly-cut pCDFDuet1 plasmid (NOVAGEN). TOP10 ultracompetent cells (INVITROGEN) were transformed with this mixture and selected in 50 µg/ml Streptomycin-Spectinomycin sulphate. Clones were verified by DNA sequencing. BL21(DE3) ultracompetent cells were co-transformed with 10 ng of each plasmid (pCLS1211-KTG-A2 and pCDFDuet1-QAN-B3). The double transformants were selected by growing the transformed colonies in presence of Kanamycin and Streptomycin-Spectinomycin sulphate.

### b) Purification of meganucleases

The experimental procedures are as described in example 2.

### c) DNA digestion assays.

The experimental procedures are as described in example 2, with a DNA digestion buffer consisting of: 25 mM HEPES (pH 8), 5 % Glycerol, 10 mM MgCl₂, and 225 mM NaCl (high ionic strength).

### d) Analytical Centrifugation

The experimental procedures are as described in example 3.

### 2) Results

The results presented in example 3 suggested that the heterodimer designs might have been functioning, but that the expression of the monomeric enzymes resulted in strong aggregation and thus in partly inactive enzymes. To avoid this problem, the monomer gene expression cassettes were subcloned into complementary plasmids and co-transformed into bacterial cells, such that one monomer would be expressed (with a His-tag) from the original pET-series plasmid and that the partner monomer would be expressed (without a His-tag) from a compatible pCDFDuet vector (INVITROGEN). Dual antibiotic selection ensured that each cell contained both plasmids.

Expression analysis of the co-expressed KTG-A2/QAN-B3 proteins showed that inclusion bodies were avoided, suggesting that the previous aggregation problem had been solved. SDS-page analysis of the purified enzyme subsequently revealed 2 bands with approximately the same amount of protein, suggesting that we the heterodimer and not homodimer, was purified. Mass spectroscopy directly confirmed the presence of the two proteins and of the heterodimeric complex. Furthermore, an analytical ultracentrifugation of the purified proteins gave an exceptionally clean single profile at the expected molecular weight for a dimer (Figure 6C).

Digestion of the various DNA targets with the purified co-expressed heterodimer designs was carried out and clear specific cleavage of the heterodimer DNA target (gtt/agg) and barely of the homodimeric targets (cct/agg and gtt/aac; Figure 7A) was observed. Although the KTG-A2/QAN-B3 heterodimer seemed to exhibit specific cleavage against the hetero DNA target it could have been possible that it was less active. To rule out this we compared the activities of the QAN, KTG and KTG-A2/QAN-B3 in a time-course (Fig 7B). This experiment shows that KTG and the heterodimer have similar activities, whereas QAN requires 4-times longer incubation for 50% cleavage of its substrate, using the same apparent protein concentration.

Thus, the protein design was successful, allowing the specific formation of heterodimeric I-*Cre*I enzyme variants, as long as the monomer moieties were co-expressed.

The same experiments repeated with the co-expressed QAN-A1/KTG-B3 proteins showed mixed results, with 2 bands after purification but one stronger than the other and specific cleavage of the heterodimer, but at reduced level compared to the KTG-A2/QAN-B3 combination (Figure 6D). Analytical centrifugation showed formation of a dimer with a small proportion of aggregate.

Finally the third co-expression combination, KTG-A2/QAN-B4, resulted in only one band being purified and a monomer detected by analytical centrifugation (Figure 6D). Therefore this design failed to make a heterodimer, even when co-expressed.

Interestingly enough, the proportion of dimer and activity between KTG-A2/QAN-B3, QAN-A1/KTG-B3 and KTG-A2/QAN-B4 correlate perfectly well with the energies predicted by FoldX (Table I). The best design, in terms of predicted energy *in silico,* forms the best heterodimer *in vitro.*

### Example 5: Competition experiments

To measure the relative discrimination of the enzymes for homodimer and heterodimer DNA sites, competition experiments where the enzymes had access to equimolar amounts of both substrates simultaneously were carried out (Figure 8). the KTG-A2/QAN-B3 heterodimer and KTG-wt were selected for these experiments, because they exhibited similar activities against their respective targets (Figure 8B). In a time course experiment, KTG cleaved preferentially its target, although there was a slight digestion of the KTG-A2/QAN-B3 target cognate site (Figure 8A). By contrast, KTG-A2-QAN-B3 heterodimer preferentially cleaved the Q-K heterodimer DNA with little cleavage of the KTG site.

To compare the relative cutting preferences more directly, an enzyme titration was carried out against equimolar mixtures of both DNA targets (Figure 8B). This allowed the determination of the apparent concentrations for 50 % cleavage for cognate and non-cognate targets under competitive conditions: KTG-wt for cognate target = 0.1 µM; KTG-wt for non-cognate target = 1.5 µM; KTG-A2/QAN-B3 for cognate target = 0.3 µM; KTG-A2/QAN-B3 for non-cognate target = 3.2 µM. Therefore, for both enzymes there was found to be an approximate 10- to 15-fold difference in enzyme concentration separating 50 % cleavage of the cognate and the non-cognate targets.

In summary, these results show that although the specificities of both the parent constructs and the mutant derivatives are not absolute, it was possible to design obligate heterodimer meganucleases which have similar activity to the best wt parent, and a clear cleavage preference for their heterodimer targets, whereas the original homodimers have the opposite preference for their homodimer targets.

### Example 6: Making of RAG1.10 obligatory heterodimer by targeting the Arginine 51-Aspartate 137 interaction.

An heterodimer able to cleave a target from the RAG1 gene (RAG1.10 sequence; figure 9) was obtained previously, by co-expression in yeast, of two I-*Cre*I mutants able to cleave the palindromic RAG1.10 derived targets RAG1.10.2 and RAG1.10.3 (Smith et al., Nucleic Acids Res., 2006, 34, e149; figure 10). However, the coexpression of two I-*Cre*I mutants leads to the presence of three molecular species in solution: the two homodimers and the heterodimer. Obligatory heterodimers were made *via* the introduction of additional mutations, to aim at preventing the formation of functional homodimers without affecting the heterodimer stability and cleavage properties. In consequence, only the RAG1.10 target would be cleaved. To realize such obligatory heterodimers, the two residues Arginine 51 (R51) and Aspartate 137 (D137), which make an intermolecular interaction R51-D137 between the two I-*Cre*I monomers, were selected. These two residues are also close to the active site and are involved in the coordination of the water molecules shell surrounding the active site (Figure 11). By making for example the R51D mutation (arginine replaced by an aspartate residue) on a RAG1.10.2 cutter (an I-*Cre*I mutant, which cleaves the RAG1.10.2 target) and the compensatory D137R mutation on a RAG1.10.3 cutter, there is still presence of an attractive D51-R137 interaction in the heterodimer, while there is a repulsive D51-D137 interaction in the RAG1.10.2 homodimer and also a repulsive R51-R137 interaction in the RAG1.10.3 homodimer. It was postulated that such repulsive interactions could destabilize the homodimers formation and/or interfere with the catalytic mechanism.

The RAG1.10.2 cutter (called M2) with the sequence KRSNQS/AYSDR (residues at positions 28, 30, 32, 33, 38, 40 / 44, 68, 70, 75, 77 are indicated) and one RAG1.10.3 cutter (called M3) with the sequence NNSSRR/YRSQV, previously obtained as described in Smith et al., Nucleic Acids Res., 2006, 34, e149, and for each mutant, either the R51D mutation or the D137R mutation were introduced in order to create four single mutants.

In this example and the following examples, the cleavage activity of the obligate heterodimer meganuclease according to the present invention against the non-palindromic target of interest and the derived palindromic targets cleaved by the two homodimers was measured by a direct repeat recombination assay, in yeast or mammalian cells, as described previously (International PCT Application WO 2004/067736 and WO 2006/097853; Epinat et al., Nucleic Acids Res., 2003, 31, 2952-2962; Chames et al., Nucleic Acids Res., 2005, 33, e178 and and Arnould et al., J. Mol. Biol., 2006, 355, 443-458). The reporter vector comprises two truncated, non-functional copies of the LacZ reporter gene (direct repeats) and the DNA target sequence, within the intervening sequence, cloned in a yeast or a mammalian expression vector. Expression of the meganuclease results in cleavage of the genomic chimeric DNA target sequence. This cleavage induces homologous recombination between the direct repeats, resulting in a functional LacZ gene, whose expression can be monitored by appropriate assay.

### 1) Material and Methods

### a) Introduction of the R51D and D137R mutations:

Each mutation was introduced using two PCR reactions carried on the DNA of the RAG1.10.2 and RAG1.10.3 cutters.

For the R51D mutation, the first PCR reaction was performed with the primers: Gal10F 5'-gcaactttagtgctgacacatacagg-3' (SEQ ID NO: 37) and R51DRev 5'-tttgtccagaaaccaacggtcctgggtcttttgagtcac-3' (SEQ ID NO: 38) and the second with the primers R51DFor 5'-gtgactcaaaagacccaggaccgttggtttctggacaaac-3' (SEQ ID NO: 39) and Gal10R 5'-acaaccttgattggagacttgacc-3' (SEQ ID NO: 40).

To introduce the D137R mutation, the same approach was used with the primers D137RRev 5'-ggttttacgcgtcttagaacggttcagagctgcaatctg-3' (SEQ ID NO: 41) and D137RFor 5'-cagattgcagctctgaaccgttctaagacgcgtaaaacc-3' (SEQ ID NO: 42). All the PCR fragments were purified and were used either with the previously described vector pCLS0542 (see for example figure 5 of International PCT Application WO 2006/097853), linearized with *Nco*I and *Eag*I (for the RAG1.10.2 cutter) or with the previously described vector pCLS1107 (see for example figure 14 of International PCT Application WO 2007/093918), linearized with *NgoM*IV and *Dra*III (for the RAG1.10.3 cutter) to transform the yeast *Saccharomyces cerevisiae* strain FYC2-6A (MATα, trp1Δ63, leu2Δ1, his3200) using a high efficiency LiAc transformation protocol. An intact coding sequence for the I-*Cre*I mutant is then generated by *in vivo* homologous recombination in yeast. The yeast DNA was recovered and used to transform a DH5a *E.coli* strain. The bacteria DNA was purified and the presence of the R51D or D 13 7R mutation was checked by sequencing.

### b) Mutants co-expression

The yeast strain FYC2-6A was transformed with DNA coding for a mutant deriving from the initial RAG1.10.2 cutter in pCLS0542 expression vector and with DNA coding for a mutant deriving from the initial RAG1.10.3 cutter in pCLS1107 expression vector. Transformants were selected on -L Glu + G418 medium.

### c) Mating of meganucleases coexpressing clones and screening in yeast

Mating was performed using a colony gridder (QpixII, Genetix). Mutants were gridded on nylon filters covering YPD plates, using a low gridding density (about 4 spots/cm²). A second gridding process was performed on the same filters to spot a second layer consisting of different reporter-harbouring yeast strains for each target. Membranes were placed on solid agar YPD rich medium, and incubated at 30°C for one night, to allow mating. Next, filters were transferred to synthetic medium, lacking leucine and tryptophan, adding G418, with galactose (1 %) as a carbon source, and incubated for five days at 37°C, to select for diploids carrying the expression and target vectors. After 5 days, filters were placed on solid agarose medium with 0.02 % X-Gal in 0.5 M sodium phosphate buffer, pH 7.0, 0.1 % SDS, 6 % dimethyl formamide (DMF), 7mM β-mercaptoethanol, 1 % agarose, and incubated at 37°C, to monitor β-galactosidase activity. Results were analyzed by scanning and quantification was performed using an appropriate software.

### 2) Results

Co-expression of the R51D RAG1.10.2 cutter with the D137R RAG1.10.3 cutter resulted in an efficient cleavage of the RAG1.10 target while no cleavage of the RAG1.10.2 and RAG1.10.3 targets was visible (Figure 12). This clearly shows that the heterodimer is active on the RAG1.10 target sequence, while the two homodimers do not cleave the palindromic RAG1.10.2 and RAG1.10.3 targets. Such an heterodimer matches the criteria used to define an obligatory heterodimer. The coexpression of the D137R RAG1.10.2 cutter with the R51D RAG1.10.3 cutter leads also to the formation of an obligatory heterodimer, which is yet less active than the precedent one on the RAG1.10 target.

In order to compare this heterodimer with those described in examples 1, 3 and 4, the free energies of interaction of the R51D and D137 mutants, were calculated as described in example 1. As shown on Table II, the heterodimer (S1_S2) was very similar to the wild-type homodimer, while the mutant homodimers (S1_S1 and S2_S2) were significantly destabilized.

**Table II: FoldX calculated free energies of interaction for wild-type or designed homodimers and heterodimers.**

| **Dimers** | **Difference in Interaction energies between mutants and wild type (kcal/mol)** |
|---|---|
| **A1_B5** | **0,911** |
| **A2_B3** | **0,9113** |
| **A2_B5** | **1,0488** |
| **A1_B3** | **1,2375** |
| **S1_S2** | **1,3827** |
| **A2_B4** | **3,7899** |
| **A2_B6** | **3,9856** |
| **S1_S1** | **5,9036** |
| **B3_B3** | **7,9921** |
| **B5_B5** | **8,9138** |
| **S2_S2** | **9,7611** |
| **A1_A1** | **9,8489** |
| **A2_A2** | **12,0421** |
| **B4_B4** | **12,6754** |
| **B6_B6** | **13,1897** |

| | |
|---|---|
| *S1 and S2 stands for the R51D and D137R mutants, respectively | |

### Example 7: Making of a RAG1.10 obligatory heterodimer by targeting the Lysine7 - Glutamate8 interaction.

In this example, the lysine7-glutamate8 interaction which occurs between the two monomers was targeted. Therefore, the single mutation K7E or E8K was introduced in the M2 or M3 mutant of example 6. The resulting mutants were then coexpressed in CHO cells and the activity of the heterodimer and of the two homodimers was monitored against the three RAG1.10 targets using the previously described Single-strand annealing (SSA) extrachromosomal assay in CHO cells (International PCT Applications WO 2004/067736 and WO 2006/097853; Epinat et al., Nucleic Acids Res., 2003, 31, 2952-2962; Chames et al., Nucleic Acids Res., 2005, 33, e178; Arnould et al., J. Mol. Biol., 2006, 355, 443-458).

### 1) Material and Methods

### a) Introduction of the K7E and E8K mutations

The I-*Cre*I derived mutants M2 and M3 were already cloned in a mammalian expression vector. Each mutation was introduced using two overlapping PCR reactions carried out on the DNA of the M2 and M3 mutants. For the K7E mutation, the first PCR reaction was done with the primers CMVFor (5'-cgcaaatgggcggtaggcgtg-3'; SEQ ID NO: 44) and K7ERev (5'-gtacagcaggaactcttcgttatatttggtattgg-3'; SEQ ID NO: 45) and the second reaction with the primers K7EFor (5'-aataccaaatataacgaagagttcctgctgtacc-3'; SEQ ID NO: 46) and V5epitopeRev (5'-cgtagaatcgagaccgaggagagg-3'; SEQ ID NO: 47). The two PCR fragments were gel purified, mixed and a third assembly PCR was conducted using the CMVFor and V5epitopeRev primers. The obtained PCR fragment contains the open reading frame of the I-*Cre*I mutant with the K7E mutation. The PCR fragment was then purified, digested with the restriction enzymes *Sac*I and *Xba*I and ligated into the pCLS1088 (Figure 13) also digested by *Sac*I and *Xba*I. The resulting clones M2 K7E or M3 K7E were verified by sequencing (MILLEGEN). Introduction of the E8K mutation in the M2 and M3 mutants was carried out using absolutely the same protocol but the two primers E8KRev (5'- caggtacagcaggaactttttgttatatttgg-3'; SEQ ID NO: 48) and E8KFor (5'- accaaatataacaaaaagttcctgctgtacctgg-3'; SEQ ID NO: 49). b) Cloning of the RAG1.10, RAG1.10.2 and RAG1.10.3 targets in a vector for

### extrachromosomal assay in CHO cells

The target of interest was cloned as follows: oligonucleotide corresponding to the target sequence flanked by gateway cloning sequence was ordered from PROLIGO. Double-stranded target DNA, generated by PCR amplification of the single stranded oligonucleotide, was cloned using the Gateway protocol (INVITROGEN) into CHO reporter vector (pCLS1058, Figure 14).

### c) Extrachromosomal assay in CHO cells

CHO cells were transfected with Polyfect transfection reagent according to the supplier's (QIAGEN) protocol. 72 hours after transfection, culture medium was removed and 150 µL of lysis/revelation buffer added for β-galactosidase liquid assay (typically, 1 liter of buffer contained 100 ml of lysis buffer (Tris-HCl 10 mM pH7.5, NaCl 150 mM, Triton X100 0.1 %, BSA 0.1 mg/ml, protease inhibitors), 10 ml of Mg 100X buffer (MgCl₂ 100 mM, β-mercaptoethanol 35%), 110 ml ONPG 8 mg/ml and 780 ml of sodium phosphate 0.1 M pH7.5). After incubation at 37 °C, optical density was measured at 420 nm. The entire process was performed on an automated Velocity 11 BioCel platform.

### 2) Results

Activity of the single mutants M2 K7E or E8K and M3 K7E or E8K against their respective DNA targets RAG1.10.2 and RAG 1.10.3 was monitored using the previously described extra-chromosomal assay in CHO cells. As shown in figure 15A, the mutated proteins at position 7 or 8 are as active as the initial ones. In a second step, M2 and M3 mutants were co-expressed to form the three possible heterodimers: M2 / M3, M2 K7E / M3 E8K, M2 E8K / M3 K7E. Activity was then monitored against the three RAG1.10 targets. Figure 15B shows that when M2 or M3 mutants mutated at position 7 or 8 are co-expressed, a decrease in the homodimeric activities can be observed especially for the mutant which bears the E8K mutation. In the same time, the two heterodimers with mutations at position 7 and 8 keep the same activity level toward the natural RAG1.10 target in comparison with the initial M2 /M3 heterodimer. The two heterodimers M2 K7E / M3 E8K and M2 E8K / M3 K7E are not strict obligatory heterodimers because homodimeric activities can still be detected, and are even unmodified when the protein can form only homodimer. However, when proteins can form homodimers and heterodimers, targeting the K7 - E8 interaction decreases significantly at least one homodimeric activities. Therefore, such mutations strongly favour functional heterodimer versus functional homodimer formation.

### Example 8: Making of RAG1.10 obligatory heterodimers by targeting the Lysine7 - Glutamate8 and Glutamate61 - Lysine96 interactions.

Example 7 shows that the M2/M3 heterodimers derived from the M2 and M3 mutants having a single K7E or E8K mutation (M2K7E/M3E8K and M2E8K/M3K7E) retain the homodimeric activity. Therefore, additional mutations were introduced in the M2 or M3 mutants by targeting the Glutamate61 - Lysine96 interaction, which occurs between the two monomers forming the heterodimer. For each M2 or M3 mutant, 4 double mutants were created: K7E E61R, E8K E61R, K7E K96E and E8K K96E. Homodimeric activity of the eight double mutants was then monitored in CHO cells. In a second stage, the four M2 double mutants were co-expressed with the four M3 double mutants and activity of the sixteen resulting heterodimers against the three RAG1.10 targets was monitored using the previously described yeast screening assay and extrachromosomal CHO assay.

### 1) Material and Methods

### a) Introduction of the E61R and K96E mutations

Each mutation was introduced using two overlapping PCR reactions carried on the DNA of the M2 or M3 derived mutants cloned in a yeast expression vector. For the E61R mutation, the first PCR reaction was done with the primers Gal10F (5'-GCAACTTTAGTGCTGACACATACAGG-3'; SEQ ID NO: 37 and E61RRev (5'-gtaaccaacgccaatacgatccactagtttgtcc-3'; SEQ ID NO: 50) and the second with the primers E61RFor (5'-gacaaactagtggatcgtattggcgttggttacg-3'; SEQ ID NO: 51) and Gal10R (5'-ACAACCTTGATTGGAGACTTGACC-3'; SEQ ID NO: 40). To introduce the K96E mutation, we used exactly the same approach with the primers K96ERev (5'-cctgtttctgtttcagttccagaaacggctgcag-3'; SEQ ID NO: 52) and K96EFor (5'-ctgcagccgtttctggaactgaaacagaaacagg-3'; ; SEQ ID NO: 53). All the PCR fragments were purified and were used either with the pCLS0542 linearized vector with *Nco*I and *Eag*I (for the M2 derived mutants) or with the pCLS1107 linearized vector with *NgoM*IV and *Dra*III (for the M3 derived mutants) to transform the yeast *Saccharomyces cerevisiae* strain FYC2-6A (MATα, trp1Δ63, leu2Δ1, his3Δ200) using a high efficiency LiAc transformation protocol. An intact coding sequence for the I-*Cre*I mutant was then generated by *in vivo* homologous recombination in yeast. The yeast DNA was recovered and used to transform a DH5α *E.coli* strain. The bacteria DNA was purified and the presence of the E61 R or K96E mutation was checked by sequencing.

### b) Mutants coexpression

Yeast strain expressing a M2 derived mutant in pCLS0542 expression vector was transformed with DNA coding for a M3 derived mutant in pCLS1107 expression vector. Transformants were selected on -L Glu + G418 medium.

### c) Cloning of the M2 or M3 double muatnts into a mammalian expression vector

Each mutant ORF was amplified by PCR using the primers CCM2For (5'-aagcagagctctctggctaactagagaacccactgcttactggcttatcgaccatggccaatacca aatataacaaagagttcc-3'; SEQ ID NO: 54) and CCMRev (5'-ctgctctagattagtcggccgccggggaggatttcttc-3'; SEQ ID NO: 55). The PCR fragment was digested by the restriction enzymes *Sac*I and *Xba*I*,* and was then ligated into the vector pCLS1088 digested also by *Sac*I and *Xba*I. Resulting clones were verified by sequencing (MilleGen).

### 2) Results

Homodimeric activity of the four double mutated M2 or M3 mutants is displayed in figure 16, which shows that this activity is reduced to almost background levels especially for a mutant carrying the mutations K7E and K96E. In a second step, all the M2 derived mutants were co-expressed with all the M3 derived mutants. Activity of the resulting heterodimers against the three RAG1.10 targets was then monitored using the yeast screening assay (figure 17) and the extrachromosomal CHO assay (figure 18). Figure 17 shows that four heterodimers underlined by a red ellipsoid among the sixteen possible heterodimers behave as obligatory heterodimers in yeast. They have a strong cleavage activity toward the RAG1.10 target equivalent to the M2 / M3 heterodimer activity, while they display almost non detectable homodimeric activities. To underline this notion of obligatory heterodimer, figure 18 shows that a double mutated M2 mutant works only when it is co-expressed with a double mutated M3 mutant, which bears the compensatory mutations. The four obtained obligatory heterodimers have been called OH1 to OH4 and are respectively:
OH1 = M2 K7E E61 R / M3 E8K K96E
OH2 = M2 E8K E61 R / M3 K7E K96E
OH3 = M2 K7E K96E / M3 E8K E61R
OH4 = M2 E8K K96E / M3 K7E E61 R

Finally, figure 19 shows that the four obligatory heterodimers OH1 to OH4 have the same cleavage activity toward the RAG1.10 target than the initial M2 /M3 heterodimer in CHO cells but with homodimeric activities reduced to almost non detectable levels. These data show that is possible to generate obligatory heterodimers by introducing the K7E/E8K and E61R/K96E mutations in I-*Cre*I derived mutants forming a heterodimer.

### Example 9: Use of the K7E/E8K and E61R/K96E mutations in the single chain molecule design.

To further validate the use of the K7E/E8K and E61R/K96E mutations to improve a meganuclease specificity, these mutations were introduced in a RAG single chain construct. A single chain molecule M3-RM2-M2 was engineered by joining the C-terminal of the M3 mutant to residue 6 from the N-terminal of the M2 mutant with a long linker of 32 amino acids called RM2. As this molecule displayed some homodimeric activity against the RAG1.10.2 target, mutations K7E, K96E were introduced into the M3 mutant and mutations E8K, E61 R into the M2 mutant to create the single chain molecule: M3(K7E K96E)-RM2-M2(E8K E61R) that is called further SC_OH. Activity of both single chain molecules against the three RAG1.10 targets was then monitored using the previously described yeast screening assay.

### 1) Material and Methods

### Cloning of the SC_OH single chain molecule

A PCR reaction was performed on the M2 mutant carrying the E8K and E61R mutations cloned in the pCLS0542 yeast expression vector. The PCR reaction uses the reverse primer CreCterSacI (5'-tagacgagctcctacggggaggatttcttcttctcgct-3'; SEQ ID NO: 56) and the forward primer. RM2 (5'-tatcggccggtggatctgataagtataatcaggctctgtctaaatacaaccaagcactgtccaagtaca atcaggccctgtctggtggaggcggttccaacaaagagttcctgctgtatcttgctggattt-3' SEQ ID NO: 57).

The PCR fragment was purified and digested by *Eag*I and *Sac*I and ligated into the yeast expression vector for the M3 mutant carrying the mutations K7E and K96E also digested with *Eag*I and *Sac*I. After sequencing of the clones, a SC_OH single chain molecule was obtained

### 2) Results

The yeast screen of the two single chain molecules M3-RM2-M2 and SC_OH against the three RAG1.10 targets depicted in figure 20 shows that introduction of the K7E/E8K and E61R/K96E allows for the abolition of the homodimeric activity against the RAG1.10.2 target without reducing the single chain cleavage activity for the RAG1.10 target. It is therefore possible to introduce the mutations that have been described in example 8 in the obligatory heterodimer design, in a single chain molecule to improve its specificity without affecting its activity toward the DNA target of interest.

## Claims

1. An obligate heterodimer meganuclease **characterized in that** it consists of a first and a second monomer, deriving from two different homodimeric I-*Cre*I monomers (parent monomers), and having at least one pair of mutations in residues of said parent monomers which make an intermolecular interaction between the two monomers of each parent homodimeric I-*Cre*I and that the first mutation of said pair(s) is in the first monomer and the second mutation of said pair(s) is in the second monomer and said pair(s) of mutations impairs the formation of functional homodimers from each monomer without preventing the formation of a functional heterodimer able to cleave a non-palindromic hybrid DNA target comprising one different half of the DNA target cleaved by each parent homodimeric I-*Cre*I, wherein the monomers have at least one of the following pairs of mutations, respectively for the first and the second monomer:
a) the substitution of the leucine at position 97 by an aromatic amino acid and the substitution of the phenylalanine at position 54 by a small amino acid, and
b) the substitution of the aspartic acid at position 137 by a basic amino acid and the substitution of the arginine at position 51 by an acidic amino acid, said positions being indicated by reference to the I-*Cre*I amino acid sequence SEQ ID NO: 1.

2. The obligate heterodimer I-*Cre*I according to claim 1, wherein the monomers also comprise at least one of the following pairs of mutations, respectively for the first and the second monomer:
c) the substitution of the glutamic acid at position 8 by a basic amino acid and the substitution of the lysine at position 7 by an acidic amino acid,
d) the substitution of the glutamic acid at position 61 by a basic amino acid and the substitution of the lysine at position 96 by an acidic amino acid, said positions being indicated by reference to the I-*Cre*I amino acid sequence SEQ ID NO: 1.

3. The obligate heterodimer I-*Cre*I according to claim 1 or 2, wherein the monomer having the substitution of the glutamic acid at position 8 or 61 by a basic amino acid, as defined in c) or d), further comprises the substitution of at least one of the lysine residues at positions 7 and 96, by an arginine; and/or wherein the monomer having the substitution of the leucine 97 by an aromatic amino acid as defined in a), further comprises the substitution of the phenylalanine at position 54 by a tryptophane; and/or wherein the monomer having the substitution of the phenylalanine at position 54 by a small amino acid as defined in a), further comprises the substitution of the leucine at position 58 or lysine at position 57, by a methionine.

4. The obligate heterodimer I-*Cre*I according to claim 1 or 2, wherein said acidic amino acid is a glutamic acid;
wherein said basic amino acid is an arginine;
wherein said aromatic amino acid is a phenylalanine;
wherein said small amino acid is a glycine.

5. The obligate heterodimer I-*Cre*I according to anyone of claims 1 to 4, which consists of a first monomer having at least the mutation D137R and a second monomer having at least the mutation R51D.

6. The obligate heterodimer I-*Cre*I according to claim 1, which consists of a first monomer having at least the mutations K7R, E8R, E61R, K96R and L97F or K7R, E8R, F54W, E61R, K96R and L97F and a second monomer having at least the mutations K7E, F54G, L58M and K96E or K7E, F54G, K57M and K96E.

7. The obligate heterodimer I-*Cre*I according to anyone of claims 1 to 6, which consists of two I-*Cre*I monomer variants, further comprising different mutations at positions 26 to 40 and 44 to 77 of I*-Cre*I, in particular wherein said obligate heterodimer meganuclease is able to cleave a non-palindromic hybrid DNA target from a genomic sequence of interest corresponding to one different half of the DNA target that is cleaved by each parent homodimeric endonuclease.

8. A single-chain I-*Cre*I **characterised in that** the first and the second monomer of the obligate heterodimer meganuclease as defined in anyone of claims 1 to 7, connected by a peptidic linker.

9. A polynucleotide fragment encoding the monomer of said obligate heterodimer I-*Cre*I according to any one of claims 1 to 7 or the single-chain meganuclease according to claim 8.

10. A recombinant vector comprising at least one polynucleotide fragment of claim 9; in particular an expression vector comprising two polynucleotide fragments each encoding one of the two monomers of the obligate heterodimer I-*Cre*I of anyone of claims 1 to 7, said fragment(s) being operatively linked to regulatory sequences allowing the production of the two monomers; in particular wherein said vector includes a targeting DNA construct comprising sequences sharing homologies with the region surrounding the hybrid DNA target sequence as defined in claim 1.

11. A host cell, non-human transgenic animal or transgenic plant comprising one or two polynucleotide fragments as defined in claim 9 or a vector according to claim 10.

12. A composition comprising at least one obligate heterodimer I-*Cre*I according to anyone of claims 1 to 7, one single-chain I-*Cre*I according to claim 8, one or two polynucleotide fragments as defined in claim 9 or a vector according to claim 10, in particular wherein said composition further comprises a targeting DNA construct comprising the sequence which repairs the site of interest flanked by sequences sharing homologies with the targeted locus.

13. Use of at least one obligate heterodimer I*-Cre*I according to anyone of claims 1 to 7, one single-chain I-*Cre*I according to claim 8, one or two polynucleotide fragments as defined in claim 9, a vector according to claim 10, for the preparation of a medicament for preventing, improving or curing a genetic disease or infectious disease in an individual in need thereof, said medicament being administrated by any means to said individual.

14. Use of at least one obligate heterodimer I-*Cre*I according to anyone of claims 1 to 7, one single-chain I-*Cre*I according to claim 8, one or two polynucleotide fragments as defined in claim 9, a vector according to claim 10, *in vitro,* for inhibiting the propagation, inactivating or deleting an infectious agent that presents a DNA intermediate, in biological derived products or products intended for biological uses or for disinfecting an object, in particular wherein said infectious agent is a virus.

## Patentansprüche

1. Obligate Heterodimer-Meganuklease, **dadurch gekennzeichnet, dass** sie aus einem ersten und einem zweiten Monomer besteht, die sich von zwei verschiedenen homodimeren I-*Cre*I Monomeren (Ursprungsmonomere) ableiten, und, dass sie mindestens ein Paar von Mutationen in Resten der Ursprungsmonomere aufweist, die eine intermolekulare Wechselwirkung zwischen den zwei Monomeren jeder ursprünglichen homodimeren I-*Cre*I verursachen, und, dass die erste Mutation des/der beschriebenen Paares/Paare in dem ersten Monomer vorliegt und die zweite Mutation des/der beschriebenen Paares/Paare in dem zweiten Monomer vorliegt und, dass das/die beschriebene/beschriebenen Paar/Paare von Mutationen die Bildung von funktionellen Homodimeren jedes Monomers beinträchtigt/beeinträchtigen, ohne dass die Bildung eines funktionellen Heterodimers verhindert wird, das in der Lage ist, eine nicht-palindromische Hybrid-DNS-Zielsequenz zu spalten, die eine unterschiedliche Hälfte der DNS-Zielsequenz aufweist, die von jeder ursprünglichen homodimeren I-CreI gespalten wird, wobei die Monomere, beziehungsweise jeweils das erste und das zweite Monomer, mindestens eines der folgenden Paare von Mutationen aufweisen:
a) Substitution von Leucin in Position 97 durch eine aromatische Aminosäure und die Substitution von Phenylalanin in Position 45 durch eine kleine Aminosäure, und
b) Substitution von Asparaginsäure in Position 137 durch eine basische Aminosäure und die Substitution von Arginin in Position 51 durch eine saure Aminosäure, wobei die Positionen unter Bezugnahme auf die I-CreI Aminosäuresequenz SEQ ID NO: 1 angegeben sind.

2. Obgligate Heterodimer I-CreI nach Anspruch 1, wobei die Monomere, beziehungsweise jeweils das erste und das zweite Monomer, mindestens eines der folgenden Paare von Mutationen aufweisen:
c) Substitution von Glutaminsäure in Position 8 durch eine basische Aminosäure und Substitution von Lysin in Position 7 durch eine saure Aminosäure,
d) Substitution von Glutaminsäure in Position 61 durch eine basische Aminosäure und Substitution von Lysin in Position 96 durch eine saure Aminosäure, wobei die Positionen unter Bezugnahme auf die I-CreI Aminosäuresequenz SEG ID NO: 1 angegeben sind.

3. Obligate Heterodimer I-CreI nach Anspruch 1 oder 2, wobei das Monomer, das, wie in c) oder d) definiert, die Substitution von Glutaminsäure in Position 8 oder 61 durch eine basische Aminosäure aufweist, ferner die Substitution von mindestens einem der Lysinreste in Positionen 7 und 96 durch ein Arginin aufweist; und/oder wobei das Monomer, das, wie in a) definiert, die Substitution von Leucin 97 durch eine aromatische Aminosäure aufweist, ferner die Substitution von Phenylalanin in Position 54 durch Tryptophan aufweist; und/oder wobei das Monomer, das, wie in a) definiert, die Substitution des Phenylalanin in Position 54 durch eine kleine Aminosäure aufweist, ferner die Substitution von Leucin in Position 58 oder Lysin in Position 57 durch Methionin aufweist.

4. Obligate Heterodimer I-CreI nach Anspruch 1 oder 2, wobei die saure Aminosäure Glutaminsäure ist;
wobei die basische Aminosäure Arginin ist;
wobei die aromatische Aminosäure Phenylalanin ist;
wobei die kleine Aminosäure Glycin ist.

5. Obligate Heterodimer I-CreI nach einem der Ansprüche 1 bis 4, die aus einem ersten Monomer, das mindestens die Mutation D137R aufweist und aus einem zweiten Monomer besteht, das mindestens die Mutation R51D aufweist.

6. Obligate Heterodimer I-CreI nach Anspruch 1, die aus einem ersten Monomer besteht, das mindestens die Mutationen K7R, E8R, E61R, K96R und L97F oder K7R, E8R, F54W, E61R, K96R und L97F aufweist, und aus einem zweiten Monomer, das mindestens die Mutationen K7E, F54G, L58M und K96E oder K7E, F54G, K57M und K96E aufweist.

7. Obligate Heterodimer I-CreI nach einem der Ansprüche 1 bis 6, die aus zwei I-CreI Monomervarianten besteht, die ferner verschiedene Mutationen in den Positionen 26 bis 40 und 44 bis 77 der I-CreI aufweisen, wobei insbesondere die obligate Heterodimer-Meganuklease in der Lage ist, eine nicht-palindromische Hybrid-DNS-Zielsequenz von einer genomischen Sequenz von Interesse zu spalten, die einer unterschiedlichen Hälfte der DNS-Zielsequenz entspricht, die durch jede ursprüngliche homodimere Endonuklease gespalten wird.

8. Einkettige I-CreI, **dadurch gekennzeichnet, dass** das erste Monomer und das zweite Monomer der obligaten Heterodimer-Meganuklease, wie in einem der Ansprüche 1 bis 7 definiert, mit einem Peptidlinker verbunden sind.

9. Polynukleotid Fragment, das für das Monomer der beschriebenen obligaten Heterodimer I-CreI nach einem der Ansprüche 1 bis 7 oder die einkettige Meganuklease nach Anspruch 8 codiert.

10. Rekombinanter Vektor, der mindestens ein Polynukleotidfragment nach Anspruch 9 umfasst; insbesondere ein Expressionsvektor, der zwei Polynukleotidfragmente umfasst, von denen jedes für eines der zwei Monomere der obligaten heterodimeren I-CreI nach einem der Ansprüche 1 bis 7 codiert, wobei das/die beschriebenen Fragment(-e) operativ mit regulatorischen Sequenzen verbunden sind, welche die Herstellung der zwei Monomere ermöglichen; wobei insbesondere der Vektor ein Ziel-DNS-Konstrukt beinhaltet, welches Sequenzen umfasst, die Homologien mit der Region teilen, welche die Hybrid-DNS-Zielsequenz, wie in Anspruch 1 definiert, umgibt.

11. Wirtszelle, nicht menschliches transgenes Tier oder transgene Pflanze, umfassend ein oder zwei Polynukleotidfragmente, wie in Anspruch 9 definiert, oder einen Vektor nach Anspruch 10.

12. Zusammensetzung, die mindestens eine obligate Heterodimer I-CreI nach einem der Ansprüche 1 bis 7 umfasst, eine einkettige I-CreI nach Anspruch 8, ein oder zwei Polynukleotidfragmente, wie in Anspruch 9 definiert, oder einen Vektor nach Anspruch 10, wobei insbesondere die Zusammensetzung ferner ein Ziel-DNS-Konstrukt umfasst, dass die Sequenz umfasst, welche die Stelle von Interesse repariert, die von Sequenzen flankiert wird, die Homologien mit dem Ziellokus teilen.

13. Verwendung mindestens einer obligaten Heterodimer I-CreI nach einem der Ansprüche 1 bis 7, einer einkettigen I-CreI nach Anspruch 8, eines oder zweier Polynukleotid Fragmente, wie in Anspruch 9 definiert, eines Vektors nach Anspruch 10, für die Herstellung eines Medikamentes, zum Verhindern, Verbessern oder Heilen einer genetischen Erkrankung oder Infektionserkrankung an einem Individuum, welches dieses benötigt, wobei das Medikament dem Individuum auf irgendeine Weise verabreicht werden kann.

14. Verwendung mindestens einer obligaten Heterodimer I-CreI nach einem der Ansprüche 1 bis 7, einer einkettigen I-CreI nach Anspruch 8, eines oder zweier Polynukleotidfragmente, wie in Anspruch 9 definiert, eines Vektors nach Anspruch 10, zum Verhindern der Ausbreitung, Inaktivierung oder Auslöschung eines infektiösen Agens, das ein DNS-Intermediat darstellt, in biologisch hergestellten Produkten oder Produkten, die für biologische Verwendungen vorgesehen sind, oder zum Desinfizieren eines Objektes, wobei insbesondere das infektiöse Agens ein Virus ist.

## Revendications

1. Méganucléase hétérodimérique obligatoire **caractérisée en ce qu'**elle est constituée par un premier et un second monomères, provenant de deux monomères I-*Cre*I homodimériques différents (monomères parents), et ayant au moins une paire de mutations dans des résidus desdits monomères parents, qui ont une interréaction intermoléculaire entre les deux monomères de chaque I-*Cre*I homodimérique parent, et que la première mutation de ladite (desdites) paire(s) se trouve dans le premier monomère et que la deuxième mutation de ladite (desdites) paire(s) se trouve dans le second monomère et que ladite (lesdites) paire(s) de mutation(s) altère(nt) la formation d'homodimères fonctionnels à partir de chaque monomère sans empêcher la formation d'un hétérodimère fonctionnel capable de couper une cible d'ADN hybride non palindromique, comprenant une moitié différente de la cible d'ADN coupée par chaque I-*Cre*I homodimérique parent, dans laquelle les monomères ont au moins l'une des paires de mutations suivantes, respectivement pour les premier et second monomères :
a) la substitution de la leucine en position 97 par un acide aminé aromatique et la substitution de la phénylalanine en position 54 par un petit acide aminé, et
b) la substitution de l'acide aspartique en position 137 par un acide aminé basique et la substitution de l'arginine en position 51 par un acide aminé acide,
lesdites positions étant indiquées par référence à la séquence d'acides aminés SEQ ID n° : 1 d'I-CreI.

2. I-*Cre*I hétérodimérique obligatoire selon la revendication 1, dans laquelle les monomères comprennent également au moins l'une des paires de mutations suivantes, respectivement pour les premier et second monomères :
c) la substitution de l'acide glutamique en position 8 par un acide aminé basique et la substitution de la lysine en position 7 par un acide aminé acide,
d) la substitution de l'acide glutamique en position 61 par un acide aminé basique et la substitution de la lysine en position 96 par un acide aminé acide,
lesdites positions étant indiquées par référence à la séquence d'acides aminés SEQ ID n° : 1 d'I-CreI.

3. I-*Cre*I hétérodimérique obligatoire selon la revendication 1 ou la revendication 2, dans laquelle le monomère ayant la substitution de l'acide glutamique en position 8 ou 61 par un acide aminé basique, tel que défini en c) ou en d), comprend en outre la substitution d'au moins l'un des résidus de lysine aux positions 7 et 96, par une arginine ; et/ou dans laquelle le monomère ayant la substitution de la leucine 97 par un acide aminé aromatique, tel que défini en a), comprend en outre la substitution de la phénylalanine en position 54 par un tryptophane ; et/ou dans laquelle le monomère ayant la substitution de la phénylalanine en position 54 par un petit acide aminé, tel que défini en a), comprend en outre la substitution de la leucine en position 58 ou de la lysine en position 57, par une méthionine.

4. I-*Cre*I hétérodimérique obligatoire selon la revendication 1 ou la revendication 2, dans laquelle ledit acide aminé acide est un acide glutamique ;
dans laquelle ledit acide aminé basique est une arginine ;
dans laquelle ledit acide aminé aromatique est une phénylalanine ;
dans laquelle ledit petit acide aminé est une glycine.

5. I-*Cre*I hétérodimérique obligatoire, selon l'une quelconque des revendications 1 à 4, qui est constituée par un premier monomère, ayant au moins la mutation D137R, et un second monomère ayant au moins la mutation R51D.

6. I-*Cre*I hétérodimérique obligatoire selon la revendication 1, qui est constituée par un premier monomère, ayant au moins les mutations K7R, E8R, E61R, K96R et L97F ou K7R, E8R, F54W, E61R, K96R et L97F, ainsi qu'un second monomère ayant au moins les mutations K7E, F54G, L58M et K96E ou K7E, F54G, K57M et K96E.

7. I-*Cre*I hétérodimérique obligatoire, selon l'une quelconque des revendications 1 à 6, qui est constituée par deux variants monomériques d'I-*Cre*I, comprenant en outre différentes mutations aux positions 26 à 40 et 44 à 77 d'I-*Cre*I, en particulier dans laquelle ladite méganucléase hétérodimérique obligatoire est capable de couper une cible d'ADN hybride non palindromique provenant d'une séquence génomique d'intérêt, qui correspond à une moitié différente de la cible d'ADN qui est coupée par chaque endonucléase homodimérique parente.

8. I-*Cre*I monocaténaire **caractérisée en ce que** les premier et second monomères de la méganucléase hétérodimérique obligatoire, telle que définie dans l'une quelconque des revendications 1 à 7, sont reliés par un lieur peptidique.

9. Fragment de polynucléotides codant pour le monomère de ladite I-*Cre*I hétérodimérique obligatoire, selon l'une quelconque des revendications 1 à 7, ou la méganucléase monocaténaire, selon la revendication 8.

10. Vecteur recombinant comprenant au moins un fragment de polynucléotides, selon la revendication 9 ; en particulier, un vecteur d'expression comprenant deux fragments de polynucléotides, codant chacun pour l'un des deux monomères de l'I-*Cre*I hétérodimérique obligatoire, selon l'une quelconque des revendications 1 à 7, ledit (lesdits) fragment(s) étant lié(s), de manière opérationnelle, à des séquences de régulation qui permettent la production des deux monomères ; en particulier, dans laquelle ledit vecteur comprend une construction d'ADN de ciblage comprenant des séquences qui partagent des homologies avec la région entourant la séquence de la cible d'ADN hybride, telle que définie dans la revendication 1.

11. Cellule hôte, animal transgénique non humain ou plante transgénique comprenant un ou deux fragment(s) de polynucléotides, tel(s) que défini(s) dans la revendication 9, ou un vecteur selon la revendication 10.

12. Composition comprenant au moins une I-*Cre*I hétérodimérique obligatoire, selon l'une quelconque des revendications 1 à 7, une I-*Cre*I monocaténaire, selon la revendication 8, un ou deux fragment(s) de polynucléotides, tel(s) que défini(s) dans la revendication 9, ou un vecteur, selon la revendication 10, en particulier dans laquelle ladite composition comprend en outre une construction d'ADN de ciblage comprenant la séquence qui répare le site d'intérêt flanqué par des séquences qui partagent des homologies avec le locus ciblé.

13. Utilisation d'au moins une I-*Cre*I hétérodimérique obligatoire, selon l'une quelconque des revendications 1 à 7, une I-*Cre*I monocaténaire, selon la revendication 8, un ou deux fragment(s) de polynucléotides, tel(s) que défini(s) dans la revendication 9, un vecteur, selon la revendication 10, pour la préparation d'un médicament destiné à prévenir, améliorer ou guérir une maladie génétique ou une maladie infectieuse chez un individu le nécessitant, ledit médicament étant administré par n'importe quel moyen au dit individu.

14. Utilisation d'au moins une I-*Cre*I hétérodimérique obligatoire, selon l'une quelconque des revendications 1 à 7, une I-*Cre*I monocaténaire, selon la revendication 8, un ou deux fragment(s) de polynucléotides, tel(s) que défini(s) dans la revendication 9, un vecteur, selon la revendication 10, *in* vitro, pour inhiber la propagation, l'inactivation ou la suppression d'un agent infectieux qui présente un intermédiaire d'ADN, dans des produits biologiques dérivés ou des produits étant destinés à des utilisations biologiques ou bien pour désinfecter un objet, en particulier dans laquelle ledit agent infectieux est un virus.


### SEQUENCE LISTING

<110> CELLECTIS
   FAJARDO SANCHEZ, Emmanuel
   GRIZOT, Sylvestre
   ISALAN, Mark
   SERRANO PUBUL, Luis
   STRICHER, François
<120> obligate heterodimer meganucleases and uses thereof
<130> HLP/bv-1546/14PCT
<160> 65
<170> PatentIn version 3.3
<210> 1
   <211> 163
   <212> PRT
   <213> Chlamydomonas reinhardtii
<400> 1
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Lys Pro Asn Gln Ser
   20 25 30
   Tyr Lys Phe Lys His Gln Leu Ser Leu Ala Phe Gln Val Thr Gln Lys
   35 40 45
   Thr Gln Arg Arg Trp Phe Leu Asp Lys Leu Val Asp Glu Ile Gly Val
   50 55 60
   Gly Tyr Val Arg Asp Arg Gly Ser Val Ser Asp Tyr Ile Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 2
   <211> 24
   <212> DNA
   <213> Artificial sequence
<220>
   <223> I-CreI target DNA
<400> 2
   tcaaaacgtc gtacgacgtt ttga 24
<210> 3
   <211> 41
   <212> DNA
   <213> Artificial sequence
<220>
   <223> Primer
<400> 3
   caataccaaa tataacaggc ggttcctgct gtacctggcc g 41
<210> 4
   <211> 41
   <212> DNA
   <213> Artificial sequence
<220>
   <223> Primer
<400> 4
   cggccaggta cagcaggaac cgcctgttat atttggtatt g 41
<210> 5
   <211> 44
   <212> DNA
   <213> Artificial sequence
<220>
   <223> Primer
<400> 5
   tcaactgcag ccgtttctga gattcaaaca gaaacaggca aacc 44
<210> 6
   <211> 44
   <212> DNA
   <213> Artificial sequence
<220>
   <223> Primer
<400> 6
   ggtttgcctg tttctgtttg aatctcagaa acggctgcag ttga 44
<210> 7
   <211> 53
   <212> DNA
   <213> Artificial sequence
<220>
   <223> Primer
<400> 7
   ccagcgccgt tggtggctgg acaaactagt ggatagaatt ggcgttggtt acg 53
<210> 8
   <211> 53
   <212> DNA
   <213> Artificial sequence
<220>
   <223> Primer
<400> 8
   cgtaaccaac gccaattcta tccactagtt tgtccagcca ccaacggcgc tgg 53
<210> 9
   <211> 41
   <212> DNA
   <213> Artificial sequence
<220>
   <223> Primer
<400> 9
   caataccaaa tataacgaag agttcctgct gtacctggcc g 41
<210> 10
   <211> 41
   <212> DNA
   <213> Artificial sequence
<220>
   <223> Primer
<400> 10
   cggccaggta cagcaggaac tcttcgttat atttggtatt g 41
<210> 11
   <211> 53
   <212> DNA
   <213> Artificial sequence
<220>
   <223> Primer
<400> 11
   ccagcgccgt tggggtctgg acaaaatggt ggatgaaatt ggcgttggtt acg 53
<210> 12
   <211> 53
   <212> DNA
   <213> Artificial sequence
<220>
   <223> Primer
<400> 12
   cgtaaccaac gccaatttca tccaccattt tgtccagacc ccaacggcgc tgg 53
<210> 13
   <211> 44
   <212> DNA
   <213> Artificial sequence
<220>
   <223> Primer
<400> 13
   tcaactgcag ccgtttctgg aactgaaaca gaaacaggca aacc 44
<210> 14
   <211> 44
   <212> DNA
   <213> Artificial sequence
<220>
   <223> Primer
<400> 14
   ggtttgcctg tttctgtttc agttccagaa acggctgcag ttga 44
<210> 15
   <211> 528
   <212> DNA
   <213> Artificial sequence
<220>
   <223> QAN variant
<220>
   <221> CDS
   <222> (1)..(528)
<400> 15
   atg gcc aat acc aaa tat aac aaa gag ttc ctg ctg tac ctg gcc ggc 48
   Met Ala Asn Thr Lys Tyr Asn Lys Glu Phe Leu Leu Tyr Leu Ala Gly
   1 5 10 15
   ttt gtg gac ggt gac ggt agc atc atc gct cag att aaa cca aac cag 96
   Phe Val Asp Gly Asp Gly Ser Ile Ile Ala Gln Ile Lys Pro Asn Gln
   20 25 30
   tct tat aag ttt aaa cat cag cta agc ttg acc ttt cag gtg act caa 144
   Ser Tyr Lys Phe Lys His Gln Leu Ser Leu Thr Phe Gln Val Thr Gln
   35 40 45
   aag acc cag cgc cgt tgg ttt ctg gac aaa cta gtg gat gaa att ggc 192
   Lys Thr Gln Arg Arg Trp Phe Leu Asp Lys Leu Val Asp Glu Ile Gly
   50 55 60
   gtt ggt tac gta gct gat aat gga tcc gtt tcc aac tac atc tta agc 240
   Val Gly Tyr Val Ala Asp Asn Gly Ser Val Ser Asn Tyr Ile Leu Ser
   65 70 75 80
   gaa atc aag ccg ctg cac aac ttc ctg act caa ctg cag ccg ttt ctg 288
   Glu Ile Lys Pro Leu His Asn Phe Leu Thr Gln Leu Gln Pro Phe Leu
   85 90 95
   aaa ctg aaa cag aaa cag gca aac ctg gtt ctg aaa att atc gaa cag 336
   Lys Leu Lys Gln Lys Gln Ala Asn Leu Val Leu Lys Ile Ile Glu Gln
   100 105 110
   ctg ccg tct gca aaa gaa tcc ccg gac aaa ttc ctg gaa gtt tgt acc 384
   Leu Pro Ser Ala Lys Glu Ser Pro Asp Lys Phe Leu Glu Val Cys Thr
   115 120 125
   tgg gtg gat cag att gca gct ctg aac gat tct aag acg cgt aaa acc 432
   Trp Val Asp Gln Ile Ala Ala Leu Asn Asp Ser Lys Thr Arg Lys Thr
   130 135 140
   act tct gaa acc gtt cgt gct gtg ctg gac agc ctg agc gag aag aag 480
   Thr Ser Glu Thr Val Arg Ala Val Leu Asp Ser Leu Ser Glu Lys Lys
   145 150 155 160
   aaa tcc tcc ccg gcg gcc gca ctc gag cac cac cac cac cac cac tga 528
   Lys Ser Ser Pro Ala Ala Ala Leu Glu His His His His His His
   165 170 175
<210> 16
   <211> 175
   <212> PRT
   <213> Artificial sequence
<220>
   <223> Synthetic Construct
<400> 16
   Met Ala Asn Thr Lys Tyr Asn Lys Glu Phe Leu Leu Tyr Leu Ala Gly
   1               5                   10                  15
   Phe Val Asp Gly Asp Gly Ser Ile Ile Ala Gln Ile Lys Pro Asn Gln
               20                  25                  30
   Ser Tyr Lys Phe Lys His Gln Leu Ser Leu Thr Phe Gln Val Thr Gln
           35                  40                  45
   Lys Thr Gln Arg Arg Trp Phe Leu Asp Lys Leu Val Asp Glu Ile Gly
       50                  55                  60
   Val Gly Tyr Val Ala Asp Asn Gly Ser Val Ser Asn Tyr Ile Leu Ser
   65                  70                  75                  80
   Glu Ile Lys Pro Leu His Asn Phe Leu Thr Gln Leu Gln Pro Phe Leu
                   85                  90                  95
   Lys Leu Lys Gln Lys Gln Ala Asn Leu Val Leu Lys Ile Ile Glu Gln
               100                 105                 110
   Leu Pro Ser Ala Lys Glu Ser Pro Asp Lys Phe Leu Glu Val Cys Thr
           115                 120                 125
   Trp Val Asp Gln Ile Ala Ala Leu Asn Asp Ser Lys Thr Arg Lys Thr
       130                 135                 140
   Thr Ser Glu Thr Val Arg Ala Val Leu Asp Ser Leu Ser Glu Lys Lys
   145                 150                 155                 160
   Lys Ser Ser Pro Ala Ala Ala Leu Glu His His His His His His
                   165                 170                 175
<210> 17
   <211> 528
   <212> DNA
   <213> Artificial sequence
<220>
   <223> KTG variant
<220>
   <221> CDS
   <222> (1)..(528)
<400> 17
   atg gcc aat acc aaa tat aac aaa gag ttc ctg ctg tac ctg gcc ggc      48
   Met Ala Asn Thr Lys Tyr Asn Lys Glu Phe Leu Leu Tyr Leu Ala Gly
   1               5                   10                  15
   ttt gtg gac ggt gac ggt agc atc atc gct cag att aaa cca aac cag      96
   Phe Val Asp Gly Asp Gly Ser Ile Ile Ala Gln Ile Lys Pro Asn Gln
               20                  25                  30
   tct tat aag ttt aaa cat cag cta agc ttg acc ttt aag gtg act caa     144
   Ser Tyr Lys Phe Lys His Gln Leu Ser Leu Thr Phe Lys Val Thr Gln
           35                  40                  45
   aag acc cag cgc cgt tgg ttt ctg gac aaa cta gtg gat gaa att ggc     192
   Lys Thr Gln Arg Arg Trp Phe Leu Asp Lys Leu Val Asp Glu Ile Gly
       50                  55                  60
   gtt ggt tac gta acg gat ggg gga tcc gtt tcc aac tac atc tta agc     240
   Val Gly Tyr Val Thr Asp Gly Gly Ser Val Ser Asn Tyr Ile Leu Ser
   65                  70                  75                  80
   gaa atc aag ccg ctg cac aac ttc ctg act caa ctg cag ccg ttt ctg     288
   Glu Ile Lys Pro Leu His Asn Phe Leu Thr Gln Leu Gln Pro Phe Leu
                   85                  90                  95
   aaa ctg aaa cag aaa cag gca aac ctg gtt ctg aaa att atc gaa cag     336
   Lys Leu Lys Gln Lys Gln Ala Asn Leu Val Leu Lys Ile Ile Glu Gln
               100                 105                 110
   ctg ccg tct gca aaa gaa tcc ccg gac aaa ttc ctg gaa gtt tgt acc     384
   Leu Pro Ser Ala Lys Glu Ser Pro Asp Lys Phe Leu Glu Val Cys Thr
           115                 120                 125
   tgg gtg gat cag att gca gct ctg aac gat tct aag acg cgt aaa acc     432
   Trp Val Asp Gln Ile Ala Ala Leu Asn Asp Ser Lys Thr Arg Lys Thr
       130                 135                 140
   act tct gaa acc gtt cgt gct gtg ctg gac agc ctg agc gag aag aag     480
   Thr Ser Glu Thr Val Arg Ala Val Leu Asp Ser Leu Ser Glu Lys Lys
   145                 150                 155                 160
   aaa tcc tcc ccg gcg gcc gca ctc gag cac cac cac cac cac cac tga     528
   Lys Ser Ser Pro Ala Ala Ala Leu Glu His His His His His His
                   165                 170                 175
<210> 18
   <211> 175
   <212> PRT
   <213> Artificial sequence
<220>
   <223> Synthetic Construct
<400> 18
   Met Ala Asn Thr Lys Tyr Asn Lys Glu Phe Leu Leu Tyr Leu Ala Gly
   1               5                   10                  15
   Phe Val Asp Gly Asp Gly Ser Ile Ile Ala Gln Ile Lys Pro Asn Gln
               20                  25                  30
   Ser Tyr Lys Phe Lys His Gln Leu Ser Leu Thr Phe Lys Val Thr Gln
           35                  40                  45
   Lys Thr Gln Arg Arg Trp Phe Leu Asp Lys Leu Val Asp Glu Ile Gly
       50                  55                  60
   Val Gly Tyr Val Thr Asp Gly Gly Ser Val Ser Asn Tyr Ile Leu Ser
   65                  70                  75                  80
   Glu Ile Lys Pro Leu His Asn Phe Leu Thr Gln Leu Gln Pro Phe Leu
                   85                  90                  95
   Lys Leu Lys Gln Lys Gln Ala Asn Leu Val Leu Lys Ile Ile Glu Gln
               100                 105                 110
   Leu Pro Ser Ala Lys Glu Ser Pro Asp Lys Phe Leu Glu Val Cys Thr
           115                 120                 125
   Trp Val Asp Gln Ile Ala Ala Leu Asn Asp Ser Lys Thr Arg Lys Thr
       130                 135                 140
   Thr Ser Glu Thr Val Arg Ala Val Leu Asp Ser Leu Ser Glu Lys Lys
   145                 150                 155                 160
   Lys Ser Ser Pro Ala Ala Ala Leu Glu His His His His His His
                   165                 170                 175
<210> 19
   <211> 528
   <212> DNA
   <213> Artificial sequence
<220>
   <223> QAN-A1 variant
<220>
   <221> CDS
   <222> (1)..(528)
<400> 19
   atg gcc aat acc aaa tat aac agg cgg ttc ctg ctg tac ctg gcc ggc      48
   Met Ala Asn Thr Lys Tyr Asn Arg Arg Phe Leu Leu Tyr Leu Ala Gly
   1               5                   10                  15
   ttt gtg gac ggt gac ggt agc atc atc gct cag att aaa cca aac cag      96
   Phe Val Asp Gly Asp Gly Ser Ile Ile Ala Gln Ile Lys Pro Asn Gln
               20                  25                  30
   tct tat aag ttt aaa cat cag cta agc ttg acc ttt cag gtg act caa     144
   Ser Tyr Lys Phe Lys His Gln Leu Ser Leu Thr Phe Gln Val Thr Gln
           35                  40                  45
   aag acc cag cgc cgt tgg ttt ctg gac aaa cta gtg gat aga att ggc      192
   Lys Thr Gln Arg Arg Trp Phe Leu Asp Lys Leu Val Asp Arg Ile Gly
       50                  55                  60
   gtt ggt tac gta gct gat aat gga tcc gtt tcc aac tac atc tta agc      240
   Val Gly Tyr Val Ala Asp Asn Gly Ser Val Ser Asn Tyr Ile Leu Ser
   65                  70                  75                  80
   gaa atc aag ccg ctg cac aac ttc ctg act caa ctg cag ccg ttt ctg      288
   Glu Ile Lys Pro Leu His Asn Phe Leu Thr Gln Leu Gln Pro Phe Leu
                   85                  90                  95
   aga ttc aaa cag aaa cag gca aac ctg gtt ctg aaa att atc gaa cag      336
   Arg Phe Lys Gln Lys Gln Ala Asn Leu Val Leu Lys Ile Ile Glu Gln
               100                 105                 110
   ctg ccg tct gca aaa gaa tcc ccg gac aaa ttc ctg gaa gtt tgt acc      384
   Leu Pro Ser Ala Lys Glu Ser Pro Asp Lys Phe Leu Glu Val Cys Thr
           115                 120                 125
   tgg gtg gat cag att gca gct ctg aac gat tct aag acg cgt aaa acc      432
   Trp Val Asp Gln Ile Ala Ala Leu Asn Asp Ser Lys Thr Arg Lys Thr
       130                 135                 140
   act tct gaa acc gtt cgt gct gtg ctg gac agc ctg agc gag aag aag      480
   Thr Ser Glu Thr Val Arg Ala Val Leu Asp Ser Leu Ser Glu Lys Lys
   145                 150                 155                 160
   aaa tcc tcc ccg gcg gcc gca ctc gag cac cac cac cac cac cac tga      528
   Lys Ser Ser Pro Ala Ala Ala Leu Glu His His His His His His
                   165                 170                 175
<210> 20
   <211> 175
   <212> PRT
   <213> Artificial sequence
<220>
   <223> Synthetic Construct
<400> 20
   Met Ala Asn Thr Lys Tyr Asn Arg Arg Phe Leu Leu Tyr Leu Ala Gly
   1 5 10 15
   Phe Val Asp Gly Asp Gly Ser Ile Ile Ala Gln Ile Lys Pro Asn Gln
   20 25 30
   Ser Tyr Lys Phe Lys His Gln Leu Ser Leu Thr Phe Gln Val Thr Gln
   35 40 45
   Lys Thr Gln Arg Arg Trp Phe Leu Asp Lys Leu Val Asp Arg Ile Gly
   50 55 60
   Val Gly Tyr Val Ala Asp Asn Gly Ser Val Ser Asn Tyr Ile Leu Ser
   65 70 75 80
   Glu Ile Lys Pro Leu His Asn Phe Leu Thr Gln Leu Gln Pro Phe Leu
   85 90 95
   Arg Phe Lys Gln Lys Gln Ala Asn Leu Val Leu Lys Ile Ile Glu Gln
   100 105 110
   Leu Pro Ser Ala Lys Glu Ser Pro Asp Lys Phe Leu Glu Val Cys Thr
   115 120 125
   Trp Val Asp Gln Ile Ala Ala Leu Asn Asp Ser Lys Thr Arg Lys Thr
   130 135 140
   Thr Ser Glu Thr Val Arg Ala Val Leu Asp Ser Leu Ser Glu Lys Lys
   145 150 155 160
   Lys Ser Ser Pro Ala Ala Ala Leu Glu His His His His His His
   165 170 175
<210> 21
   <211> 528
   <212> DNA
   <213> Artificial sequence
<220>
   <223> QAN-A2 variant
<220>
   <221> CDS
   <222> (1)..(528)
<400> 21
   atg gcc aat acc aaa tat aac agg cgg ttc ctg ctg tac ctg gcc ggc      48
   Met Ala Asn Thr Lys Tyr Asn Arg Arg Phe Leu Leu Tyr Leu Ala Gly
   1               5                   10                  15
   ttt gtg gac ggt gac ggt agc atc atc gct cag att aaa cca aac cag      96
   Phe Val Asp Gly Asp Gly Ser Ile Ile Ala Gln Ile Lys Pro Asn Gln
               20                  25                  30
   tct tat aag ttt aaa cat cag cta agc ttg acc ttt cag gtg act caa      144
   Ser Tyr Lys Phe Lys His Gln Leu Ser Leu Thr Phe Gln Val Thr Gln
           35                  40                  45
   aag acc cag cgc cgt tgg tgg ctg gac aaa cta gtg gat aga att ggc      192
   Lys Thr Gln Arg Arg Trp Trp Leu Asp Lys Leu Val Asp Arg Ile Gly
       50                  55                  60
   gtt ggt tac gta gct gat aat gga tcc gtt tcc aac tac atc tta agc      240
   Val Gly Tyr Val Ala Asp Asn Gly Ser Val Ser Asn Tyr Ile Leu Ser
   65                  70                  75                  80
   gaa atc aag ccg ctg cac aac ttc ctg act caa ctg cag ccg ttt ctg      288
   Glu Ile Lys Pro Leu His Asn Phe Leu Thr Gln Leu Gln Pro Phe Leu
                   85                  90                  95
   aga ttc aaa cag aaa cag gca aac ctg gtt ctg aaa att atc gaa cag      336
   Arg Phe Lys Gln Lys Gln Ala Asn Leu Val Leu Lys Ile Ile Glu Gln
               100                 105                 110
   ctg ccg tct gca aaa gaa tcc ccg gac aaa ttc ctg gaa gtt tgt acc      384
   Leu Pro Ser Ala Lys Glu Ser Pro Asp Lys Phe Leu Glu Val Cys Thr
           115                 120                 125
   tgg gtg gat cag att gca gct ctg aac gat tct aag acg cgt aaa acc      432
   Trp Val Asp Gln Ile Ala Ala Leu Asn Asp Ser Lys Thr Arg Lys Thr
       130                 135                 140
   act tct gaa acc gtt cgt gct gtg ctg gac agc ctg agc gag aag aag      480
   Thr Ser Glu Thr Val Arg Ala Val Leu Asp Ser Leu Ser Glu Lys Lys
   145                 150                 155                 160
   aaa tcc tcc ccg gcg gcc gca ctc gag cac cac cac cac cac cac tga      528
   Lys Ser Ser Pro Ala Ala Ala Leu Glu His His His His His His
                   165                 170                 175
<210> 22
   <211> 175
   <212> PRT
   <213> Artificial sequence
<220>
   <223> Synthetic Construct
<400> 22
   Met Ala Asn Thr Lys Tyr Asn Arg Arg Phe Leu Leu Tyr Leu Ala Gly
   1               5                   10                  15
   Phe Val Asp Gly Asp Gly Ser Ile Ile Ala Gln Ile Lys Pro Asn Gln
               20                  25                  30
   Ser Tyr Lys Phe Lys His Gln Leu Ser Leu Thr Phe Gln Val Thr Gln
           35                  40                  45
   Lys Thr Gln Arg Arg Trp Trp Leu Asp Lys Leu Val Asp Arg Ile Gly
       50                  55                  60
   Val Gly Tyr Val Ala Asp Asn Gly Ser Val Ser Asn Tyr Ile Leu Ser
   65                  70                  75                  80
   Glu Ile Lys Pro Leu His Asn Phe Leu Thr Gln Leu Gln Pro Phe Leu
                   85                  90                  95
   Arg Phe Lys Gln Lys Gln Ala Asn Leu Val Leu Lys Ile Ile Glu Gln
               100                 105                 110
   Leu Pro Ser Ala Lys Glu Ser Pro Asp Lys Phe Leu Glu Val Cys Thr
           115                 120                 125
   Trp Val Asp Gln Ile Ala Ala Leu Asn Asp Ser Lys Thr Arg Lys Thr
       130                 135                 140
   Thr Ser Glu Thr Val Arg Ala Val Leu Asp Ser Leu Ser Glu Lys Lys
   145                 150                 155                 160
   Lys Ser Ser Pro Ala Ala Ala Leu Glu His His His His His His
                   165                 170                 175
<210> 23
   <211> 528
   <212> DNA
   <213> Artificial sequence
<220>
   <223> KTG-A1 variant
<220>
   <221> CDS
   <222> (1)..(528)
<400> 23
   atg gcc aat acc aaa tat aac agg cgg ttc ctg ctg tac ctg gcc ggc      48
   Met Ala Asn Thr Lys Tyr Asn Arg Arg Phe Leu Leu Tyr Leu Ala Gly
   1               5                   10                  15
   ttt gtg gac ggt gac ggt agc atc atc gct cag att aaa cca aac cag      96
   Phe Val Asp Gly Asp Gly Ser Ile Ile Ala Gln Ile Lys Pro Asn Gln
               20                  25                  30
   tct tat aag ttt aaa cat cag cta agc ttg acc ttt aag gtg act caa      144
   Ser Tyr Lys Phe Lys His Gln Leu Ser Leu Thr Phe Lys Val Thr Gln
           35                  40                  45
   aag acc cag cgc cgt tgg ttt ctg gac aaa cta gtg gat aga att ggc      192
   Lys Thr Gln Arg Arg Trp Phe Leu Asp Lys Leu Val Asp Arg Ile Gly
       50                  55                  60
   gtt ggt tac gta acg gat ggg gga tcc gtt tcc aac tac atc tta agc      240
   Val Gly Tyr Val Thr Asp Gly Gly Ser Val Ser Asn Tyr Ile Leu Ser
   65                  70                  75                  80
   gaa atc aag ccg ctg cac aac ttc ctg act caa ctg cag ccg ttt ctg      288
   Glu Ile Lys Pro Leu His Asn Phe Leu Thr Gln Leu Gln Pro Phe Leu
                   85                  90                  95
   aga ttc aaa cag aaa cag gca aac ctg gtt ctg aaa att atc gaa cag      336
   Arg Phe Lys Gln Lys Gln Ala Asn Leu Val Leu Lys Ile Ile Glu Gln
               100                 105                 110
   ctg ccg tct gca aaa gaa tcc ccg gac aaa ttc ctg gaa gtt tgt acc      384
   Leu Pro Ser Ala Lys Glu Ser Pro Asp Lys Phe Leu Glu Val Cys Thr
           115                 120                 125
   tgg gtg gat cag att gca gct ctg aac gat tct aag acg cgt aaa acc      432
   Trp Val Asp Gln Ile Ala Ala Leu Asn Asp Ser Lys Thr Arg Lys Thr
       130                 135                 140
   act tct gaa acc gtt cgt gct gtg ctg gac agc ctg agc gag aag aag      480
   Thr Ser Glu Thr Val Arg Ala Val Leu Asp Ser Leu Ser Glu Lys Lys
   145                 150                 155                 160
   aaa tcc tcc ccg gcg gcc gca ctc gag cac cac cac cac cac cac tga      528
   Lys Ser Ser Pro Ala Ala Ala Leu Glu His His His His His His
                   165                 170                 175
<210> 24
   <211> 175
   <212> PRT
   <213> Artificial sequence
<220>
   <223> Synthetic construct
<400> 24
   Met Ala Asn Thr Lys Tyr Asn Arg Arg Phe Leu Leu Tyr Leu Ala Gly
   1               5                   10                  15
   Phe Val Asp Gly Asp Gly Ser Ile Ile Ala Gln Ile Lys Pro Asn Gln
               20                  25                  30
   Ser Tyr Lys Phe Lys His Gln Leu Ser Leu Thr Phe Lys Val Thr Gln
           35                  40                  45
   Lys Thr Gln Arg Arg Trp Phe Leu Asp Lys Leu Val Asp Arg Ile Gly
       50                  55                  60
   Val Gly Tyr Val Thr Asp Gly Gly Ser Val Ser Asn Tyr Ile Leu Ser
   65                  70                  75                  80
   Glu Ile Lys Pro Leu His Asn Phe Leu Thr Gln Leu Gln Pro Phe Leu
                   85                  90                  95
   Arg Phe Lys Gln Lys Gln Ala Asn Leu Val Leu Lys Ile Ile Glu Gln
               100                 105                 110
   Leu Pro Ser Ala Lys Glu Ser Pro Asp Lys Phe Leu Glu Val Cys Thr
           115                 120                 125
   Trp Val Asp Gln Ile Ala Ala Leu Asn Asp Ser Lys Thr Arg Lys Thr
       130                 135                 140
   Thr Ser Glu Thr Val Arg Ala Val Leu Asp Ser Leu Ser Glu Lys Lys
   145                 150                 155                 160
   Lys Ser Ser Pro Ala Ala Ala Leu Glu His His His His His His
                   165                 170                 175
<210> 25
   <211> 528
   <212> DNA
   <213> Artificial sequence
<220>
   <223> KTG-A2 variant
<220>
   <221> CDS
   <222> (1)..(528)
<400> 25
   atg gcc aat acc aaa tat aac agg cgg ttc ctg ctg tac ctg gcc ggc      48
   Met Ala Asn Thr Lys Tyr Asn Arg Arg Phe Leu Leu Tyr Leu Ala Gly
   1               5                   10                  15
   ttt gtg gac ggt gac ggt agc atc atc gct cag att aaa cca aac cag      96
   Phe Val Asp Gly Asp Gly Ser Ile Ile Ala Gln Ile Lys Pro Asn Gln
               20                  25                  30
   tct tat aag ttt aaa cat cag cta agc ttg acc ttt aag gtg act caa      144
   Ser Tyr Lys Phe Lys His Gln Leu Ser Leu Thr Phe Lys Val Thr Gln
           35                  40                  45
   aag acc cag cgc cgt tgg tgg ctg gac aaa cta gtg gat aga att ggc      192
   Lys Thr Gln Arg Arg Trp Trp Leu Asp Lys Leu Val Asp Arg Ile Gly
       50                  55                  60
   gtt ggt tac gta acg gat ggg gga tcc gtt tcc aac tac atc tta agc      240
   Val Gly Tyr Val Thr Asp Gly Gly Ser Val Ser Asn Tyr Ile Leu Ser
   65                  70                  75                  80
   gaa atc aag ccg ctg cac aac ttc ctg act caa ctg cag ccg ttt ctg      288
   Glu Ile Lys Pro Leu His Asn Phe Leu Thr Gln Leu Gln Pro Phe Leu
                   85                  90                  95
   aga ttc aaa cag aaa cag gca aac ctg gtt ctg aaa att atc gaa cag      336
   Arg Phe Lys Gln Lys Gln Ala Asn Leu Val Leu Lys Ile Ile Glu Gln
               100                 105                 110
   ctg ccg tct gca aaa gaa tcc ccg gac aaa ttc ctg gaa gtt tgt acc      384
   Leu Pro Ser Ala Lys Glu Ser Pro Asp Lys Phe Leu Glu Val Cys Thr
           115                 120                 125
   tgg gtg gat cag att gca gct ctg aac gat tct aag acg cgt aaa acc      432
   Trp Val Asp Gln Ile Ala Ala Leu Asn Asp Ser Lys Thr Arg Lys Thr
       130                 135                 140
   act tct gaa acc gtt cgt gct gtg ctg gac agc ctg agc gag aag aag      480
   Thr Ser Glu Thr Val Arg Ala Val Leu Asp Ser Leu Ser Glu Lys Lys
   145                 150                 155                 160
   aaa tcc tcc ccg gcg gcc gca ctc gag cac cac cac cac cac cac tga      528
   Lys Ser Ser Pro Ala Ala Ala Leu Glu His His His His His His
                   165                 170                 175
<210> 26
   <211> 175
   <212> PRT
   <213> Artificial sequence
<220>
   <223> Synthetic Construct
<400> 26
   Met Ala Asn Thr Lys Tyr Asn Arg Arg Phe Leu Leu Tyr Leu Ala Gly
   1               5                   10                  15
   Phe Val Asp Gly Asp Gly Ser Ile Ile Ala Gln Ile Lys Pro Asn Gln
               20                  25                  30
   Ser Tyr Lys Phe Lys His Gln Leu Ser Leu Thr Phe Lys Val Thr Gln
           35                  40                  45
   Lys Thr Gln Arg Arg Trp Trp Leu Asp Lys Leu Val Asp Arg Ile Gly
       50                  55                  60
   Val Gly Tyr Val Thr Asp Gly Gly Ser Val Ser Asn Tyr Ile Leu Ser
   65                  70                  75                  80
   Glu Ile Lys Pro Leu His Asn Phe Leu Thr Gln Leu Gln Pro Phe Leu
                   85                  90                  95
   Arg Phe Lys Gln Lys Gln Ala Asn Leu Val Leu Lys Ile Ile Glu Gln
               100                 105                 110
   Leu Pro Ser Ala Lys Glu Ser Pro Asp Lys Phe Leu Glu Val Cys Thr
           115                 120                 125
   Trp Val Asp Gln Ile Ala Ala Leu Asn Asp Ser Lys Thr Arg Lys Thr
       130                 135                 140
   Thr Ser Glu Thr Val Arg Ala Val Leu Asp Ser Leu Ser Glu Lys Lys
   145                 150                 155                 160
   Lys Ser Ser Pro Ala Ala Ala Leu Glu His His His His His His
                   165                 170                 175
<210> 27
   <211> 528
   <212> DNA
   <213> Artificial sequence
<220>
   <223> QAN-B3 variant
<220>
   <221> CDS
   <222> (1)..(528)
<400> 27
   atg gcc aat acc aaa tat aac gaa gag ttc ctg ctg tac ctg gcc ggc      48
   Met Ala Asn Thr Lys Tyr Asn Glu Glu Phe Leu Leu Tyr Leu Ala Gly
   1               5                   10                  15
   ttt gtg gac ggt gac ggt agc atc atc gct cag att aaa cca aac cag      96
   Phe Val Asp Gly Asp Gly Ser Ile Ile Ala Gln Ile Lys Pro Asn Gln
               20                  25                  30
   tct tat aag ttt aaa cat cag cta agc ttg acc ttt cag gtg act caa     144
   Ser Tyr Lys Phe Lys His Gln Leu Ser Leu Thr Phe Gln Val Thr Gln
           35                  40                  45
   aag acc cag cgc cgt tgg ggt ctg gac aaa atg gtg gat gaa att ggc     192
   Lys Thr Gln Arg Arg Trp Gly Leu Asp Lys Met Val Asp Glu Ile Gly
       50                  55                  60
   gtt ggt tac gta gct gat aat gga tcc gtt tcc aac tac atc tta agc     240
   Val Gly Tyr Val Ala Asp Asn Gly Ser Val Ser Asn Tyr Ile Leu Ser
   65                  70                  75                  80
   gaa atc aag ccg ctg cac aac ttc ctg act caa ctg cag ccg ttt ctg     288
   Glu Ile Lys Pro Leu His Asn Phe Leu Thr Gln Leu Gln Pro Phe Leu
                   85                  90                  95
   gaa ctg aaa cag aaa cag gca aac ctg gtt ctg aaa att atc gaa cag     336
   Glu Leu Lys Gln Lys Gln Ala Asn Leu Val Leu Lys Ile Ile Glu Gln
               100                 105                 110
   ctg ccg tct gca aaa gaa tcc ccg gac aaa ttc ctg gaa gtt tgt acc     384
   Leu Pro Ser Ala Lys Glu Ser Pro Asp Lys Phe Leu Glu Val Cys Thr
           115                 120                 125
   tgg gtg gat cag att gca gct ctg aac gat tct aag acg cgt aaa acc     432
   Trp Val Asp Gln Ile Ala Ala Leu Asn Asp Ser Lys Thr Arg Lys Thr
       130                 135                 140
   act tct gaa acc gtt cgt gct gtg ctg gac agc ctg agc gag aag aag     480
   Thr Ser Glu Thr Val Arg Ala Val Leu Asp Ser Leu Ser Glu Lys Lys
   145                 150                 155                 160
   aaa tcc tcc ccg gcg gcc gca ctc gag cac cac cac cac cac cac tga     528
   Lys Ser Ser Pro Ala Ala Ala Leu Glu His His His His His His
                   165                 170                 175
<210> 28
   <211> 175
   <212> PRT
   <213> Artificial sequence
<220>
   <223> Synthetic Construct
<400> 28
   Met Ala Asn Thr Lys Tyr Asn Glu Glu Phe Leu Leu Tyr Leu Ala Gly
   1               5                   10                  15
   Phe Val Asp Gly Asp Gly Ser Ile Ile Ala Gln Ile Lys Pro Asn Gln
               20                  25                  30
   Ser Tyr Lys Phe Lys His Gln Leu Ser Leu Thr Phe Gln Val Thr Gln
           35                  40                  45
   Lys Thr Gln Arg Arg Trp Gly Leu Asp Lys Met Val Asp Glu Ile Gly
       50                  55                  60
   Val Gly Tyr Val Ala Asp Asn Gly Ser Val Ser Asn Tyr Ile Leu Ser
   65                  70                  75                  80
   Glu Ile Lys Pro Leu His Asn Phe Leu Thr Gln Leu Gln Pro Phe Leu
                   85                  90                  95
   Glu Leu Lys Gln Lys Gln Ala Asn Leu Val Leu Lys Ile Ile Glu Gln
               100                 105                 110
   Leu Pro Ser Ala Lys Glu Ser Pro Asp Lys Phe Leu Glu Val Cys Thr
           115                 120                 125
   Trp Val Asp Gln Ile Ala Ala Leu Asn Asp Ser Lys Thr Arg Lys Thr
       130                 135                 140
   Thr Ser Glu Thr Val Arg Ala Val Leu Asp Ser Leu Ser Glu Lys Lys
   145                 150                 155                 160
   Lys Ser Ser Pro Ala Ala Ala Leu Glu His His His His His His
                   165                 170                 175
<210> 29
   <211> 528
   <212> DNA
   <213> Artificial sequence
<220>
   <223> QAN-B4 variant
<220>
   <221> CDS
   <222> (1)..(528)
<400> 29
   atg gcc aat acc aaa tat aac gaa gag ttc ctg ctg tac ctg gcc ggc      48
   Met Ala Asn Thr Lys Tyr Asn Glu Glu Phe Leu Leu Tyr Leu Ala Gly
   1               5                   10                  15
   ttt gtg gac ggt gac ggt agc atc atc gct cag att aaa cca aac cag      96
   Phe Val Asp Gly Asp Gly Ser Ile Ile Ala Gln Ile Lys Pro Asn Gln
               20                  25                  30
   tct tat aag ttt aaa cat cag cta agc ttg acc ttt cag gtg act caa      144
   Ser Tyr Lys Phe Lys His Gln Leu Ser Leu Thr Phe Gln Val Thr Gln
           35                  40                  45
   aag acc cag cgc cgt tgg ggt ctg gac aaa atg gtg gat gaa att ggc      192
   Lys Thr Gln Arg Arg Trp Gly Leu Asp Lys Met Val Asp Glu Ile Gly
       50                  55                  60
   gtt ggt tac gta gct gat aat gga tcc gtt tcc aac tac atc tta agc      240
   Val Gly Tyr Val Ala Asp Asn Gly Ser Val Ser Asn Tyr Ile Leu Ser
   65                  70                  75                  80
   gaa atc aag ccg ctg cac aac ttc ctg act caa ctg cag ccg ttt ctg      288
   Glu Ile Lys Pro Leu His Asn Phe Leu Thr Gln Leu Gln Pro Phe Leu
                   85                  90                  95
   gaa ggg aaa cag aaa cag gca aac ctg gtt ctg aaa att atc gaa cag      336
   Glu Gly Lys Gln Lys Gln Ala Asn Leu Val Leu Lys Ile Ile Glu Gln
               100                 105                 110
   ctg ccg tct gca aaa gaa tcc ccg gac aaa ttc ctg gaa gtt tgt acc      384
   Leu Pro Ser Ala Lys Glu Ser Pro Asp Lys Phe Leu Glu Val Cys Thr
           115                 120                 125
   tgg gtg gat cag att gca gct ctg aac gat tct aag acg cgt aaa acc      432
   Trp Val Asp Gln Ile Ala Ala Leu Asn Asp Ser Lys Thr Arg Lys Thr
       130                 135                 140
   act tct gaa acc gtt cgt gct gtg ctg gac agc ctg agc gag aag aag      480
   Thr Ser Glu Thr Val Arg Ala Val Leu Asp Ser Leu Ser Glu Lys Lys
   145                 150                 155                 160
   aaa tcc tcc ccg gcg gcc gca ctc gag cac cac cac cac cac cac tga      528
   Lys Ser Ser Pro Ala Ala Ala Leu Glu His His His His His His
                   165                 170                 175
<210> 30
   <211> 175
   <212> PRT
   <213> Artificial sequence
<220>
   <223> synthetic Construct
<400> 30
   Met Ala Asn Thr Lys Tyr Asn Glu Glu Phe Leu Leu Tyr Leu Ala Gly
   1               5                   10                  15
   Phe Val Asp Gly Asp Gly Ser Ile Ile Ala Gln Ile Lys Pro Asn Gln
   20 25 30
   Ser Tyr Lys Phe Lys His Gln Leu Ser Leu Thr Phe Gln Val Thr Gln
   35 40 45
   Lys Thr Gln Arg Arg Trp Gly Leu Asp Lys Met Val Asp Glu Ile Gly
   50 55 60
   Val Gly Tyr Val Ala Asp Asn Gly Ser Val Ser Asn Tyr Ile Leu Ser
   65 70 75 80
   Glu Ile Lys Pro Leu His Asn Phe Leu Thr Gln Leu Gln Pro Phe Leu
   85 90 95
   Glu Gly Lys Gln Lys Gln Ala Asn Leu Val Leu Lys Ile Ile Glu Gln
   100 105 110
   Leu Pro Ser Ala Lys Glu Ser Pro Asp Lys Phe Leu Glu Val Cys Thr
   115 120 125
   Trp Val Asp Gln Ile Ala Ala Leu Asn Asp Ser Lys Thr Arg Lys Thr
   130 135 140
   Thr Ser Glu Thr Val Arg Ala Val Leu Asp Ser Leu Ser Glu Lys Lys
   145 150 155 160
   Lys Ser Ser Pro Ala Ala Ala Leu Glu His His His His His His
   165 170 175
<210> 31
   <211> 528
   <212> DNA
   <213> Artificial sequence
<220>
   <223> KTG-B3 variant
<220>
   <221> CDS
   <222> (1)..(528)
<400> 31
   atg gcc aat acc aaa tat aac gaa gag ttc ctg ctg tac ctg gcc ggc      48
   Met Ala Asn Thr Lys Tyr Asn Glu Glu Phe Leu Leu Tyr Leu Ala Gly
   1               5                   10                  15
   ttt gtg gac ggt gac ggt agc atc atc gct cag att aaa cca aac cag      96
   Phe Val Asp Gly Asp Gly Ser Ile Ile Ala Gln Ile Lys Pro Asn Gln
               20                  25                  30
   tct tat aag ttt aaa cat cag cta agc ttg acc ttt aag gtg act caa     144
   Ser Tyr Lys Phe Lys His Gln Leu Ser Leu Thr Phe Lys Val Thr Gln
           35                  40                  45
   aag acc cag cgc cgt tgg ggt ctg gac aaa atg gtg gat gaa att ggc     192
   Lys Thr Gln Arg Arg Trp Gly Leu Asp Lys Met Val Asp Glu Ile Gly
       50                  55                  60
   gtt ggt tac gta acg gat ggg gga tcc gtt tcc aac tac atc tta agc     240
   Val Gly Tyr Val Thr Asp Gly Gly Ser Val Ser Asn Tyr Ile Leu Ser
   65                  70                  75                  80
   gaa atc aag ccg ctg cac aac ttc ctg act caa ctg cag ccg ttt ctg     288
   Glu Ile Lys Pro Leu His Asn Phe Leu Thr Gln Leu Gln Pro Phe Leu
                   85                  90                  95
   gaa ctg aaa cag aaa cag gca aac ctg gtt ctg aaa att atc gaa cag     336
   Glu Leu Lys Gln Lys Gln Ala Asn Leu Val Leu Lys Ile Ile Glu Gln
               100                 105                 110
   ctg ccg tct gca aaa gaa tcc ccg gac aaa ttc ctg gaa gtt tgt acc     384
   Leu Pro Ser Ala Lys Glu Ser Pro Asp Lys Phe Leu Glu Val Cys Thr
           115                 120                 125
   tgg gtg gat cag att gca gct ctg aac gat tct aag acg cgt aaa acc     432
   Trp Val Asp Gln Ile Ala Ala Leu Asn Asp Ser Lys Thr Arg Lys Thr
       130                 135                 140
   act tct gaa acc gtt cgt gct gtg ctg gac agc ctg agc gag aag aag     480
   Thr Ser Glu Thr Val Arg Ala Val Leu Asp Ser Leu Ser Glu Lys Lys
   145                 150                 155                 160
   aaa tcc tcc ccg gcg gcc gca ctc gag cac cac cac cac cac cac tga     528
   Lys Ser Ser Pro Ala Ala Ala Leu Glu His His His His His His
                   165                 170                 175
<210> 32
   <211> 175
   <212> PRT
   <213> Artificial sequence
<220>
   <223> Synthetic Construct
<400> 32
   Met Ala Asn Thr Lys Tyr Asn Glu Glu Phe Leu Leu Tyr Leu Ala Gly
   1               5                   10                  15
   Phe Val Asp Gly Asp Gly Ser Ile Ile Ala Gln Ile Lys Pro Asn Gln
               20                  25                  30
   Ser Tyr Lys Phe Lys His Gln Leu Ser Leu Thr Phe Lys Val Thr Gln
           35                  40                  45
   Lys Thr Gln Arg Arg Trp Gly Leu Asp Lys Met Val Asp Glu Ile Gly
       50                  55                  60
   Val Gly Tyr Val Thr Asp Gly Gly Ser Val Ser Asn Tyr Ile Leu Ser
   65                  70                  75                  80
   Glu Ile Lys Pro Leu His Asn Phe Leu Thr Gln Leu Gln Pro Phe Leu
                   85                  90                  95
   Glu Leu Lys Gln Lys Gln Ala Asn Leu Val Leu Lys Ile Ile Glu Gln
               100                 105                 110
   Leu Pro Ser Ala Lys Glu Ser Pro Asp Lys Phe Leu Glu Val Cys Thr
           115                 120                 125
   Trp Val Asp Gln Ile Ala Ala Leu Asn Asp Ser Lys Thr Arg Lys Thr
       130                 135                 140
   Thr Ser Glu Thr Val Arg Ala Val Leu Asp Ser Leu Ser Glu Lys Lys
   145                 150                 155                 160
   Lys Ser Ser Pro Ala Ala Ala Leu Glu His His His His His His
                   165                 170                 175
<210> 33
   <211> 528
   <212> DNA
   <213> Artificial sequence
<220>
   <223> KTG-B4 variant
<220>
   <221> CDS
   <222> (1)..(528)
<400> 33
   atg gcc aat acc aaa tat aac gaa gag ttc ctg ctg tac ctg gcc ggc      48
   Met Ala Asn Thr Lys Tyr Asn Glu Glu Phe Leu Leu Tyr Leu Ala Gly
   1               5                   10                  15
   ttt gtg gac ggt gac ggt agc atc atc gct cag att aaa cca aac cag      96
   Phe Val Asp Gly Asp Gly Ser Ile Ile Ala Gln Ile Lys Pro Asn Gln
               20                  25                  30
   tct tat aag ttt aaa cat cag cta agc ttg acc ttt aag gtg act caa      144
   Ser Tyr Lys Phe Lys His Gln Leu Ser Leu Thr Phe Lys Val Thr Gln
           35                  40                  45
   aag acc cag cgc cgt tgg ggt ctg gac aaa atg gtg gat gaa att ggc      192
   Lys Thr Gln Arg Arg Trp Gly Leu Asp Lys Met Val Asp Glu Ile Gly
       50                  55                  60
   gtt ggt tac gta acg gat ggg gga tcc gtt tcc aac tac atc tta agc      240
   Val Gly Tyr Val Thr Asp Gly Gly Ser Val Ser Asn Tyr Ile Leu Ser
   65                  70                  75                  80
   gaa atc aag ccg ctg cac aac ttc ctg act caa ctg cag ccg ttt ctg      288
   Glu Ile Lys Pro Leu His Asn Phe Leu Thr Gln Leu Gln Pro Phe Leu
                   85                  90                  95
   gaa ggg aaa cag aaa cag gca aac ctg gtt ctg aaa att atc gaa cag      336
   Glu Gly Lys Gln Lys Gln Ala Asn Leu Val Leu Lys Ile Ile Glu Gln
               100                 105                 110
   ctg ccg tct gca aaa gaa tcc ccg gac aaa ttc ctg gaa gtt tgt acc      384
   Leu Pro Ser Ala Lys Glu Ser Pro Asp Lys Phe Leu Glu Val Cys Thr
           115                 120                 125
   tgg gtg gat cag att gca gct ctg aac gat tct aag acg cgt aaa acc      432
   Trp Val Asp Gln Ile Ala Ala Leu Asn Asp Ser Lys Thr Arg Lys Thr
       130                 135                 140
   act tct gaa acc gtt cgt gct gtg ctg gac agc ctg agc gag aag aag      480
   Thr Ser Glu Thr Val Arg Ala Val Leu Asp Ser Leu Ser Glu Lys Lys
   145                 150                 155                 160
   aaa tcc tcc ccg gcg gcc gca ctc gag cac cac cac cac cac cac tga      528
   Lys Ser Ser Pro Ala Ala Ala Leu Glu His His His His His His
                   165                 170                 175
<210> 34
   <211> 175
   <212> PRT
   <213> Artificial sequence
<220>
   <223> Synthetic Construct
<400> 34
   Met Ala Asn Thr Lys Tyr Asn Glu Glu Phe Leu Leu Tyr Leu Ala Gly
   1               5                   10                  15
   Phe Val Asp Gly Asp Gly Ser Ile Ile Ala Gln Ile Lys Pro Asn Gln
               20                  25                  30
   Ser Tyr Lys Phe Lys His Gln Leu Ser Leu Thr Phe Lys Val Thr Gln
           35                  40                  45
   Lys Thr Gln Arg Arg Trp Gly Leu Asp Lys Met Val Asp Glu Ile Gly
       50                  55                  60
   Val Gly Tyr Val Thr Asp Gly Gly Ser Val Ser Asn Tyr Ile Leu Ser
   65                  70                  75                  80
   Glu Ile Lys Pro Leu His Asn Phe Leu Thr Gln Leu Gln Pro Phe Leu
                   85                  90                  95
   Glu Gly Lys Gln Lys Gln Ala Asn Leu Val Leu Lys Ile Ile Glu Gln
               100                 105                 110
   Leu Pro Ser Ala Lys Glu Ser Pro Asp Lys Phe Leu Glu Val Cys Thr
           115                 120                 125
   Trp Val Asp Gln Ile Ala Ala Leu Asn Asp Ser Lys Thr Arg Lys Thr
       130                 135                 140
   Thr Ser Glu Thr Val Arg Ala Val Leu Asp Ser Leu Ser Glu Lys Lys
   145                 150                 155                 160
   Lys Ser Ser Pro Ala Ala Ala Leu Glu His His His His His His
                   165                 170                 175
<210> 35
   <211> 37
   <212> DNA
   <213> Artificial sequence
<220>
   <223> Primer
<400> 35
   ctggacaaac tagtggatag aattggcgtt ggttacg 37
<210> 36
   <211> 44
   <212> DNA
   <213> Artificial sequence
<220>
   <223> Primer
<400> 36
   tcaactgcag ccgtttctgg aagggaaaca gaaacaggca aacc 44
<210> 37
   <211> 26
   <212> DNA
   <213> Artificial sequence
<220>
   <223> Primer
<400> 37
   gcaactttag tgctgacaca tacagg 26
<210> 38
   <211> 39
   <212> DNA
   <213> Artificial sequence
<220>
   <223> Primer
<400> 38
   tttgtccaga aaccaacggt cctgggtctt ttgagtcac 39
<210> 39
   <211> 40
   <212> DNA
   <213> Artificial sequence
<220>
   <223> Primer
<400> 39
   gtgactcaaa agacccagga ccgttggttt ctggacaaac 40
<210> 40
   <211> 24
   <212> DNA
   <213> Artificial sequence
<220>
   <223> Primer
<400> 40
   acaaccttga ttggagactt gacc 24
<210> 41
   <211> 39
   <212> DNA
   <213> Artificial sequence
<220>
   <223> Primer
<400> 41
   ggttttacgc gtcttagaac ggttcagagc tgcaatctg 39
<210> 42
   <211> 39
   <212> DNA
   <213> Artificial sequence
<220>
   <223> Primer
<400> 42
   cagattgcag ctctgaaccg ttctaagacg cgtaaaacc 39
<210> 43
   <211> 163
   <212> PRT
   <213> Chlamydomonas reinhardtii
<400> 43
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Lys Pro Asn Gln Ser
   20 25 30
   Tyr Lys Phe Lys His Gln Leu Ser Leu Thr Phe Gln Val Thr Gln Lys
   35 40 45
   Thr Gln Arg Arg Trp Phe Leu Asp Lys Leu Val Asp Glu Ile Gly Val
   50 55 60
   Gly Tyr Val Arg Asp Arg Gly Ser Val Ser Asp Tyr Ile Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Glu Gln Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 44
   <211> 21
   <212> DNA
   <213> artificial sequence
<220>
   <223> CMVfor primer
<400> 44
   cgcaaatggg cggtaggcgt g 21
<210> 45
   <211> 35
   <212> DNA
   <213> artificial sequence
<220>
   <223> K7ERev primer
<400> 45
   gtacagcagg aactcttcgt tatatttggt attgg 35
<210> 46
   <211> 34
   <212> DNA
   <213> artificial sequence
<220>
   <223> K7EFor primer
<400> 46
   aataccaaat ataacgaaga gttcctgctg tacc 34
<210> 47
   <211> 24
   <212> DNA
   <213> artificial sequence
<220>
   <223> v5epitope Rev primer
<400> 47
   cgtagaatcg agaccgagga gagg 24
<210> 48
   <211> 32
   <212> DNA
   <213> artificial sequence
<220>
   <223> E8Krev primer
<400> 48
   caggtacagc aggaactttt tgttatattt gg 32
<210> 49
   <211> 34
   <212> DNA
   <213> artificial sequence
<220>
   <223> E8KFor primer
<400> 49
   accaaatata acaaaaagtt cctgctgtac ctgg 34
<210> 50
   <211> 34
   <212> DNA
   <213> artificial sequence
<220>
   <223> E61RRev primer
<400> 50
   gtaaccaacg ccaatacgat ccactagttt gtcc 34
<210> 51
   <211> 34
   <212> DNA
   <213> artificial sequence
<220>
   <223> E61RFor primer
<400> 51
   gacaaactag tggatcgtat tggcgttggt tacg 34
<210> 52
   <211> 34
   <212> DNA
   <213> artificial sequence
<220>
   <223> K96ERev primer
<400> 52
   cctgtttctg tttcagttcc agaaacggct gcag 34
<210> 53
   <211> 34
   <212> DNA
   <213> artificial sequence
<220>
   <223> K96EFor primer
<400> 53
   ctgcagccgt ttctggaact gaaacagaaa cagg 34
<210> 54
   <211> 84
   <212> DNA
   <213> artificial sequence
<220>
   <223> CCM2For primer
<400> 54
   aagcagagct ctctggctaa ctagagaacc cactgcttac tggcttatcg accatggcca 60
   ataccaaata taacaaagag ttcc 84
<210> 55
   <211> 38
   <212> DNA
   <213> artificial sequence
<220>
   <223> CCMRev primer
<400> 55
   ctgctctaga ttagtcggcc gccggggagg atttcttc 38
<210> 56
   <211> 38
   <212> DNA
   <213> artificial sequence
<220>
   <223> CreCterSacI primer
<400> 56
   tagacgagct cctacgggga ggatttcttc ttctcgct 38
<210> 57
   <211> 131
   <212> DNA
   <213> artificial sequence
<220>
   <223> RM2 primer
<400> 57
   tatcggccgg tggatctgat aagtataatc aggctctgtc taaatacaac caagcactgt 60
   ccaagtacaa tcaggccctg tctggtggag gcggttccaa caaagagttc ctgctgtatc 120
   ttgctggatt t 131
<210> 58
   <211> 22
   <212> DNA
   <213> artificial sequence
<220>
   <223> C1221 target
<400> 58
   caaaacgtcg tacgacgttt tg 22
<210> 59
   <211> 22
   <212> DNA
   <213> artificial sequence
<220>
   <223> 10GTT_P target
<400> 59
   cgttacgtcg tacgacgtaa cg 22
<210> 60
   <211> 22
   <212> DNA
   <213> artificial sequence
<220>
   <223> 5CAG_P target
<400> 60
   caaaaccagg tacctggttt tg 22
<210> 61
   <211> 22
   <212> DNA
   <213> artificial sequence
<220>
   <223> 10TGG_P target
<400> 61
   ctggacgtcg tacgacgtcc ag 22
<210> 62
   <211> 22
   <212> DNA
   <213> artificial sequence
<220>
   <223> 5GAG_P target
<400> 62
   caaaacgagg tacctcgttt tg 22
<210> 63
   <211> 22
   <212> DNA
   <213> artificial sequence
<220>
   <223> RAG1.10 target
<400> 63
   tgttctcagg tacctcagcc ag 22
<210> 64
   <211> 22
   <212> DNA
   <213> artificial sequence
<220>
   <223> RAG1.10.2 target
<400> 64
   tgttctcagg tacctgagaa ca 22
<210> 65
   <211> 22
   <212> DNA
   <213> artificial sequence
<220>
   <223> RAG1.10.3 target
<400> 65
   ctggctgagg tacctcagcc ag 22